# EUROPEAN PATENT APPLICATION

(11) **EP 3 925 965 A1**
(43) Date of publication of application: **22.12.2021**
(21) Application number: 20756660.5
(22) Date of filing: 10.02.2020
(51) Int. Cl.: C07H 21/04, C12N 15/113

(54) **5'-MODIFIED NUCLEOSIDE AND NUCLEOTIDE USING SAME**

(30) Priority: 13.02.2019 JP 2019023514
(71) Applicant: Osaka University, Suita-shi, Osaka 565-0871 (JP)
(72) Inventor: OBIKA, Satoshi, Suita-shi, Osaka 565-0871 (JP); YAMAGUCHI, Takao, Suita-shi, Osaka 565-0871 (JP); HABUCHI, Takaki, Suita-shi, Osaka 565-0871 (JP); KATO, Go, Suita-shi, Osaka 565-0871 (JP); INOUE, Takao, Suita-shi, Osaka 565-0871 (JP); YOSHIDA, Tokuyuki, Suita-shi, Osaka 565-0871 (JP); ISLAM, Md Ariful, Suita-shi, Osaka 565-0871 (JP)
(74) Representative: Clements, Andrew Russell Niel
(86) International application number: PCT/JP2020/005095
(87) International publication number: WO 2020/166551

(57) **Abstract**

Disclosed are a 5'-modified nucleoside and a nucleotide using the same. The nucleoside of the present invention is represented by the formula (I) below. The 5'-modified nucleoside of the present invention is usable as a substitute for a phosphorothioate-modified nucleic acid, which has a risk of, for example, accumulation in a specific organ. The 5'-modified nucleoside also has excellent industrial productivity because a diastereomer separation step is not involved in the production process thereof.

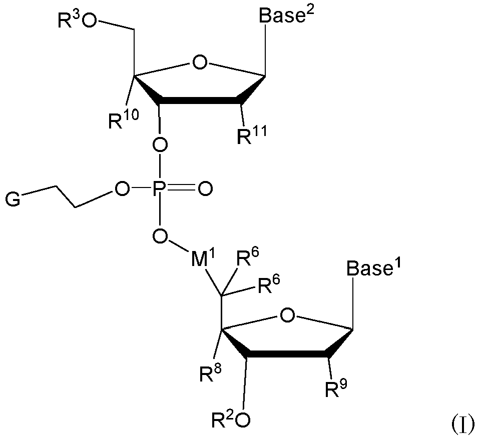

## Description

### Technical Field

The present invention relates to a 5'-modified nucleoside and a nucleotide using the same. More specifically, the invention relates to a 5'-modified nucleoside that has good nuclease-resistant ability and can be produced with high efficiency, and a nucleotide using the same.

### Background Art

Treatments of disorders using nucleic acid drugs include antisense therapies, antigene therapies, aptamers, siRNAs, and the like. An antisense therapy is the procedure for treatment or prevention of diseases involving inhibiting a translation process of pathogenic RNAs by externally introducing oligonucleotides (antisense strands) complementary to disease-associated mRNAs to form the double strands. The mechanism of siRNAs is similar to that of the antisense therapies, involving inhibiting translation from mRNAs to proteins by administration of double-stranded RNAs to the body. Meanwhile, in the antigene therapies, transcription of DNA to RNA is suppressed by externally introducing triple-strand-forming oligonucleotides corresponding to the DNA sites transcribed into the pathogenic RNA. Aptamers, which are small nucleic acid molecules (oligonucleotides), exert their functions by binding to disease-related biological components, such as proteins.

Various artificial nucleic acids have been developed as materials for such nucleic acid drugs. In particular, 2',4'-BNA (bridged nucleic acid, also known as LNA) in which the conformation of the sugar moiety in the nucleic acid is fixed through cross-linking has been reported to have excellent binding affinity for a single-stranded RNA (ssRNA) (Non-Patent Documents 1 and 2), and is expected to be suitable for various nucleic acid drugs for antisense therapies and the like.

Meanwhile, an artificial nucleic acid obtained by introducing a methyl group into the 5' position of a nucleic acid has been reported to have excellent properties in terms of the nuclease-resistant ability (Patent Documents 1 to 3). Therefore, an artificial nucleic acid obtained by introducing a substituent into the 5' position is also expected to have applications to diagnosis and medicine.

However, synthesis of such an artificial nucleic acid obtained by introducing a substituent into the 5' position involves separation of diastereomers (Non-Patent Documents 3 and 4), and thus, the production process is complicated as a whole. Therefore, further development for enabling industrial production thereof is desired.

### Related Art Documents

Patent Document
Patent Document 1: WO2010/048549
Patent Document 2: WO2010/048585
Patent Document 3: WO2010/077578

Non-Patent Document [0007]
Non-Patent Document 1: S.Obika et al., T. Tetrahedron Lett. 1997, 38, 8735-8738
Non-Patent Document 2: S. Singh et al., J. Chem. Commun. 1998, 455-456
Non-Patent Document 3: Yawman et al., J. Org. Chem. 1995, 60, 788-789
Non-Patent Document 4: Manoharan et al., J. Org. Chem. 2016, 81, 2261-2279

### Summary of Invention

### Problem to be Solved by the Invention

The present invention was made to address the above-described problems, and it is an object thereof to provide a nucleoside modified at the 5' position that has good nuclease-resistant ability and can be produced with high efficiency without involving the separation of diastereomers in the synthetic pathway thereto, and a nucleotide using the same.

The present invention is a compound represented by a formula (I) below or a salt thereof: 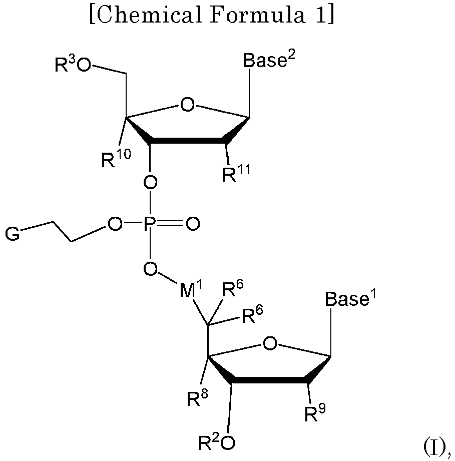 wherein Base¹ and Base² each independently represent a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, a C₁ to C₆ linear alkyl group, a C₁ to C₆ linear alkoxy group, a mercapto group, a mercapto group protected by a protecting group for nucleic acid synthesis, a C₁ to C₆ linear alkylthio group, an amino group, a C₁ to C₆ linear alkylamino group, an amino group protected by a protecting group for nucleic acid synthesis, and halogen atoms;
G is a cyano group or a nitro group;
R² and R³ each independently represent a hydrogen atom, a hydroxy group protecting group for nucleic acid synthesis, a C₁ to C₇ alkyl group that may be branched or form a ring, a C₂ to C₇ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R⁴)R⁵, wherein R⁴ and R⁵ each independently represent a hydroxy group, a hydroxy group protected by a protecting group for nucleic acid synthesis, a mercapto group, a mercapto group protected by a protecting group for nucleic acid synthesis, an amino group, a C₁ to C₆ alkoxy group, a C₁ to C₆ alkylthio group, a C₁ to C₆ cyanoalkoxy group, or a dialkylamino group having a C₁ to C₆ alkyl group;
R⁶ is a C₁ or C₂ alkyl group atoms that may be substituted with a halogen atom;
R⁸ is a hydrogen atom, and R⁹ is a hydrogen atom or a halogen atom; a C₁ to C₆ linear alkoxy group that may be substituted with a C₁ to C₆ linear alkoxy group; or -OR¹², wherein R¹² is a hydrogen atom or a hydroxy group protecting group for nucleic acid synthesis, or
R⁸ and R⁹ together represent a divalent group represented by a formula below: 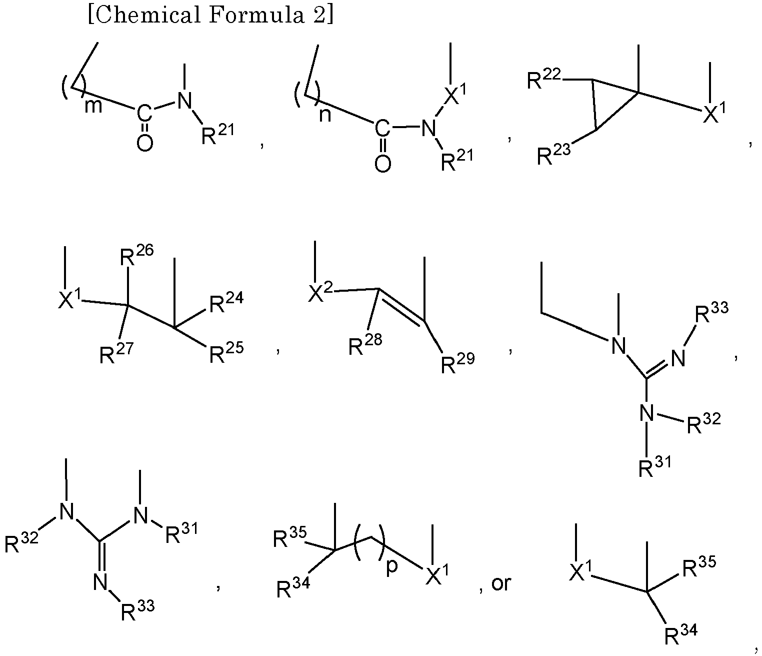 wherein R²¹ is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R²² and R²³ are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R²² and R²³ together represent -(CH₂)_{q1}-, wherein q1 is an integer from 2 to 5;
R²⁴ and R²⁵ are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R²⁴ and R²⁵ together represent =C(R³⁶)R³⁷, wherein R³⁶ and R³⁷ each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R²⁶ and R²⁷ are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁸ is a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁹ is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R³¹, R³², and R³³ are each independently a hydrogen atom, a C₁ to C₆ alkyl group that may be branched or form a ring, or an amino group protecting group for nucleic acid synthesis;
R³⁴ and R³⁵ are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m is an integer from 0 to 2;
   n is an integer of 0 or 1;
   p is an integer of 0 or 1;
   X¹ is an oxygen atom, a sulfur atom, or an amino group; and
   X² is an oxygen atom or a sulfur atom;
R¹⁰ is a hydrogen atom, and R¹¹ is a hydrogen atom or a halogen atom; a C₁ to C₆ linear alkoxy group that may be substituted with a C₁ to C₆ linear alkoxy group; or -OR^{12b}, wherein R^{12b} is a hydrogen atom or a hydroxy group protecting group for nucleic acid synthesis, or
R¹⁰ and R¹¹ together represent a divalent group represented by a formula below: 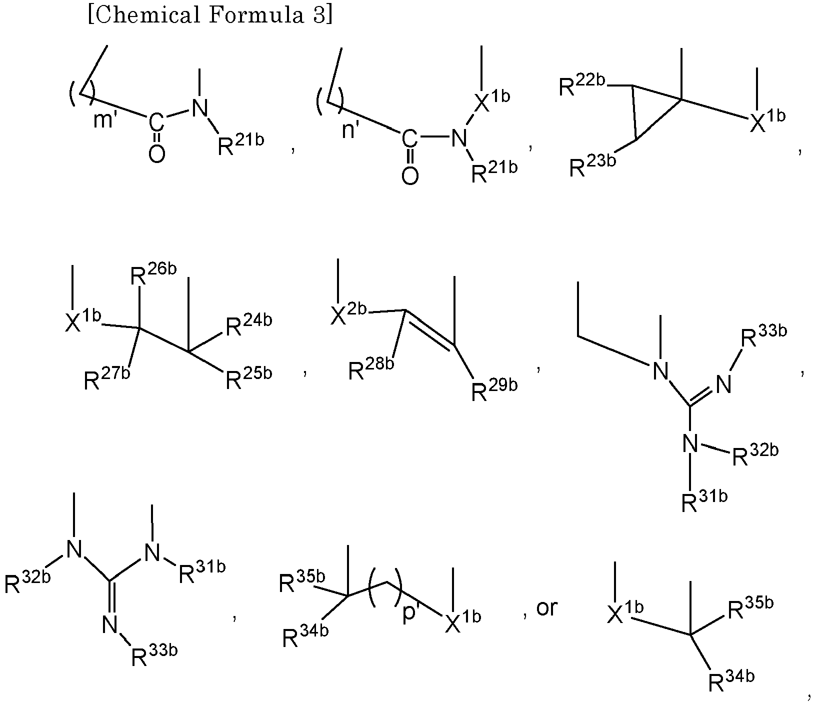 wherein R^{21b} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R^{22b} and R^{23b} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22b} and R^{23b} together represent -(CH₂)_{q2}-, wherein q2 is an integer from 2 to 5;
R^{24b} and R^{25b} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24b} and R^{25b} together represent =C(R^{36b})R^{37b}, wherein R^{36b} and R^{37b} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26b} and R^{27b} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28b} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29b} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31b}, R^{32b}, and R^{33b} are each independently a hydrogen atom, a C₁ to C₆ alkyl group that may be branched or form a ring, or an amino group protecting group for nucleic acid synthesis;
R^{34b} and R^{35b} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m' is an integer from 0 to 2;
   n' is an integer of 0 or 1;
   p' is an integer of 0 or 1;
   X^{1b} is an oxygen atom, a sulfur atom, or an amino group; and
   X^{2b} is an oxygen atom or a sulfur atom; and
M¹ represents a single bond or a formula below: 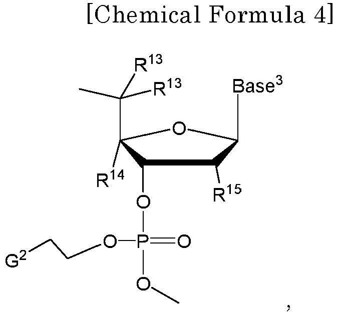 wherein Base³ represents a purin-9-yl group that may have any one or more substituents selected from the α group, or a 2-oxo-1,2-dihydropyrimidin-1-yl group that may have any one or more substituents selected from the α group;
G² is a cyano group or a nitro group;
R¹³ is a C₁ or C₂ alkyl group atoms that may be substituted with a halogen atom;
R¹⁴ is a hydrogen atom, and R¹⁵ is a hydrogen atom or a halogen atom; a C₁ to C₆ linear alkoxy group that may be substituted with a C₁ to C₆ linear alkoxy group; or -OR^{12c}, wherein R^{12c} is a hydrogen atom or a hydroxy group protecting group for nucleic acid synthesis, or
R¹⁴ and R¹⁵ together represent a divalent group represented by a formula below: 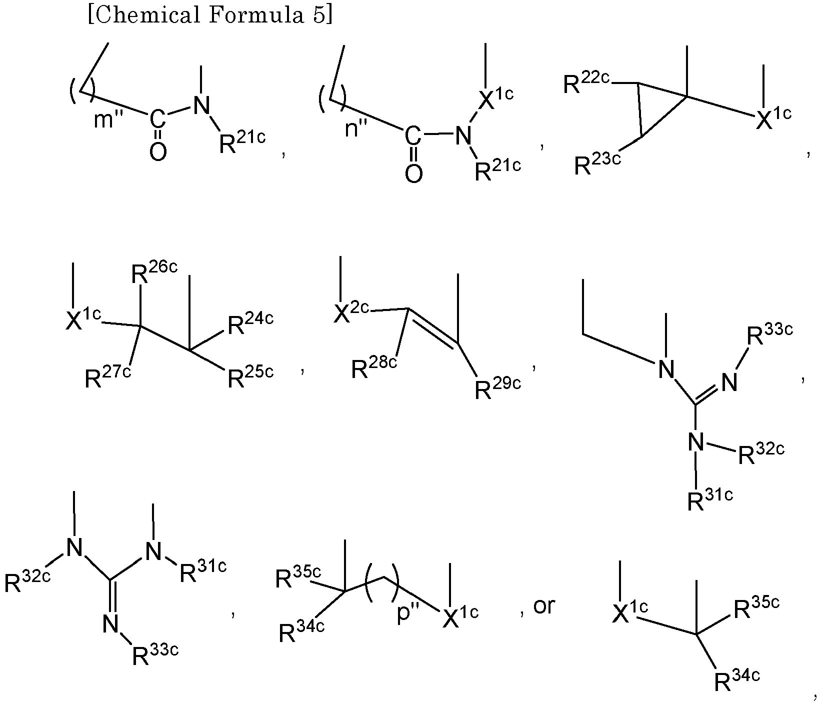 wherein R^{21c} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R^{22c} and R^{23c} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22c} and R^{23c} together represent -(CH₂)_{q3}-, wherein q3 is an integer from 2 to 5;
R^{24c} and R^{25c} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24c} and R^{25c} together represent =C(R^{36c})R^{37c}, wherein R^{36c} and R^{37c} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26c} and R^{27c} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28c} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29c} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31c}, R^{32c}, and R^{33c} are each independently a hydrogen atom, a C₁ to C₆ alkyl group that may be branched or form a ring, or an amino group protecting group for nucleic acid synthesis;
R^{34c} and R^{35c} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m" is an integer from 0 to 2;
   n" is an integer of 0 or 1;
   p" is an integer of 0 or 1;
   X^{1c} is an oxygen atom, a sulfur atom, or an amino group; and
   X^{2c} is an oxygen atom or a sulfur atom.

In one embodiment, M¹ in the formula (I) is a single bond.

In a further embodiment, the Base¹ and the Base ² in the formula (I) are each independently a 6-aminopurin-9-yl group, a 2,6-diaminopurin-9-yl group, a 2-amino-6-chloropurin-9-yl group, a 2-amino-6-fluoropurin-9-yl group, a 2-amino-6-bromopurin-9-yl group, a 2-amino-6-hydroxypurin-9-yl group, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl group, a 4-amino-2-oxo-5-fluoro-1,2-dihydropyrimidin-1-yl group, a 4-amino-2-oxo-5-chloro-1,2-dihydropyrimidin-1-yl group, a 2-oxo-4-methoxy-1,2-dihydropyrimidin-1-yl group, a 2-oxo-4 mercapto-1,2-dihydropyrimidin-1-yl group, a 2-oxo-4-hydroxy-1,2-dihydropyrimidin-1-yl group, a 2-oxo-4-hydroxy-5-methyl-1,2-dihydropyrimidin-1-yl group, or a 4-amino-5-methyl-2-oxo-1,2-dihydropyrimidin-1-yl group.

In a further embodiment, the Base¹ and the Base² in the formula (I) are each independently a group selected from the group consisting of formulae below:

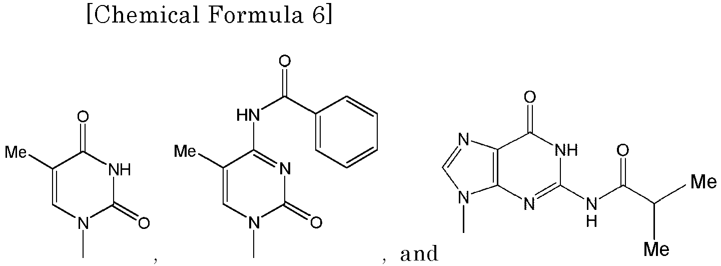

In a further embodiment, R⁶ in the formula (I) is a methyl group or an ethyl group.

In a further embodiment, R⁸, R⁹, R¹⁰, and R¹¹ in the formula (I) are all hydrogen atoms.

The present invention is also an oligonucleotide containing at least one nucleoside structure represented by a formula (II) below or a pharmacologically acceptable salt thereof: 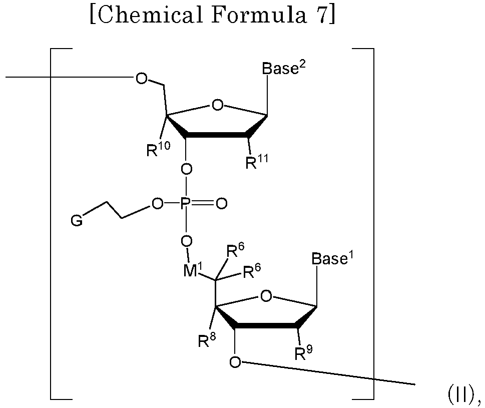 wherein Base¹ and Base² each independently represent a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, a C₁ to C₆ linear alkyl group, a C₁ to C₆ linear alkoxy group, a mercapto group, a mercapto group protected by a protecting group for nucleic acid synthesis, a C₁ to C₆ linear alkylthio group, an amino group, a C₁ to C₆ linear alkylamino group, an amino group protected by a protecting group for nucleic acid synthesis, and halogen atoms;
G is a cyano group or a nitro group;
R⁶ is a C₁ or C₂ alkyl group atoms that may be substituted with a halogen atom;
R⁸ is a hydrogen atom, and R⁹ is a hydrogen atom or a halogen atom; a C₁ to C₆ linear alkoxy group that may be substituted with a C₁ to C₆ linear alkoxy group; or -OR¹², wherein R¹² is a hydrogen atom or a hydroxy group protecting group for nucleic acid synthesis, or
R⁸ and R⁹ together represent a divalent group represented by a formula below: 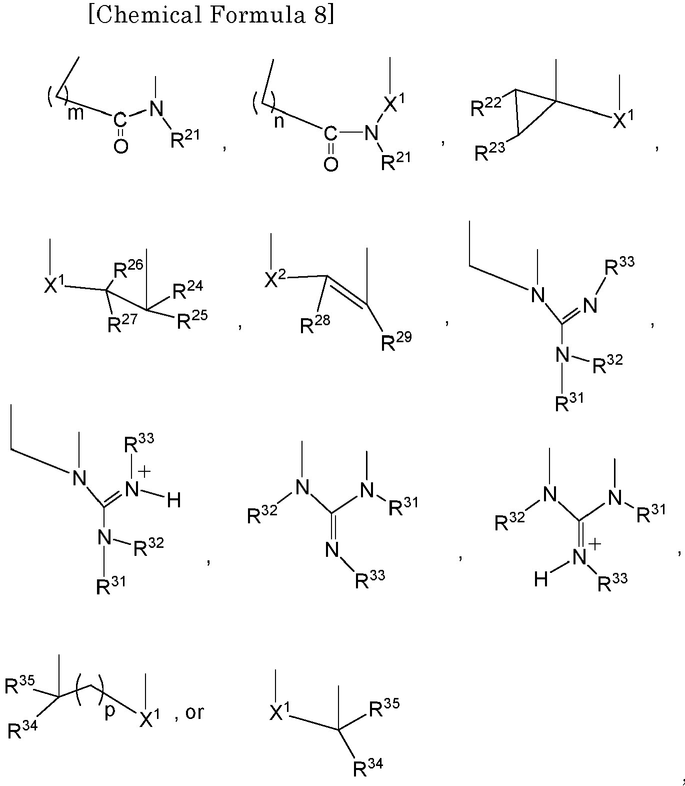 wherein R²¹ is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R²² and R²³ are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R²² and R²³ together represent -(CH₂)_{q1}-, wherein q1 is an integer from 2 to 5;
R²⁴ and R²⁵ are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R²⁴ and R²⁵ together represent =C(R³⁶)R³⁷, wherein R³⁶ and R³⁷ each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R²⁶ and R²⁷ are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁸ is a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁹ is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R³¹, R³², and R³³ are each independently a hydrogen atom, a C₁ to C₆ alkyl group that may be branched or form a ring, or an amino group protecting group for nucleic acid synthesis;
R³⁴ and R³⁵ are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m is an integer from 0 to 2;
   n is an integer of 0 or 1;
   p is an integer of 0 or 1;
   X¹ is an oxygen atom, a sulfur atom, or an amino group; and
   X² is an oxygen atom or a sulfur atom;
R¹⁰ is a hydrogen atom, and R¹¹ is a hydrogen atom or a halogen atom; a C₁ to C₆ linear alkoxy group that may be substituted with a C₁ to C₆ linear alkoxy group; or -OR^{12b}, wherein R^{12b} is a hydrogen atom or a hydroxy group protecting group for nucleic acid synthesis, or
R¹⁰ and R¹¹ together represent a divalent group represented by a formula below: 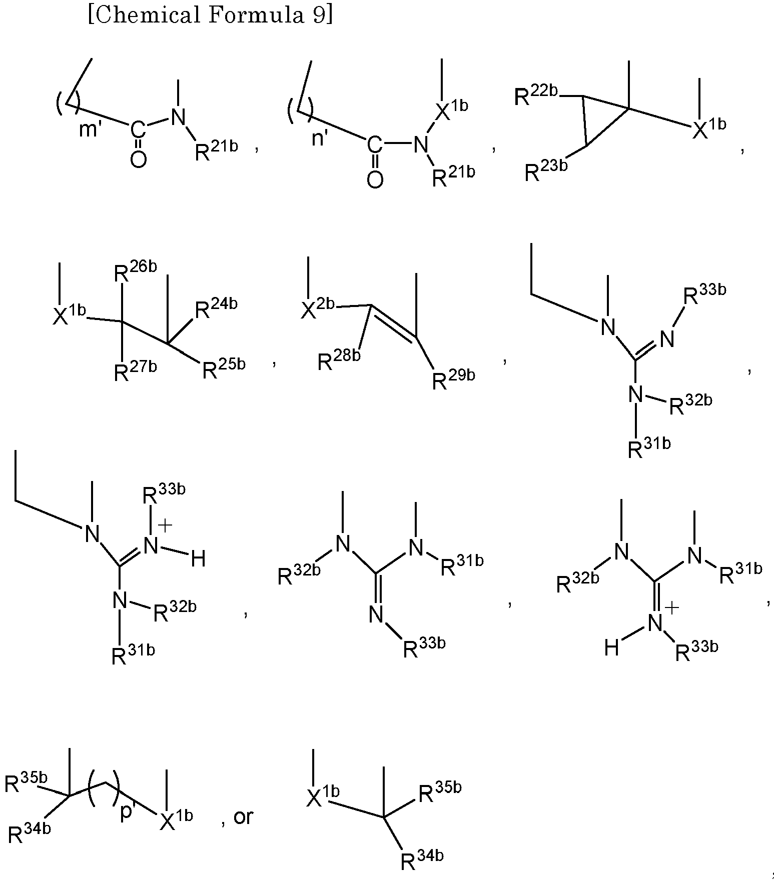
wherein R^{21b} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R^{22b} and R^{23b} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22b} and R^{23b} together represent -(CH₂)_{q2}-, wherein q2 is an integer from 2 to 5;
R^{24b} and R^{25b} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24b} and R^{25b} together represent =C(R^{36b})R^{37b}, wherein R^{36b} and R^{37b} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26b} and R^{27b} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28b} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29b} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31b}, R^{32b}, and R^{33b} are each independently a hydrogen atom, a C₁ to C₆ alkyl group that may be branched or form a ring, or an amino group protecting group for nucleic acid synthesis;
R^{34b} and R^{35b} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m' is an integer from 0 to 2;
   n' is an integer of 0 or 1;
   p' is an integer of 0 or 1;
   X^{1b} is an oxygen atom, a sulfur atom, or an amino group; and
   X^{2b} is an oxygen atom or a sulfur atom; and
   M¹ represents a single bond or a formula below: 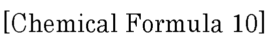 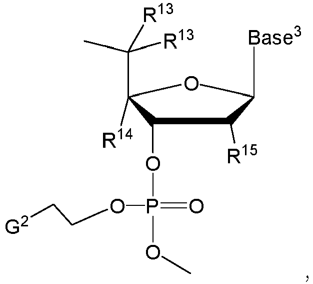 wherein Base³ represents a purin-9-yl group that may have any one or more substituents selected from the α group, or a 2-oxo-1,2-dihydropyrimidin-1-yl group that may have any one or more substituents selected from the α group;
      G² is a cyano group or a nitro group;
      R¹³ is a C₁ or C₂ alkyl group atoms that may be substituted with a halogen atom;
      R¹⁴ is a hydrogen atom, and R¹⁵ is a hydrogen atom or a halogen atom; a C₁ to C₆ linear alkoxy group that may be substituted with a C₁ to C₆ linear alkoxy group; or -OR^{12c}, wherein R^{12c} is a hydrogen atom or a hydroxy group protecting group for nucleic acid synthesis, or
      R¹⁴ and R¹⁵ together represent a divalent group represented by a formula below: 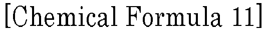 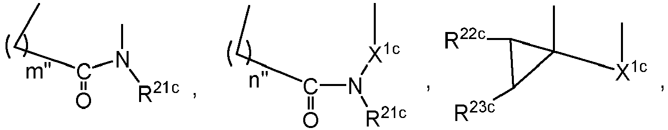 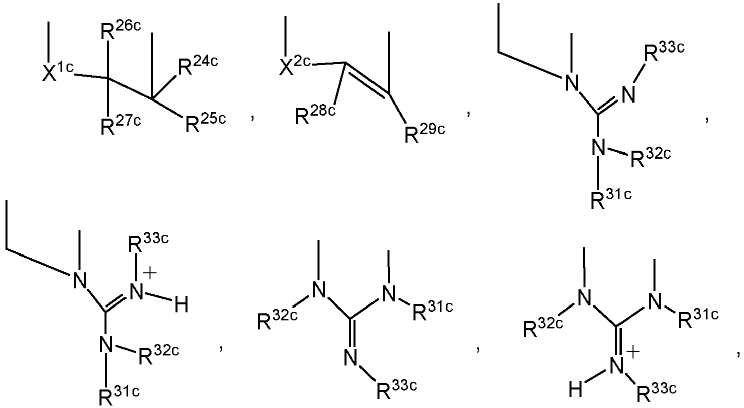 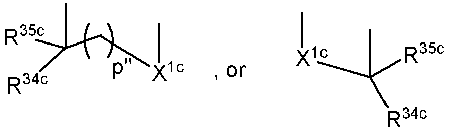 wherein R^{21c} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
      R^{22c} and R^{23c} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
      R^{22c} and R^{23c} together represent -(CH₂)_{q3}-, wherein q3 is an integer from 2 to 5;
      R^{24c} and R^{25c} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
      R^{24c} and R^{25c} together represent =C(R^{36c})R^{37c}, wherein R^{36c} and R^{37c} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
      R^{26c} and R^{27c} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
      R^{28c} is a hydrogen atom, a C₁ to C₆ alkyl group that may be branched or form a ring, a C₁ to C₆ alkoxy group that may be branched or form a ring, or a C₁ to C₆ linear or branched alkylthio group;
      R^{29c} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
      R^{31c}, R^{32c}, and R^{33c} are each independently a hydrogen atom, a C₁ to C₆ alkyl group that may be branched or form a ring, or an amino group protecting group for nucleic acid synthesis;
      R^{34c} and R^{35c} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
         m" is an integer from 0 to 2;
         n" is an integer of 0 or 1;
         p" is an integer of 0 or 1;
         X^{1c} is an oxygen atom, a sulfur atom, or an amino group; and
         X^{2c} is an oxygen atom or a sulfur atom.

In one emdobiment, M¹ in the formula (II) is a single bond.

In a further embodiment, R⁶ in the formula (II) is a methyl group or an ethyl group.

In a further embodiment, R⁸, R⁹, R¹⁰, and R¹¹ in the formula (II) are all hydrogen atoms.

The present invention is also a method for producing the oligonucleotide or pharmacologically acceptable salt thereof, which comprises:
synthesizing an oligonucleotide using a compound represented by a formula (I) below or a pharmacologically acceptable salt thereof: 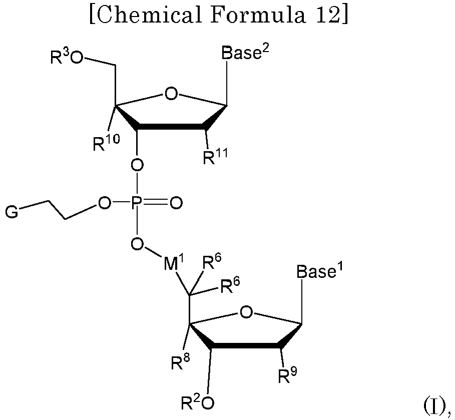
wherein Base¹ and Base² each independently represent a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, a C₁ to C₆ linear alkyl group, a C₁ to C₆ linear alkoxy group, a mercapto group, a mercapto group protected by a protecting group for nucleic acid synthesis, a C₁ to C₆ linear alkylthio group, an amino group, a C₁ to C₆ linear alkylamino group, an amino group protected by a protecting group for nucleic acid synthesis, and halogen atoms;
G is a cyano group or a nitro group;
R² and R³ each independently represent a hydrogen atom, a hydroxy group protecting group for nucleic acid synthesis, a C₁ to C₇ alkyl group that may be branched or form a ring, a C₂ to C₇ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R⁴)R⁵, wherein R⁴ and R⁵ each independently represent a hydroxy group, a hydroxy group protected by a protecting group for nucleic acid synthesis, a mercapto group, a mercapto group protected by a protecting group for nucleic acid synthesis, an amino group, a C₁ to C₆ alkoxy group, a C₁ to C₆ alkylthio group, a C₁ to C₆ cyanoalkoxy group, or a dialkylamino group having a C₁ to C₆ alkyl group;
R⁶ is a C₁ or C₂ alkyl group atoms that may be substituted with a halogen atom;
R⁸ is a hydrogen atom, and R⁹ is a hydrogen atom or a halogen atom; a C₁ to C₆ linear alkoxy group that may be substituted with a C₁ to C₆ linear alkoxy group; or -OR¹², wherein R¹² is a hydrogen atom or a hydroxy group protecting group for nucleic acid synthesis, or
R⁸ and R⁹ together represent a divalent group represented by a formula below: 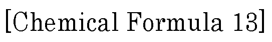 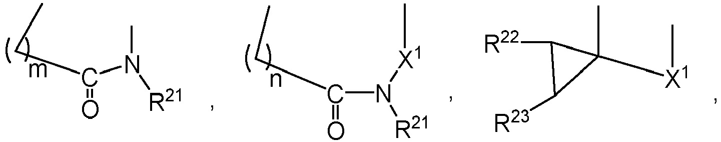 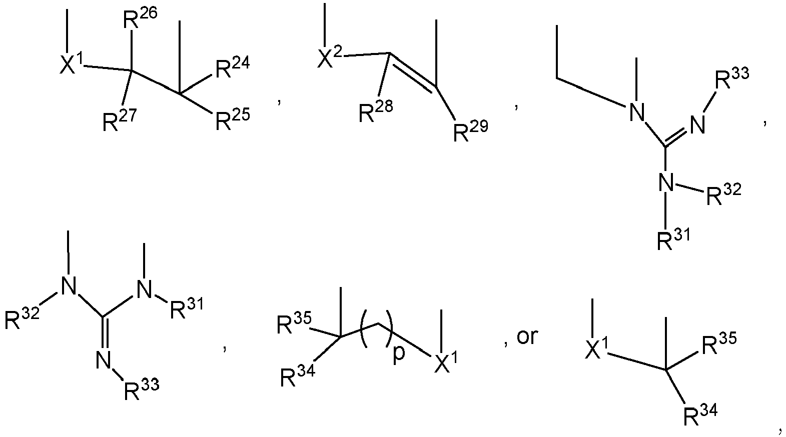
wherein R²¹ is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R²² and R²³ are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R²² and R²³ together represent -(CH₂)_{q1}-, wherein q1 is an integer from 2 to 5;
R²⁴ and R²⁵ are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R²⁴ and R²⁵ together represent =C(R³⁶)R³⁷, wherein R³⁶ and R³⁷ each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R²⁶ and R²⁷ are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁸ is a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁹ is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R³¹, R³², and R³³ are each independently a hydrogen atom, a C₁ to C₆ alkyl group that may be branched or form a ring, or an amino group protecting group for nucleic acid synthesis;
R³⁴ and R³⁵ are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m is an integer from 0 to 2;
   n is an integer of 0 or 1;
   p is an integer of 0 or 1;
   X¹ is an oxygen atom, a sulfur atom, or an amino group; and
   X² is an oxygen atom or a sulfur atom;
R¹⁰ is a hydrogen atom, and R¹¹ is a hydrogen atom or a halogen atom; a C₁ to C₆ linear alkoxy group that may be substituted with a C₁ to C₆ linear alkoxy group; or -OR^{12b}, wherein R^{12b} is a hydrogen atom or a hydroxy group protecting group for nucleic acid synthesis, or
R¹⁰ and R¹¹ together represent a divalent group represented by a formula below: 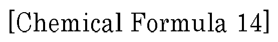 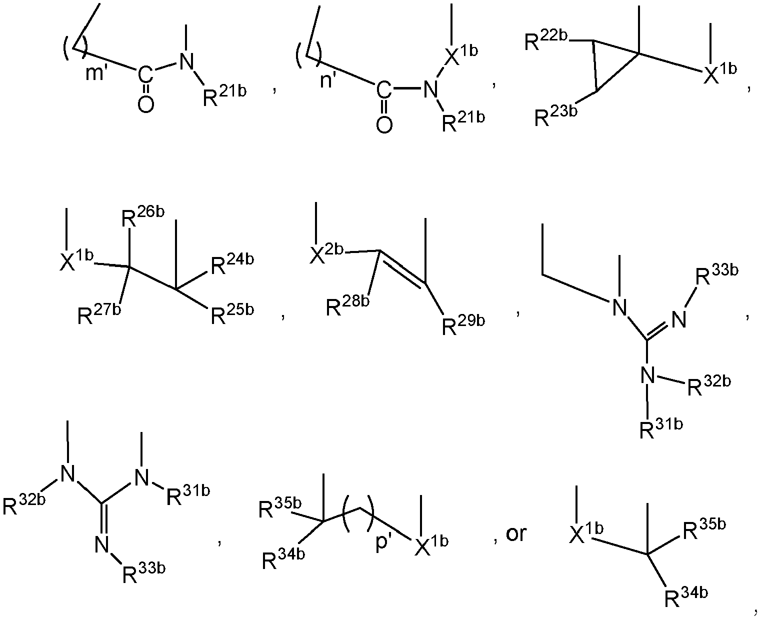
wherein R^{21b} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R^{22b} and R^{23b} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22b} and R^{23b} together represent -(CH₂)_{q2}-, wherein q2 is an integer from 2 to 5;
R^{24b} and R^{25b} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24b} and R^{25b} together represent =C(R^{36b})R^{37b}, wherein R^{36b} and R^{37b} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26b} and R^{27b} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28b} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29b} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31b}, R^{32b}, and R^{33b} are each independently a hydrogen atom, a C₁ to C₆ alkyl group that may be branched or form a ring, or an amino group protecting group for nucleic acid synthesis;
R^{34b} and R^{35b} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m' is an integer from 0 to 2;
   n' is an integer of 0 or 1;
   p' is an integer of 0 or 1;
   X^{1b} is an oxygen atom, a sulfur atom, or an amino group; and
   X^{2b} is an oxygen atom or a sulfur atom; and
M¹ represents a single bond or a formula below: 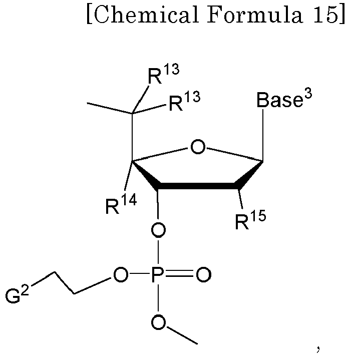
wherein Base³ represents a purin-9-yl group that may have any one or more substituents selected from the α group, or a 2-oxo-1,2-dihydropyrimidin-1-yl group that may have any one or more substituents selected from the α group;
G² is a cyano group or a nitro group;
R¹³ is a C₁ or C₂ alkyl group atoms that may be substituted with a halogen atom;
R¹⁴ is a hydrogen atom, and R¹⁵ is a hydrogen atom or a halogen atom; a C₁ to C₆ linear alkoxy group that may be substituted with a C₁ to C₆ linear alkoxy group; or -OR^{12c}, wherein R^{12c} is a hydrogen atom or a hydroxy group protecting group for nucleic acid synthesis, or
R¹⁴ and R¹⁵ together represent a divalent group represented by a formula below: 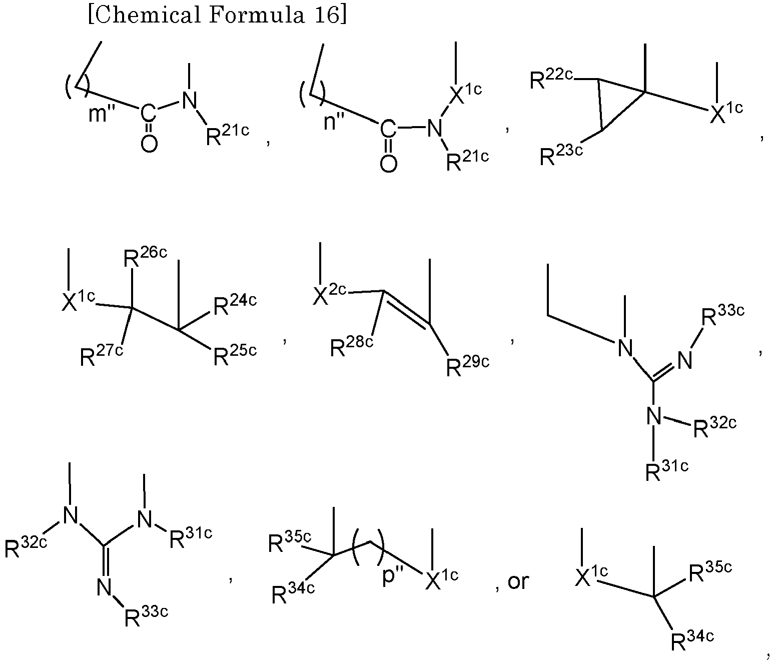
wherein R^{21c} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R^{22c} and R^{23c} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22c} and R^{23c} together represent -(CH₂)_{q3}-, wherein q3 is an integer from 2 to 5;
R^{24c} and R^{25c} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24c} and R^{25c} together represent =C(R^{36c})R^{37c}, wherein R^{36c} and R^{37c} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26c} and R^{27c} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28c} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29c} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31c}, R^{32c}, and R^{33c} are each independently a hydrogen atom, a C₁ to C₆ alkyl group that may be branched or form a ring, or an amino group protecting group for nucleic acid synthesis;
R^{34c} and R^{35c} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m" is an integer from 0 to 2;
   n" is an integer of 0 or 1;
   p" is an integer of 0 or 1;
   X^{1c} is an oxygen atom, a sulfur atom, or an amino group; and
   X^{2c} is an oxygen atom or a sulfur atom.

### Effects of the Invention

According to the present invention, a novel 5'-modified nucleoside and a nucleotide using the same are provided. The 5'-modified nucleoside of the present invention is also usable as a substitute for a phosphorothioate-modified nucleic acid, which has a risk of, for example, accumulation in a specific organ. The 5'-modified nucleoside of the present invention also has excellent industrial productivity because a diastereomer separation step is not involved in the production process thereof.

### Brief Description of Drawings

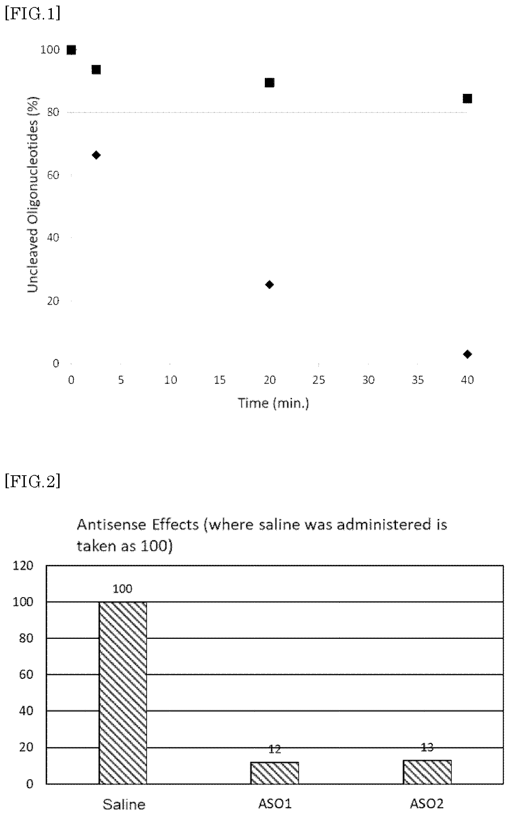

FIG. 1 is a graph showing changes in the percentage of uncleaved oligonucleotides over time when different types of oligonucleotides having the sequence of 5'-TTTTTTTTTX-3' were treated with 3'-exonuclease.
FIG. 2 is a graph showing the abundances of mRNA of a target gene NR3C1 in mouse livers in the cases where test oligonucleotides ASO1 and ASO2 were administered and in the case where saline was administered, and shows antisense effects of the different types of oligonucleotides as relative values of the mRNA abundance, where the mRNA abundance in the case where saline was administered is taken as 100.

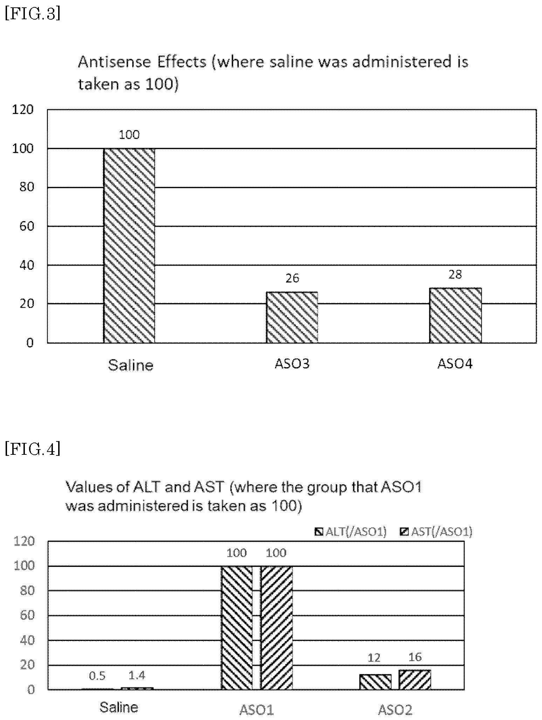

FIG. 3 is a graph showing the abundances of mRNA of the target gene NR3C1 in mouse livers in the cases where test oligonucleotides ASO3 and ASO4 were administered and in the case where saline was administered, and shows antisense effects of the different types of oligonucleotides as relative values of the mRNA abundance, where the mRNA abundance in the case where saline was administered is taken as 100.
FIG. 4 is a graph showing the activities of aspartate transaminase (AST) and alanine transaminase (ALT) in blood in the cases where the test oligonucleotides ASO1 and ASO2 were administered and in the case where saline was administered, and shows the activities as relative values of the AST and ALT, where the ALT value and the AST value in the case where ASO1 was administered are taken as 100.

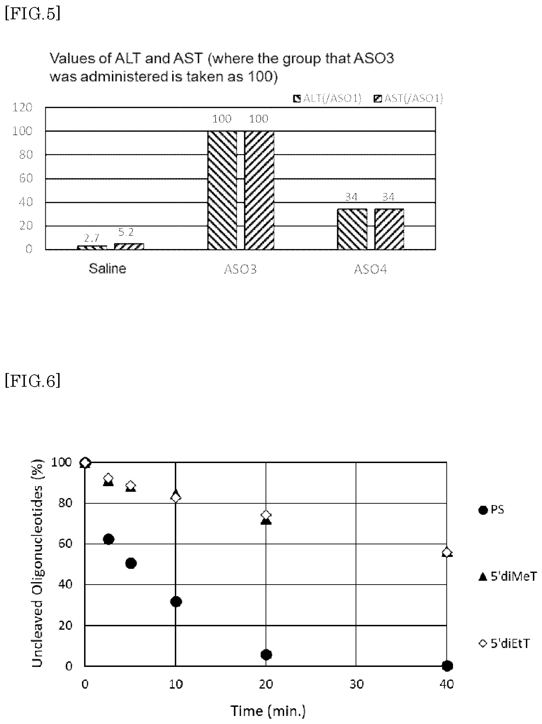

FIG. 5 is a graph showing the activities of aspartate transaminase (AST) and alanine transaminase (ALT) in blood in the cases where the test oligonucleotides ASO3 and ASO4 were administered and in the case where saline was administered, and shows the activities as relative values of the AST and ALT, where the ALT value and the AST value in the case where ASO3 was administered are taken as 100.
FIG. 6 is a graph showing changes in the percentage of uncleaved oligonucleotides over time when different types of oligonucleotides having the sequence of 5'-TTTTTTTTTX-3' were treated with 3'-exonuclease.

### Description of Embodiments

The following definitions shall apply throughout the specification.

The term "C₁ to C₆ linear alkyl group" as used herein refers to any linear alkyl group having 1 to 6 carbon atoms, and specifically to a methyl group, an ethyl group, an n-propyl group, an n-butyl group, an n-pentyl group, or an n-hexyl group. On the other hand, the term "C₁ to C₆ alkyl group" refers to any linear, branched, or cyclic alkyl group having 1 to 6 carbon atoms.

The term "C₁ to C₆ linear alkoxy group" as used herein encompasses alkoxy groups including any linear alkyl groups having 1 to 6 carbon atoms. Examples thereof include a methoxy group, an ethoxy group, and an n-propoxy group. On the other hand, the term "C₁ to C₆ alkoxy group" refers to any linear, branched, or cyclic alkoxy group having 1 to 6 carbon atoms. The term "C₁ to C₆ linear alkoxy group that may be substituted with a C₁ to C₆ linear alkoxy group" refers to the "C₁ to C₆ linear alkoxy group" as well as an alkyl group obtained by substituting one or more hydrogen atoms included in the "C₁ to C₆ linear alkoxy group" with another or other "C₁ to C₆ linear alkoxy group" that may be the same or different. Examples of such "C₁ to C₆ linear alkoxy group that may be substituted with a C₁ to C₆ linear alkoxy group" include a methoxy group, an ethoxy group, an n-propoxy group, a methoxymethoxy group, an ethoxymethoxy group, an n-propoxymethoxy group, a methoxyethoxy group (e.g., a 2-methoxyethoxy group), an ethoxyethoxy group (e.g., a 2-ethoxyethoxy group), and an n-propoxyethoxy group.

The term "C₁ to C₆ cyanoalkoxy group" as used herein refers to a group obtained by substituting at least one hydrogen atom included in any linear, branched, or cyclic alkoxy group having 1 to 6 carbon atoms with a cyano group.

The term "C₁ to C₆ linear alkylthio group" as used herein encompasses alkylthio groups including any linear alkyl groups having 1 to 6 carbon atoms. Examples thereof include a methythio group, an ethylthio group, and an n-propylthio group. On the other hand, the term "C₁ to C₆ alkylthio group" refers to any linear, branched, or cyclic alkylthio group having 1 to 6 carbon atoms.

The term "C₁ to C₆ linear alkylamino group" as used herein encompasses alkylamino groups including one or two alkylamino groups with any linear alkyl group having 1 to 6 carbon atoms. Examples thereof include a methylamino group, a dimethylamino group, an ethylamino group, a methylethylamino group, and a diethylamino group.

The term "C₁ to C₇ alkyl group that may be branched or form a ring" as used herein encompasses any linear alkyl groups having 1 to 7 carbon atoms, any branched alkyl groups having 3 to 7 carbon atoms, and any cyclic alkyl groups having 3 to 7 carbon atoms. Such groups may also be referred to merely as "lower alkyl groups". Examples of any linear alkyl groups having 1 to 7 carbon atoms include a methyl group, an ethyl group, an n-propyl group, an n-butyl group, an n-pentyl group, an n-hexyl group, and an n-heptyl group; examples of any branched alkyl groups having 3 to 7 carbon atoms include an isopropyl group, an isobutyl group, a tert-butyl group, and an isopentyl group; and examples of any cyclic alkyl groups having 3 to 7 carbon atoms include a cyclobutyl group, a cyclopentyl group, and a cyclohexyl group.

The term "C₂ to C₇ alkenyl group that may be branched or form a ring" as used herein encompasses any linear alkenyl groups having 2 to 7 carbon atoms, any branched alkenyl groups having 3 to 7 carbon atoms, and any cyclic alkenyl groups having 3 to 7 carbon atoms. Such groups may also be referred to merely as "lower alkenyl groups". Examples of any linear alkenyl groups having 2 to 7 carbon atoms include an ethenyl group, a 1-propenyl group, a 2-propenyl group, a 1-butenyl group, a 2-butenyl group, a 1-pentenyl group, a 2-pentenyl group, a 3-pentenyl group, a 4-pentenyl group, and a 1-hexenyl group; examples of any branched alkenyl groups having 3 to 7 carbon atoms include an isopropenyl group, a 1-methyl-1-propenyl group, a 1-methyl-2-propenyl group, a 2-methyl-1-propenyl group, a 2-methyl-2-propenyl group, and a 1-methyl-2-butenyl group; and examples of any cyclic alkenyl groups having 3 to 7 carbon atoms include a cyclobutenyl group, a cyclopentenyl group, and a cyclohexenyl group.

The term "C₃ to C₁₀ aryl group that may contain a heteroatom" as used herein encompasses any aryl groups having 6 to 10 carbon atoms that are constituted by only a hydrocarbon, and any heteroaryl groups having 3 to 12 carbon atoms obtained by substituting at least one carbon atom included in the ring structure of the above-mentioned aryl groups with a heteroatom (e.g., a nitrogen atom, an oxygen atom, and a sulfur atom, and a combination thereof). Examples of the aryl groups having 6 to 10 carbon atoms include a phenyl group, a naphthyl group, an indenyl group, and an azulenyl group; and examples of any heteroaryl groups having 3 to 12 carbon atoms include a pyridyl group, a pyrrolyl group, a quinolyl group, an indolyl group, an imidazolyl group, a furyl group, and a thienyl group.

Examples of the term "aralkyl group with a C₃ to C₁₂ aryl moiety that may contain a heteroatom" as used herein include a benzyl group, a phenethyl group, a naphthylmethyl group, a 3-phenylpropyl group, a 2-phenylpropyl group, a 4-phenylbutyl group, a 2-phenylbutyl group, a pyridylmethyl group, an indolylmethyl group, a furylmethyl group, a thienylmethyl group, a pyrrolylmethyl group, a 2-pyridylethyl group, a 1-pyridylethyl group, and a 3-thienylpropyl group.

Examples of the term "acyl group" as used herein include aliphatic acyl groups and aromatic acyl groups. Specifically, examples of the aliphatic acyl groups include alkylcarbonyl groups such as a formyl group, an acetyl group, a propionyl group, a butyryl group, an isobutyryl group, a pentanoyl group, a pivaloyl group, a valeryl group, an isovaleryl group, an octanoyl group, a nonanoyl group, a decanoyl group, a 3-methylnonanoyl group, a 8-methylnonanoyl group, a 3-ethyloctanoyl group, a 3,7-dimethyloctanoyl group, an undecanoyl group, a dodecanoyl group, a tridecanoyl group, a tetradecanoyl group, a pentadecanoyl group, a hexadecanoyl group, a 1-methylpentadecanoyl group, a 14-methylpentadecanoyl group, a 13,13-dimethyltetradecanoyl group, a heptadecanoyl group, a 15-methylhexadecanoyl group, an octadecanoyl group, a 1-methylheptadecanoyl group, a nonadecanoyl group, an eicosanoyl group, and a heneicosanoyl group; carboxylated alkylcarbonyl groups such as a succinoyl group, a glutaroyl group, and an adipoyl group; halogeno lower-alkyl-carbonyl groups such as a chloroacetyl group, a dichloroacetyl group, a trichloroacetyl group, and a trifluoroacetyl group; lower-alkoxy-lower-alkyl-carbonyl groups such as a methoxyacetyl group; and unsaturated alkylcarbonyl groups such as an (E)-2-methyl-2-butenoyl group. Examples of the aromatic acyl groups include arylcarbonyl groups such as a benzoyl group, an α-naphthoyl group, and a β-naphthoyl group; halogeno arylcarbonyl groups such as a 2-bromobenzoyl group and a 4-chlorobenzoyl group; low-alkylated arylcarbonyl groups such as a 2,4,6-trimethylbenzoyl group and a 4-toluoyl group; low-alkoxylated arylcarbonyl groups such as a 4-anisoyl group: carboxylated arylcarbonyl groups such as a 2-carboxybenzoyl group, a 3-carboxybenzoyl group, and a 4-carboxybenzoyl group; nitrated arylcarbonyl groups such as a 4-nitrobenzoyl group and a 2-nitrobenzoyl group; low-alkoxycarbonylated arylcarbonyl groups such as a 2-(methoxycarbonyl)benzoyl group; and arylated arylcarbonyl groups such as a 4-phenylbenzoyl group. A formyl group, an acetyl group, a propionyl group, a butyryl group, an isobutyryl group, a pentanoyl group, a pivaloyl group, and a benzoyl group are favorable.

Examples of the term "silyl group" as used herein include tri-lower-alkyl-silyl groups such as a trimethylsilyl group, a triethylsilyl group, an isopropyldimethylsilyl group, a t-butyldimethylsilyl group, a methyldiisopropylsilyl group, a methyldi-t-butylsilyl group, and a triisopropylsilyl group; and tri-lower-alkyl-silyl groups that have undergone substitution by one or two aryl groups, such as a diphenylmethylsilyl group, a butyldiphenylbutylsilyl group, a diphenylisopropylsilyl group, and a phenyldiisopropylsilyl group. A trimethylsilyl group, a triethylsilyl group, a triisopropylsilyl group, a t-butyldimethylsilyl group, and a t-butyldiphenylsilyl group are favorable, and a trimethylsilyl group is more favorable.

Examples of the term "halogen atom" as used herein include a fluorine atom, a chlorine atom, a bromine atom, and an iodine atom. A fluorine atom or a chlorine atom is favorable.

"Protecting groups" in the terms "amino group protecting group for nucleic acid synthesis", "hydroxy group protecting group for nucleic acid synthesis", "hydroxy group protected by a protecting group for nucleic acid synthesis", "phosphate group protected by a protecting group for nucleic acid synthesis", and "mercapto group protected by a protecting group for nucleic acid synthesis" as used herein are not particularly limited as long as they can stably protect an amino group, a hydroxy group, a phosphate group, or a mercapto group during nucleic acid synthesis. Specifically, the protecting groups are stable under an acidic or neutral condition and can be cleaved using chemical techniques such as hydrogenolysis, hydrolysis, electrolysis, and photolysis. Examples of such protecting groups include lower alkyl groups, lower alkenyl groups, acyl groups, tetrahydropyranyl or tetrahydrothiopyranyl groups, tetrahydrofuranyl or tetrahydrothiofuranyl groups, silyl groups, lower-alkoxy-methyl groups, low-alkoxylated lower-alkoxy-methyl groups, halogeno lower-alkoxy-methyl groups, low-alkoxylated ethyl groups, halogenated ethyl groups, methyl groups that have undergone substitution by 1 to 3 aryl groups, "methyl groups that have undergone substitution by 1 to 3 aryl groups in which an aryl ring has undergone substitution by a lower alkyl group, lower alkoxy group, halogen atom, or cyano group", lower-alkoxy-carbonyl groups, "aryl groups that have undergone substitution by a halogen atom, lower alkoxy group, or nitro group", "lower-alkoxy-carbonyl groups that have undergone substitution by a halogen atom or tri-lower-alkyl-silyl group", alkenyloxycarbonyl groups, and "aralkyloxycarbonyl groups in which an aryl ring may be substituted with a lower alkoxy group or nitro group".

More specific examples of the tetrahydropyranyl or tetrahydrothiopyranyl groups include a tetrahydropyran-2-yl group, a 3-bromotetrahydropyran-2-yl group, a 4-methoxytetrahydropyran-4-yl group, a tetrahydrothiopyran-4-yl group, and a 4-methoxytetrahydrothiopyran-4-yl group. Examples of the tetrahydrofuranyl or tetrahydrothiofuranyl groups include a tetrahydrofuran-2-yl group and a tetrahydrothiofuran-2-yl group. Examples of the lower-alkoxy-methyl groups include a methoxymethyl group, a 1,1-dimethyl-1-methoxymethyl group, an ethoxymethyl group, a propoxymethyl group, an isopropoxymethyl group, a butoxymethyl group, and a t-butoxymethyl group. An example of the low-alkoxylated lower-alkoxy-methyl groups is a 2-methoxyethoxymethyl group. Examples of the halogeno lower-alkoxy-methyl groups include a 2,2,2-trichloroethoxymethyl group and a bis(2-chloroethoxy)methyl group. Examples of the low-alkoxylated ethyl groups include a 1-ethoxyethyl group and a 1-(isopropoxy)ethyl group. An example of the halogenated ethyl groups is a 2,2,2-trichloroethyl group. Examples of the methyl groups that have undergone substitution by 1 to 3 aryl groups include a benzyl group, an α-naphthylmethyl group, a β-naphthylmethyl group, a diphenylmethyl group, a triphenylmethyl group, an α-naphthyldiphenylmethyl group, and a 9-anthrylmethyl group. Examples of the "methyl groups that have undergone substitution by 1 to 3 aryl groups in which an aryl ring has undergone substitution by a lower alkyl group, lower alkoxy group, halogen atom, or cyano group" include a 4-methylbenzyl group, a 2,4,6-trimethylbenzyl group, a 3,4,5-trimethylbenzyl group, a 4-methoxybenzyl group, a 4-methoxyphenyldiphenylmethyl group, a 4,4'-dimethoxytriphenylmethyl group, a 2-nitrobenzyl group, a 4-nitrobenzyl group, a 4-chlorobenzyl group, a 4-bromobenzyl group, and a 4-cyanobenzyl group. Examples of the lower-alkoxy-carbonyl groups include a methoxycarbonyl group, an ethoxycarbonyl group, a t-butoxycarbonyl group, and an isobutoxycarbonyl group. Examples of the "aryl groups that have undergone substitution by a halogen atom, lower alkoxy group, or nitro group" include a 4-chlorophenyl group, a 2-fluorophenyl group, a 4-methoxyphenyl group, a 4-nitrophenyl group, and a 2,4-dinitrophenyl group. Examples of the "lower-alkoxy-carbonyl groups that have undergone substitution by a halogen atom or tri-lower-alkyl-silyl group" include a 2,2,2-trichloroethoxycarbonyl group and 2-trimethylsilylethoxycarbonyl group. Examples of the alkenyloxycarbonyl groups include a vinyloxycarbonyl group and an aryloxycarbonyl group. Examples of the "aralkyloxycarbonyl groups in which an aryl ring may be substituted with a lower alkoxy group or nitro group" include a benzyloxycarbonyl group, a 4-methoxybenzyloxycarbonyl group, a 3,4-dimethoxybenzyloxycarbonyl group, a 2-nitrobenzyloxycarbonyl group, and a 4-nitrobenzyloxycarbonyl group.

In an embodiment, examples of the "hydroxy group protecting group for nucleic acid synthesis" include aliphatic acyl groups, aromatic acyl groups, methyl groups that have undergone substitution by 1 to 3 aryl groups, "methyl groups that have undergone substitution by 1 to 3 aryl groups in which an aryl ring has undergone substitution by a lower alkyl, lower alkoxy, halogen, or cyano group", and silyl groups. Alternatively, in an embodiment, examples of the "hydroxy group protecting group for nucleic acid synthesis" include an acetyl group, a benzoyl group, a benzyl group, a p-methoxybenzoyl group, a dimethoxytrityl group, a monomethoxytrityl group, a tert-butyldiphenylsilyl group, a tert-butyldimethylsilyl (TBDMS) group, a [(triisopropylsilyl)oxy]methyl (TOM) group, a [(2-nitrobenzyl)oxy]methyl (NBOM) group, a bis(acetoxyethoxy)methyl ether (ACE) group, a tetrahydro-4-methoxy-2H-pyran-2-yl (Mthp) group, a 1-(2-cyanoethoxy)ethyl (CEE) group, a 2-cyanoethoxymethyl (CEM) group, a tert-butyldithiomethyl (DTM) group, a 2-(4-tolylsulfonyl)ethoxymethyl (TEM) group, and a 4-(N-dichloroacetyl-N-methylamino)benzyloxymethyl (4-MABOM) group.

In an embodiment, examples of the protecting group used for the "hydroxy group protected by a protecting group for nucleic acid synthesis" include aliphatic acyl groups, aromatic acyl groups, "methyl groups that have undergone substitution by 1 to 3 aryl groups", "aryl groups that have undergone substitution by a halogen atom, lower alkoxy group, or nitro group", lower alkyl groups, and lower alkenyl groups. Alternatively, in an embodiment, examples of the protecting group used for the "hydroxy group protected by a protecting group for nucleic acid synthesis" include a benzoyl group, a benzyl group, a 2-chlorophenyl group, a 4-chlorophenyl group, and a 2-propenyl group.

In an embodiment, examples of the "amino group protecting group for nucleic acid synthesis" include acyl groups, and a benzoyl group is favorable.

In an embodiment, examples of the "protecting group" used for the "phosphate group protected by a protecting group for nucleic acid synthesis" include lower alkyl groups, lower alkyl groups that have undergone substitution by a cyano group, aralkyl groups, "aralkyl groups in which an aryl ring has undergone substitution by a nitro group or halogen atom", and "aryl groups that have undergone substitution by a lower alkyl group, halogen atom, or nitro group". Alternatively, in an embodiment, examples of the "protecting group" used for the "phosphate group protected by a protecting group for nucleic acid synthesis" include a 2-cyanoethyl group, a 2,2,2-trichloroethyl group, a benzyl group, a 2-chlorophenyl group, and a 4-chlorophenyl group.

In an embodiment, examples of the "protecting group" used for the "mercapto group protected by a protecting group for nucleic acid synthesis" include aliphatic acyl groups and aromatic acyl groups, and a benzoyl group is favorable.

In this specification, among groups represented by -P(R⁴)R⁵, wherein R⁴ and R⁵ each independently represent a hydroxy group, a hydroxy group protected by a protecting group for nucleic acid synthesis, a mercapto group, a mercapto group protected by a protecting group for nucleic acid synthesis, an amino group, a C₁ to C₆ alkoxy group, a C₁ to C₆ alkylthio group, a C₁ to C₆ cyanoalkoxy group, or a dialkylamino group having a C₁ to C₆ alkyl group, a group in which R⁴ is OR^{4a} and R⁵ is NR^{5a} is referred to as a "phosphoramidite group", wherein an example of R^{4a} is a C₁ to C₆ cyanoalkoxy group, and an example of R^{5a} is a C₁ to C₆ alkyl group. Favorable examples of the phosphoramidite group include a group represented by a formula -P(OC₂H₄CN)(N(iPr)₂) and a group represented by a formula -P(OCH₃)(N(iPr)₂). In these formulae, iPr represents an isopropyl group.

The terms "nucleoside" and "nucleoside analogue" as used herein refer to non-naturally occurring nucleosides of "nucleosides" in which a purine base or a pyrimidine base binds to sugar, as well as those in which a heteroaromatic ring or an aromatic hydrocarbon ring other than purine and pyrimidine that can serve as a substitute for a purine or pyrimidine base binds to sugar.

The terms "artificial oligonucleotide" and "oligonucleotide analogue" as used herein refer to non-naturally occurring derivatives of "oligonucleotides" in which, for example, two to fifty of the same or different "nucleosides" or "nucleoside analogues" are bound via phosphodiester bonds. Favorable examples of such analogues include sugar derivatives with sugar moieties modified; thioated derivatives with phosphate diester moieties thioated; esters with terminal phosphate moieties esterificated; and amides in which amino groups on purine bases are amidated. The sugar derivatives with sugar moieties modified are more favorable.

The term "salt thereof" as used herein refers to a salt of a compound represented by the formula (I) or (II) of the present invention. Examples of such salt include metal salts including alkali metal salts such as sodium salts, potassium salts, and lithium salts, alkali earth metal salts such as calcium salts and magnesium salts, and aluminum salts, iron salts, zinc salts, copper salts, nickel salts, and cobalt salts; amine salts including inorganic salts such as ammonium salts, and organic salts such as t-octylamine salts, dibenzylamine salts, morpholine salts, glucosamine salts, phenylglycine alkylester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts including halide hydroacid salts such as hydrofluoric acid salts, hydrochloric acid salt, hydrobromic acid salts, and hydroiodic acid salts, nitrates, perchlorates, sulfates, and phosphates; organic acid salts including lower-alkane-sulfonates such as methanesulfonates, trifluoromethanesulfonates, and ethanesulfonates, arylsulfonates such as benzenesulfonates and p-toluenesulfonates, acetates, malates, fumarates, succinates, citrates, tartrates, oxalates and maleates; and amino acid salts such as glycine salts, lysine salts, arginine salts, ornithine salts, glutamates, and aspartates.

The term "pharmacologically acceptable salt thereof" refers to a salt of an oligonucleotide analogue containing at least one nucleoside structure represented by the formula (II) of the present invention. Examples of such salts include metal salts including alkali metal salts such as sodium salts, potassium salts, and lithium salts, alkali earth metal salts such as calcium salts and magnesium salts, and aluminum salts, iron salts, zinc salts, copper salts, nickel salts, and cobalt salts; amine salts including inorganic salts such as ammonium salts, and organic salts such as t-octylamine salts, dibenzylamine salts, morpholine salts, glucosamine salts, phenylglycine alkylester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts including halide hydroacid salts such as hydrofluoric acid salts, hydrochloric acid salt, hydrobromic acid salts, and hydroiodic acid salts, nitrates, perchlorates, sulfates, and phosphates; organic acid salts including lower-alkane-sulfonates such as methanesulfonates, trifluoromethanesulfonates, and ethanesulfonates, arylsulfonates such as benzenesulfonates and p-toluenesulfonates, acetates, malates, fumarates, succinates, citrates, tartrates, oxalates and maleates; and amino acid salts such as glycine salts, lysine salts, arginine salts, ornithine salts, glutamates, and aspartates.

Hereinafter, the present invention will be described in detail.

The 5'-modified nucleoside of the present invention is a compound represented by a formula (I) below or a salt thereof: 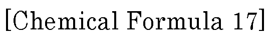 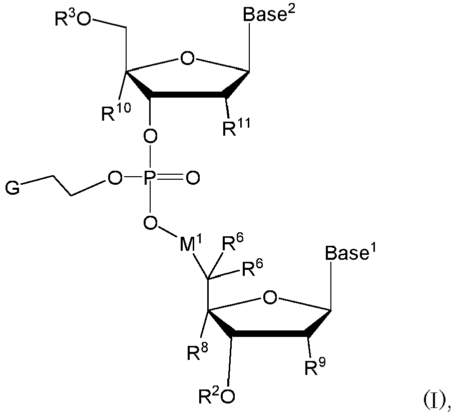 wherein Base¹ and Base² each independently represent a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, a C₁ to C₆ linear alkyl group, a C₁ to C₆ linear alkoxy group, a mercapto group, a mercapto group protected by a protecting group for nucleic acid synthesis, a C₁ to C₆ linear alkylthio group, an amino group, a C₁ to C₆ linear alkylamino group, an amino group protected by a protecting group for nucleic acid synthesis, and halogen atoms;
G is a cyano group or a nitro group;
R² and R³ each independently represent a hydrogen atom, a hydroxy group protecting group for nucleic acid synthesis, a C₁ to C₇ alkyl group that may be branched or form a ring, a C₂ to C₇ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R⁴)R⁵, wherein R⁴ and R⁵ each independently represent a hydroxy group, a hydroxy group protected by a protecting group for nucleic acid synthesis, a mercapto group, a mercapto group protected by a protecting group for nucleic acid synthesis, an amino group, a C₁ to C₆ alkoxy group, a C₁ to C₆ alkylthio group, a C₁ to C₆ cyanoalkoxy group, or a dialkylamino group having a C₁ to C₆ alkyl group;
R⁶ is a C₁ or C₂ alkyl group atoms that may be substituted with a halogen atom;
R⁸ is a hydrogen atom, and R⁹ is a hydrogen atom or a halogen atom; a C₁ to C₆ linear alkoxy group that may be substituted with a C₁ to C₆ linear alkoxy group; or -OR¹², wherein R¹² is a hydrogen atom or a hydroxy group protecting group for nucleic acid synthesis, or
R⁸ and R⁹ together represent a divalent group represented by a formula below: 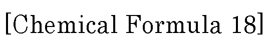 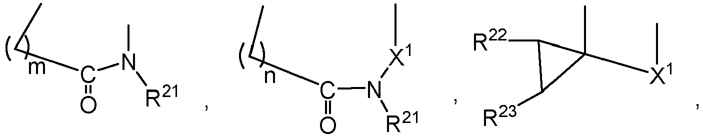 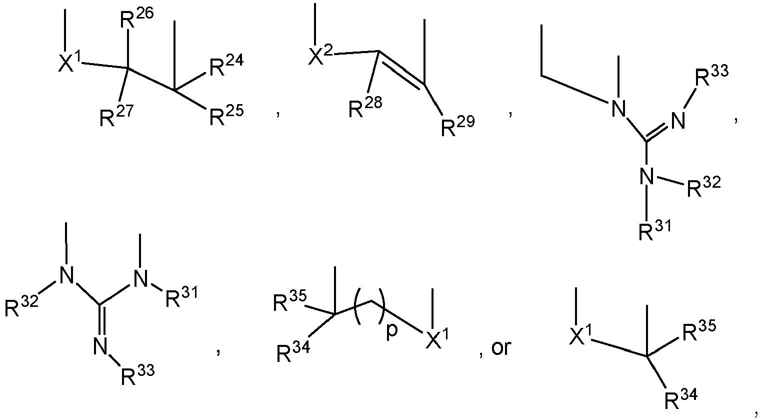 wherein R²¹ is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R²² and R²³ are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R²² and R²³ together represent -(CH₂)_{q1}-, wherein q1 is an integer from 2 to 5;
R²⁴ and R²⁵ are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R²⁴ and R²⁵ together represent =C(R³⁶)R³⁷, wherein R³⁶ and R³⁷ each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R²⁶ and R²⁷ are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁸ is a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁹ is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R³¹, R³², and R³³ are each independently a hydrogen atom, a C₁ to C₆ alkyl group that may be branched or form a ring, or an amino group protecting group for nucleic acid synthesis;
R³⁴ and R³⁵ are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m is an integer from 0 to 2;
   n is an integer of 0 or 1;
   p is an integer of 0 or 1;
   X¹ is an oxygen atom, a sulfur atom, or an amino group; and
   X² is an oxygen atom or a sulfur atom;
R¹⁰ is a hydrogen atom, and R¹¹ is a hydrogen atom or a halogen atom; a C₁ to C₆ linear alkoxy group that may be substituted with a C₁ to C₆ linear alkoxy group; or -OR^{12b}, wherein R^{12b} is a hydrogen atom or a hydroxy group protecting group for nucleic acid synthesis, or
R¹⁰ and R¹¹ together represent a divalent group represented by a formula below: 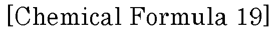 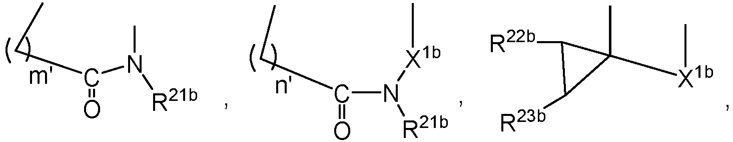 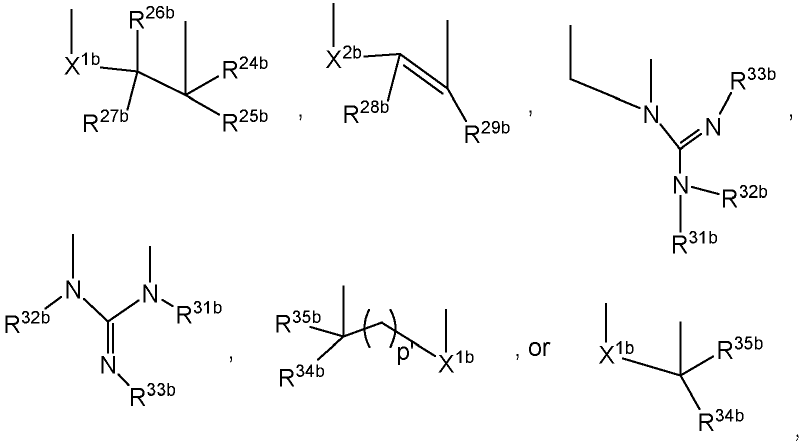 wherein R^{21b} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R^{22b} and R^{23b} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22b} and R^{23b} together represent -(CH₂)_{q2}-, wherein q2 is an integer from 2 to 5;
R^{24b} and R^{25b} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24b} and R^{25b} together represent =C(R^{36b})R^{37b}, wherein R^{36b} and R^{37b} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26b} and R^{27b} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28b} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29b} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31b}, R^{32b}, and R^{33b} are each independently a hydrogen atom, a C₁ to C₆ alkyl group that may be branched or form a ring, or an amino group protecting group for nucleic acid synthesis;
R^{34b} and R^{35b} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m' is an integer from 0 to 2;
   n' is an integer of 0 or 1;
   p' is an integer of 0 or 1;
   X^{1b} is an oxygen atom, a sulfur atom, or an amino group; and
   X^{2b} is an oxygen atom or a sulfur atom; and
M¹ represents a single bond or a formula below: 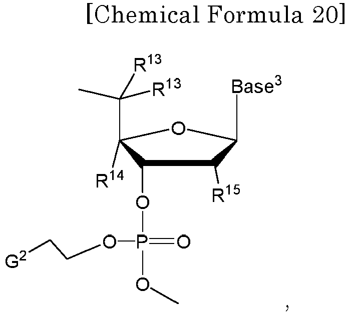 wherein Base³ represents a purin-9-yl group that may have any one or more substituents selected from the α group, or a 2-oxo-1,2-dihydropyrimidin-1-yl group that may have any one or more substituents selected from the α group;
G² is a cyano group or a nitro group;
R¹³ is a C₁ or C₂ alkyl group atoms that may be substituted with a halogen atom;
R¹⁴ is a hydrogen atom, and R¹⁵ is a hydrogen atom or a halogen atom; a C₁ to C₆ linear alkoxy group that may be substituted with a C₁ to C₆ linear alkoxy group; or -OR^{12c}, wherein R^{12c} is a hydrogen atom or a hydroxy group protecting group for nucleic acid synthesis, or
R¹⁴ and R¹⁵ together represent a divalent group represented by a formula below: 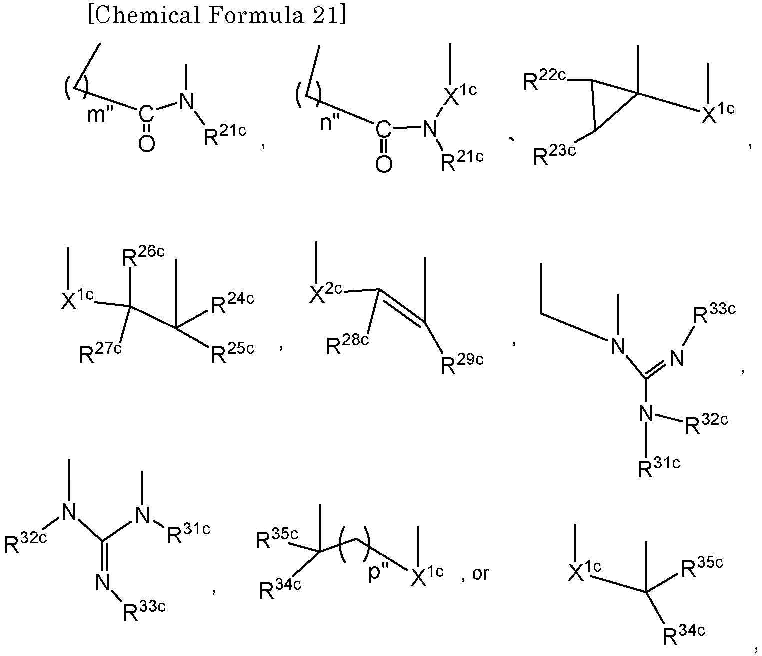 wherein R^{21c} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R^{22c} and R^{23c} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22c} and R^{23c} together represent -(CH₂)_{q3}-, wherein q3 is an integer from 2 to 5;
R^{24c} and R^{25c} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24c} and R^{25c} together represent =C(R^{36c})R^{37c}, wherein R^{36c} and R^{37c} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26c} and R^{27c} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28c} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29c} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31c}, R^{32c}, and R^{33c} are each independently a hydrogen atom, a C₁ to C₆ alkyl group that may be branched or form a ring, or an amino group protecting group for nucleic acid synthesis;
R^{34c} and R^{35c} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m" is an integer from 0 to 2;
   n" is an integer or 1;
   p" is an integer or 1;
   X^{1c} is an oxygen atom, a sulfur atom, or an amino group; and
   X^{2c} is an oxygen atom or a sulfur atom.

In the formula (I) above, Base¹, Base², and Base³ may be the same or different. For example, Base¹, Base², and Base³ are each independently a purine base (i.e., a purin-9-yl group) or a pyrimidine base (i.e., a 2-oxo-1,2-dihydropyrimidin-1-yl group). These bases may have any one or more substituents selected from the α group consisting of a hydroxy group, a C₁ to C₆ linear alkyl group, a C₁ to C₆ linear alkoxy group, a mercapto group, a C₁ to C₆ linear alkylthio group, an amino group, a C₁ to C₆ linear alkylamino group, and halogen atoms.

Specific examples of Base¹, Base², and Base³ above include an adeninyl group, a guaninyl group, a cytosinyl group, an uracinyl group, and a thyminyl group, and a 6-aminopurin-9-yl group, a 2,6-diaminopurin-9-yl group, a 2-amino-6-chloropurin-9-yl group, a 2-amino-6-fluoropurin-9-yl group, a 2-amino-6-bromopurin-9-yl group, a 2-amino-6-hydroxypurin-9-yl group, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl group, a 4-amino-2-oxo-5-fluoro-1,2-dihydropyrimidin-1-yl group, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl group, a 2-oxo-4-hydroxy-5-methyl-1,2-dihydropyrimidin-1-yl group, and a 4-amino-5-methyl-2-oxo-1,2-dihydropyrimidin-1-yl group.

Alternatively, for the purpose of introducing the 5'-modified nucleoside of the present invention into a nucleic acid drug, as Base¹, Base², and Base³, groups represented by structural formulae below: 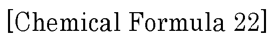 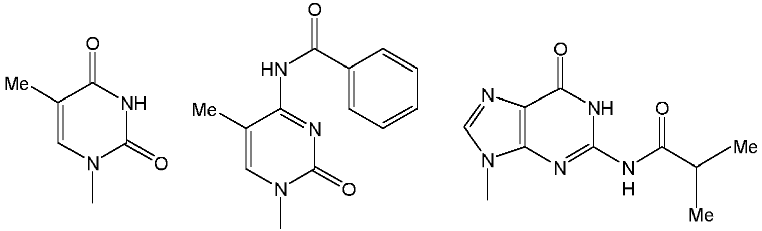 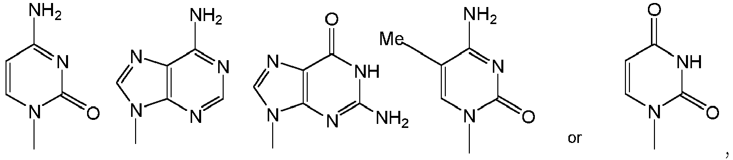 as well as a 2-oxo-4-hydroxy-5-methyl-1,2-dihydropyrimidin-1-yl group, a 2-oxo-4-amino-1,2-dihydropyrimidin-1-yl group, a 6-aminopurin-9-yl group, a 2-amino-6-hydroxypurin-9-yl group, a 4-amino-5-methyl-2-oxo-1,2-dihydropyrimidin-1-yl group, and a 2-oxo-4-hydroxy-1,2-dihydropyrimidin-1-yl group are favorable. It is preferable that a hydroxy group and an amino group included in the above-mentioned groups serving as Base¹, Base², and Base³ are protected by a protecting group during oligonucleotide synthesis.

The 5'-modified nucleoside of the present invention has a structure represented by the formula (I) as described above, that is, a dimer or trimer structure formed by combining predetermined furanose rings and represented by the formula (I') or (I") below: 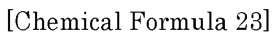 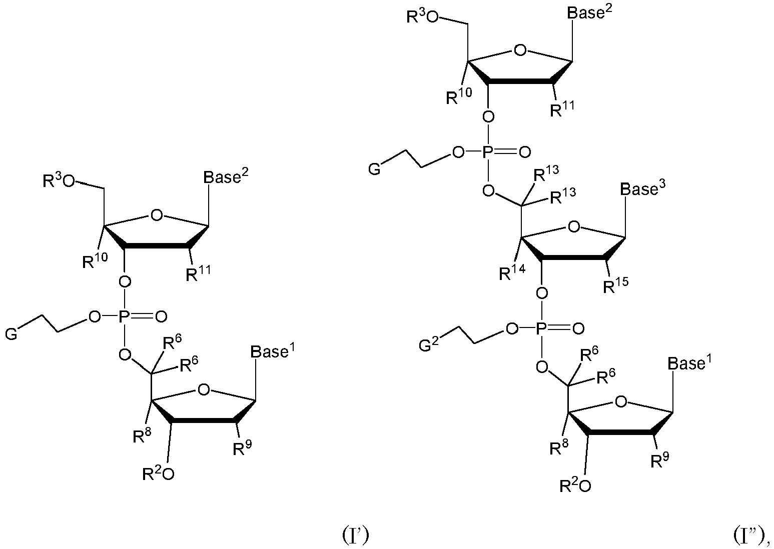 wherein Base¹, Base², G, R², R³, R⁶, R⁸, R⁹, R¹⁰, and R¹¹ in the formula (I') are as defined for the formula (I) above, and Base¹, Base², Base³, G, G², R², R³, R⁶, R⁸, R⁹, R¹⁰, R¹¹, R¹³, R¹⁴, and R¹⁵ in the formula (I") are as defined for the formula (I) above. Here, two groups R⁶ and two hydrogen atoms are bound to the 5' position of the respective furanose rings constituting the dimer represented by the formula (I'). Two groups R⁶, two hydrogen atoms, and two groups R¹³ are bound to the 5' position of the respective furanose rings constituting the trimer represented by the formula (I"). With such structure, the 5'-modified nucleoside of the present invention does not have a diastereomer structure at the 5' position of each pyranose ring included in the same molecule, and therefore, compared with conventional artificial nucleic acids obtained by introducing a substituent into the 5' position, the separation of diastereomers during synthesis is no longer necessary.

In an embodiment, R⁶ in the dimer represented by the formula (I') above is a methyl group. In an embodiment, R⁶ and R¹³ in the trimer represented by the formula (I") above are both methyl groups.

In an embodiment, R⁸, R⁹, R¹⁰, and R¹¹ in the formula (I') above are all hydrogen atoms. In an embodiment, R⁸, R⁹, R¹⁰, R¹¹, R¹⁴, and R¹⁵ in the formula (I") above are all hydrogen atoms.

The 5'-modified nucleoside of the present invention represented by the formula (I') above improves the nuclease-resistant ability of an oligonucleotide, which will be described later, because two groups R⁶ and two hydrogen atoms are introduced into the 5' position of the respective furanose rings constituting the formula (I'). Furthermore, the 5'-modified nucleoside of the present invention represented by the formula (I") above improves the nuclease-resistant ability of an oligonucleotide, which will be described later, because two groups R⁶, two hydrogen atoms, and two groups R¹³ are introduced into the 5' position of the respective furanose rings constituting the formula (I").

In the present invention, an oligonucleotide can be easily produced by using such 5'-modified nucleoside represented by the formula (I), or the formula (I') or (I"), and using, for example, an amidite method that is well known in the art, or triphosphorylation such as that described in M. Kuwahara et al., Nucleic Acids Res., 2008, Vol. 36, No. 13, pp. 4257-4265.

The oligonucleotide below or a pharmacologically acceptable salt thereof (hereinafter, these may be collectively referred to as "oligonucleotide of the present invention") contains at least one nucleoside structure represented by a formula (II): 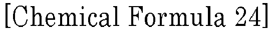 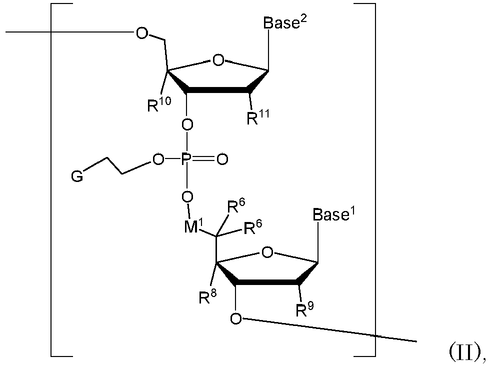 wherein Base¹ and Base² each independently represent a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, a C₁ to C₆ linear alkyl group, a C₁ to C₆ linear alkoxy group, a mercapto group, a mercapto group protected by a protecting group for nucleic acid synthesis, a C₁ to C₆ linear alkylthio group, an amino group, a C₁ to C₆ linear alkylamino group, an amino group protected by a protecting group for nucleic acid synthesis, and halogen atoms;
G is a cyano group or a nitro group;
R⁶ is a C₁ or C₂ alkyl group atoms that may be substituted with a halogen atom;
R⁸ is a hydrogen atom, and R⁹ is a hydrogen atom or a halogen atom; a C₁ to C₆ linear alkoxy group that may be substituted with a C₁ to C₆ linear alkoxy group; or -OR¹², wherein R¹² is a hydrogen atom or a hydroxy group protecting group for nucleic acid synthesis, or
R⁸ and R⁹ together represent a divalent group represented by a formula below (CR01a) to (CR11a): 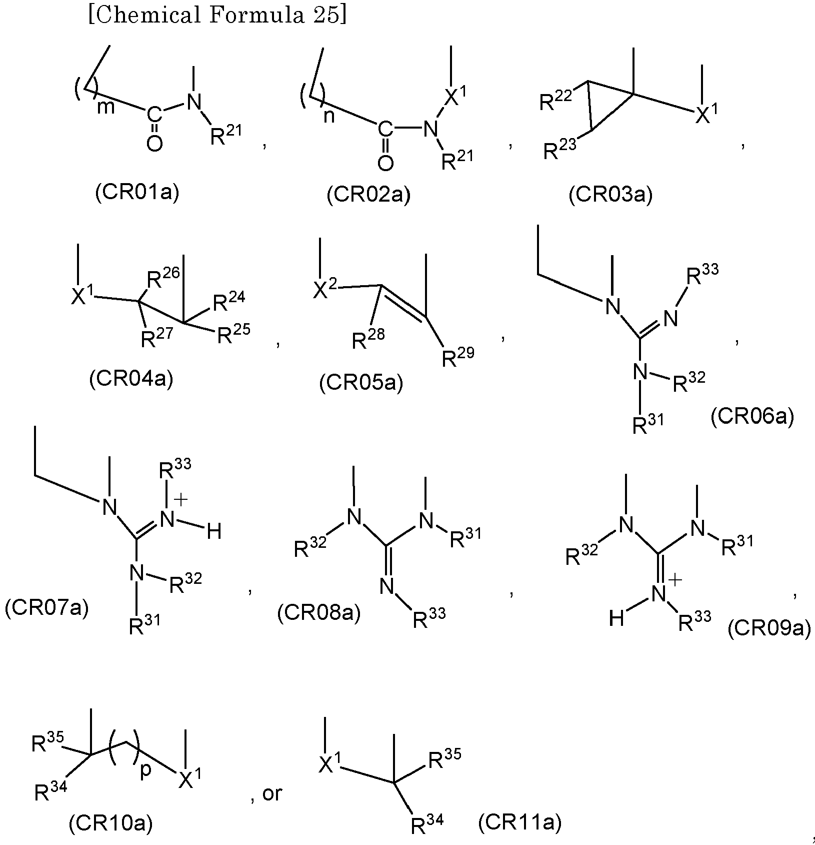 wherein R²¹ is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R²² and R²³ are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R²² and R²³ together represent -(CH₂)_{q1}-, wherein q1 is an integer from 2 to 5;
R²⁴ and R²⁵ are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R²⁴ and R²⁵ together represent =C(R³⁶)R³⁷, wherein R³⁶ and R³⁷ each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R²⁶ and R²⁷ are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁸ is a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁹ is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R³¹, R³², and R³³ are each independently a hydrogen atom, a C₁ to C₆ alkyl group that may be branched or form a ring, or an amino group protecting group for nucleic acid synthesis;
R³⁴ and R³⁵ are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m is an integer from 0 to 2;
   n is an integer or 1;
   p is an integer or 1;
   X¹ is an oxygen atom, a sulfur atom, or an amino group; and
   X² is an oxygen atom or a sulfur atom;
R¹⁰ is a hydrogen atom, and R¹¹ is a hydrogen atom or a halogen atom; a C₁ to C₆ linear alkoxy group that may be substituted with a C₁ to C₆ linear alkoxy group; or -OR^{12b}, wherein R^{12b} is a hydrogen atom or a hydroxy group protecting group for nucleic acid synthesis, or
R¹⁰ and R¹¹ together represent a divalent group represented by a formula below (CR01b) to (CR11b): 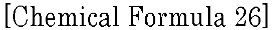 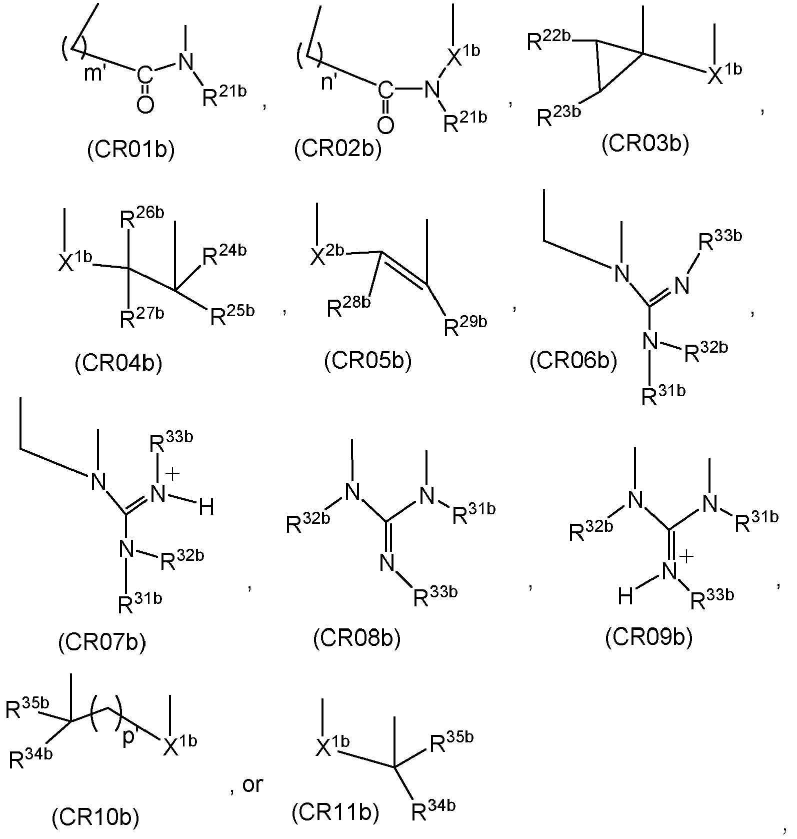 wherein R^{21b} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R^{22b} and R^{23b} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22b} and R^{23b} together represent -(CH₂)_{q2}-, wherein q2 is an integer from 2 to 5;
R^{24b} and R^{25b} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24b} and R^{25b} together represent =C(R^{36b})R^{37b}, wherein R^{36b} and R^{37b} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26b} and R^{27b} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28b} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29b} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31b}, R^{32b}, and R^{33b} are each independently a hydrogen atom, a C₁ to C₆ alkyl group that may be branched or form a ring, or an amino group protecting group for nucleic acid synthesis;
R^{34b} and R^{35b} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m' is an integer from 0 to 2;
   n' is an integer of 0 or 1;
   p' is an integer of 0 or 1;
   X^{1b} is an oxygen atom, a sulfur atom, or an amino group; and
   X^{2b} is an oxygen atom or a sulfur atom; and
M¹ represents a single bond or a formula below: 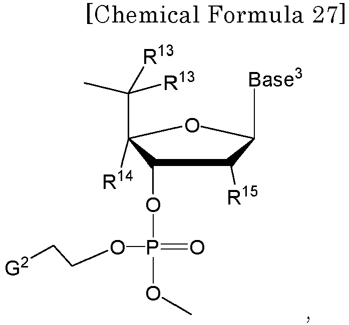 wherein Base³ represents a purin-9-yl group that may have any one or more substituents selected from the α group, or a 2-oxo-1,2-dihydropyrimidin-1-yl group that may have any one or more substituents selected from the α group;
G² is a cyano group or a nitro group;
R¹³ is a C₁ or C₂ alkyl group atoms that may be substituted with a halogen atom;
R¹⁴ is a hydrogen atom, and R¹⁵ is a hydrogen atom or a halogen atom; a C₁ to C₆ linear alkoxy group that may be substituted with a C₁ to C₆ linear alkoxy group; or -OR^{12c}, wherein R^{12c} is a hydrogen atom or a hydroxy group protecting group for nucleic acid synthesis, or
R¹⁴ and R¹⁵ together represent a divalent group represented by a formula below (CR01c) to (CR11c): 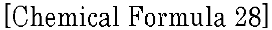 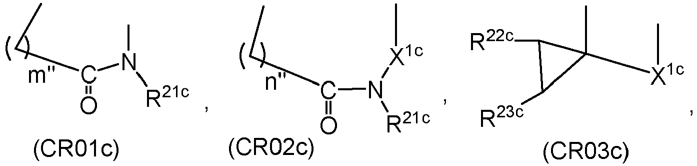 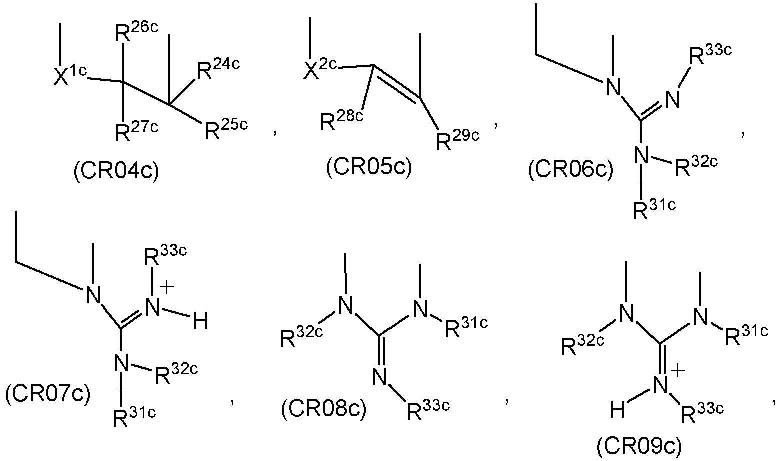 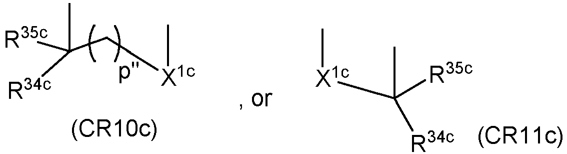 wherein R^{21c} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the α group and that may contain a heteroatom, an aralkyl group with a C₃ to C₁₂ aryl moiety that may have any one or more substituents selected from the α group and that may contain a heteroatom, or an amino group protecting group for nucleic acid synthesis;
R^{22c} and R^{23c} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22c} and R^{23c} together represent -(CH₂)_{q3}-, wherein q3 is an integer from 2 to 5;
R^{24c} and R^{25c} are each independently a group selected from the group consisting of a hydrogen atom, a hydroxy group, a C₁ to C₆ alkyl group that may be branches or form a ring, a C₁ to C₆ alkoxy group that may be branched or form a ring, an amino group, and an amino group protected by a protecting group for nucleic acid synthesis, or
R^{24c} and R^{25c} together represent =C(R^{36c})R^{37c}, wherein R^{36c} and R^{37c} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26c} and R^{27c} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28c} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29c} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31c}, R^{32c}, and R^{33c} are each independently a hydrogen atom, a C₁ to C₆ alkyl group that may be branched or form a ring, or an amino group protecting group for nucleic acid synthesis;
R^{34c} and R^{35c} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
   m" is an integer from 0 to 2;
   n" is an integer or 1;
   p" is an integer or 1;
   X^{1c} is an oxygen atom, a sulfur atom, or an amino group; and
   X^{2c} is an oxygen atom or a sulfur atom.

Note that, in the present invention, among the cross-link structures represented by the formulae (CR01a) to (CR11a), (CR01b) to (CR11b), and (CR01c) to (CR11c) above, the structures represented by the formulae (CR07a), (CR09a), (CR07b), (CR09b), (CR07c), and (CR09c) are kept electrically neutral by any anions (e.g., hydroxide ions, phosphoric ions, and chloride ions) that are present around the cross-link structure.

That is to say, the oligonucleotide of the present invention represented by the formula (II) above contains a nucleoside structure represented by the formula (II') or (II") below: 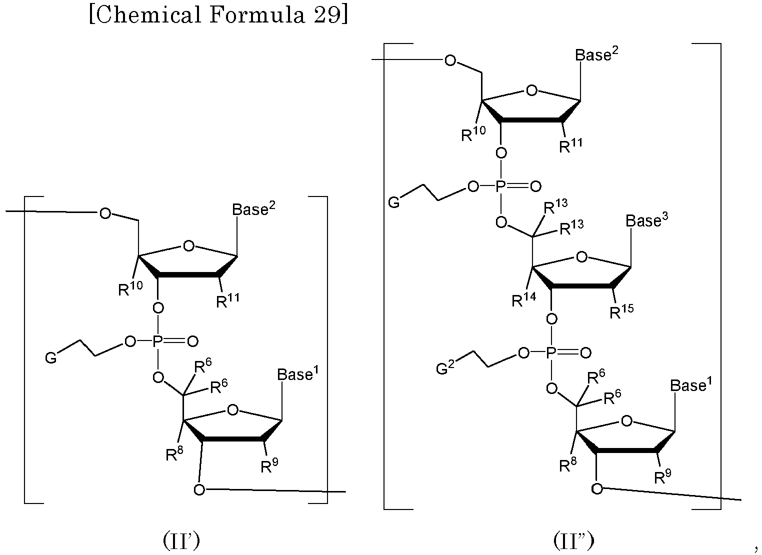 wherein Base¹, Base², G, R⁶, R⁸, R⁹, R¹⁰, and R¹¹ in the formula (II') are as defined for the formula (II) above, and Base¹, Base², Base³, G, G2, R⁶, R⁸, R⁹, R¹⁰, R¹¹, R¹³, R¹⁴, and R¹⁵ in the formula (II") are as defined for the formula (II) above.

In an embodiment, R⁶ in the oligonucleotide containing a nucleoside structure represented by the formula (II') above is a methyl group or an ethyl group. In an embodiment, R⁶ and R¹³ in the oligonucleotide containing a nucleoside structure represented by the formula (II") above are both methyl groups or ethyl groups.

In an embodiment, R⁸, R⁹, R¹⁰, and R¹¹ in the formula (II') above are all hydrogen atoms. In an embodiment, R⁸, R⁹, R¹⁰, R¹¹, R¹⁴, and R¹⁵ in the formula (II") above are all hydrogen atoms.

The oligonucleotide of the present invention has at least one nucleoside structure represented by the formula (II), or the formula (II') or (II"), above at any position. There is no particular limitation on the positions and number of the nucleoside structures, and the oligonucleotide can be designed as appropriate depending on the purpose.

An oligonucleotide (antisense molecule) containing such a nucleoside structure has significantly improved nuclease-resistant ability when compared with the cases wherein conventional 2',4'-BNA/LNA is used, and also has good binding affinity for ssRNA comparable to that of known 2',4'-BNA/LNA.

With all these facts, the oligonucleotide of the present invention synthesized using the 5'-modified nucleoside of the present invention is expected to be useful as a pharmaceutical agent (antisense molecule), such as antitumor agents and antiviral drugs, inhibiting the functions of specific genes to treat a disease.

In particular, for antisense therapies, the binding affinity for complementary sense strand RNAs and the resistance to in vivo DNA-degrading enzymes are both required. Generally, a nucleic acid in the form of a single strand is known to constantly have a structural fluctuation of a sugar moiety between the form close to a sugar moiety in a double-stranded DNA and the form close to a sugar moiety in a double-stranded DNA-RNA or a double-stranded RNA. When a single-stranded nucleic acid forms a double strand with a complementary RNA strand, its structure of the sugar moiety is fixed. Therefore, the 5'-modified nucleoside of the invention readily forms a double strand with an intended RNA strand, which may be then maintained stably, because the sugar moiety has already been kept to the structure capable of forming a double strand. Furthermore, it is also known that a double-stranded nucleic acid is stabilized with hydrated water with a chain-like structure referred to as "network of water molecules".

Additives typically used in the art of pharmaceuticals such as excipients, binders, preservatives, oxidation stabilizers, disintegrants, lubricants, and flavoring substances can be added to the oligonucleotide of the present invention to prepare parenteral formulations or liposomal formulations. Also, for example, topical formulations such as liquids, creams, and ointments may be prepared by adding pharmaceutical carriers typically used in the art.

### Examples

Hereinafter, the present invention will be described in greater detail using examples. However, the present invention is not limited to the examples below.

### (Example 1: Synthesis of 5'-Modified Nucleoside (1))

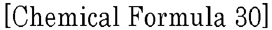

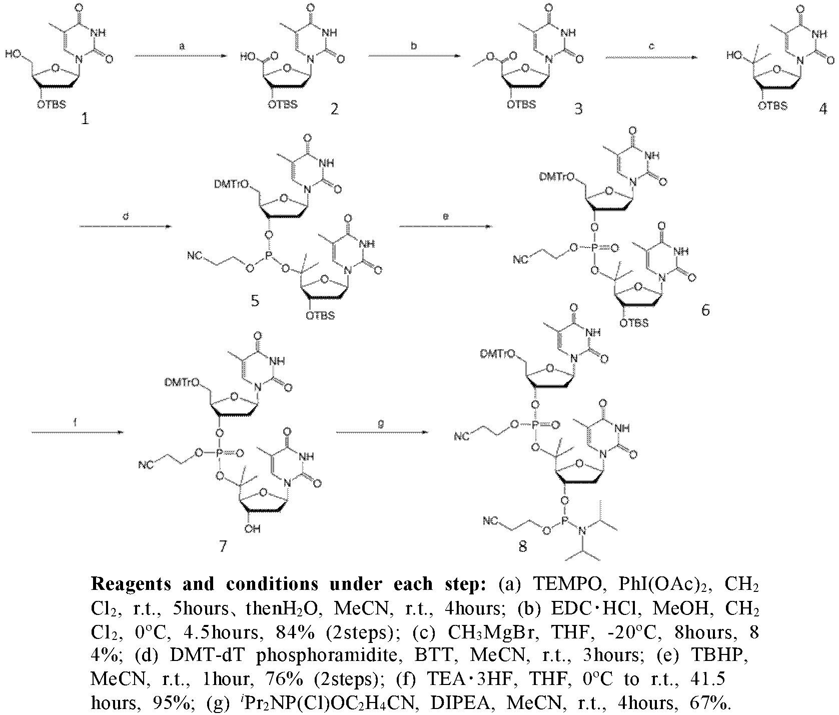

### (1-1) Synthesis of Compound 2

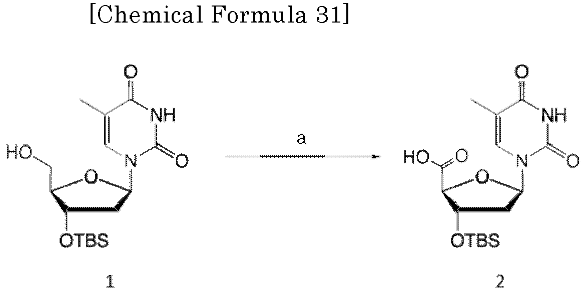

A compound 1 (4.26 g, 11.95 mmol) prepared using a method described in Caruthers et al., Tetrahedron Lett., 1996, Vol. 37, No. 35, pp. 6239-6242 was dissolved in dichloromethane (60 mL), and to the solution was then added iodobenzene diacetate (PhI (A)₂; 8.47 g, 26.30 mmol). Subsequently, 2,2,6,6-tetramethylpiperidine 1-oxyl free radical (TEMPO; 430.4 mg, 2.75 mmol) was added at 0°C, and the mixture was stirred at room temperature for 5 hours. After completion of the reaction, water/acetonitrile (= 1:1 (volume ratio), 0.66 mL) was added to the mixture, followed by stirring at room temperature for 4 hours. After completion of the reaction, methanol (20 mL) was added to the mixture, and then, the solvent was distilled away under reduced pressure to afford a compound 2 (crude product). The compound 2 was used for the next reaction without purification.

### (1-2) Synthesis of Compound 3

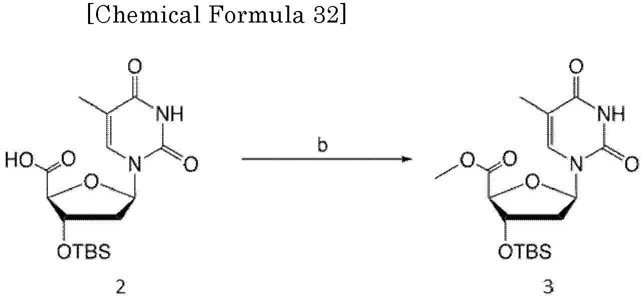

To a dichloromethane solution (120 mL) of the compound 2 (crude product) obtained above were added methanol (4.8 mL, 0.12 mol) and 1-(3-dimethylaminopropyl)-3-ethylcarbodiimide hydrochloride (EDC•HCl; 2.76 g, 14.39 mmol), and the mixture was stirred at 0°C for 4.5 hours. After completion of the reaction, water was added, and extraction with ethyl acetate was performed. The extraction fraction was washed with water and saturated saline and then dried over anhydrous sodium sulfate, and the solvent was distilled away under reduced pressure. The resultant residue was purified by silica-gel column chromatography (SiO₂, ethyl acetate:n-hexane = 1:1 (volume ratio)) to afford a compound 3 (3.84 g, yield: 84%, 2 steps) as a white solid.

Table 1 shows data on the properties of the obtained compound 3.

**[Table 1]**

| Physical property data of the obtained compound 3 |
|---|
| ¹H NMR (400 MHz, CDCl₃) δ 0.12 (s, 3H), 0.13 (s, 3H), 0.92 (s, 9H), 1.95 (ddd, *J* = 4.1, 8.7, 13.7 Hz, 1H), 1.97 (d, *J* =1.4 Hz, 3H), 2.31 (dd, *J* = 5.1, 13.3 Hz, 1H), 3.82 (s, 3H), 4.46 (s, 1H), 4.52 (d, *J* = 4.6 Hz 1H), 6.53 (dd, *J* = 5.5, 9.6 Hz 1H), 8.03 (brs, 1H), 8.09 (s, 1H); ^{1 3}C NMR (76 MHz, CDCl₃) δ ―4.9, 12.8, 18.1, 25.7, 39.8, 52.7, 75.8, 8 5.3, 86.7, 111.4, 136.3, 150.7, 164.2, 171.9; HRMS (MALDI) Calculated for C₁₇H₂₈N₂O₆NaSi [M+Na]⁺: 407.1609, Found: 407.1606. |

### (1-3) Synthesis of Compound 4

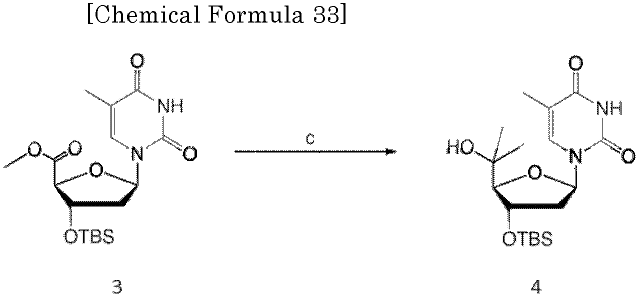

The compound 3 (3.00 g, 7.80 mmol) obtained above was azeotroped with anhydrous toluene and then dissolved in anhydrous tetrahydrofuran (80 mL). To the solution was slowly added dropwise at -20°C under nitrogen stream methylmagnesium bromide (CH₃MgBr; 31 mL, 31.0 mmol, 1 M in THF), and the mixture was stirred for 4.5 hours. Since the reaction was not completed, methylmagnesium bromide (CH₃MgBr; 7.8 mL, 7.8 mmol, 1 M in THF) was further added dropwise slowly at -20°C, and the mixture was stirred for 3.5 hours. After completion of the reaction, an aqueous solution of ammonium chloride was added, and extraction with ethyl acetate was performed. The extraction fraction was washed with saturated aqueous sodium bicarbonate and saturated saline and then dried over anhydrous sodium sulfate, and the solvent was distilled away under reduced pressure. The resultant residue was purified by silica-gel column chromatography (SiO₂, ethyl acetate:n-hexane = 3:2 (volume ratio)) to afford a compound 4 (2.52 g, yield: 84%) as a white solid.

Table 2 shows data on the properties of the obtained compound 4.

**[Table 2]**

| Physical property data of the obtained compound 4 |
|---|
| ¹H NMR (400 MHz, CDCl₃) δ 0.09 (s, 3H), 0.10 (s, 3H), 0.90 (s, 9H), 1.28 (s, 3H), 1.29 (s, 3H), 1.93 (d, *J* = 1.4 Hz, 3H), 2.10 (ddd, *J =* 2.7, 6.4, 13.8 Hz, 1H), 2.43 (ddd, *J* = 6.0, 8.2, 13.3 Hz 1H), 2.54 (s, 1 H), 3.68 (d, *J* = 2.8 Hz 1H), 4.52 (ddd, *J* = 2.3, 2.3, 6.5 Hz 1H), 6.05 (dd, *J* = 6.0, 8.3 Hz, 1H), 7.34 (d, *J* =1.4 Hz,1H), 8.02 (brs, 1H); ¹³C NMR (76 MHz, CDCl₃) δ ―4.8, -4.3, 12.5, 17.7, 25.6, 26.2, 27,4, 40. 0, 70.9, 71,7, 87.5, 93.6, 110.0, 137.7, 150.4, 163.8; HRMS (MALDI) Calculated for C₁₇H₃₂N₂O₅NaSi [M+Na]⁺: 407.1973, Fpund: 407.1882. |

### (1-4) Synthesis of Compound 5

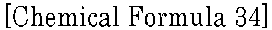

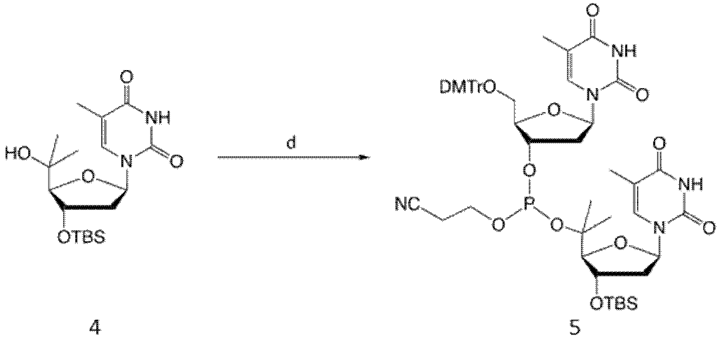

The compound 4 (1.26 g, 3.28 mmol) obtained above was azeotroped with anhydrous toluene and then dissolved in anhydrous acetonitrile (35 mL). To the solution were added sequentially under nitrogen stream 5'-(4,4'-dimethoxytrityl)-thymidine-3'-[(2-cyanoethyl)-(N,N-diisopropyl)]-phosphoramidite (DMT-dT phosphoramidite; 3.69 g, 4.95 mmol) and 5-(benzylthio)-1H-tetrazole (BTT; 0.98 g, 5.10 mmol), and the mixture was stirred at room temperature for 3 hours. After completion of the reaction, water was added, and extraction with ethyl acetate was performed. The extraction fraction was washed with water and saturated saline and then dried over anhydrous sodium sulfate, and the solvent was distilled away under reduced pressure. The resultant residue was purified by silica-gel column chromatography (SiO₂, ethyl acetate:n-hexane = 1:1 →- 5:2 (volume ratio)) to afford a compound 5 (3.48 g) as a crude product.

### (1-5) Synthesis of Compound 6

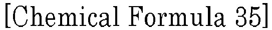

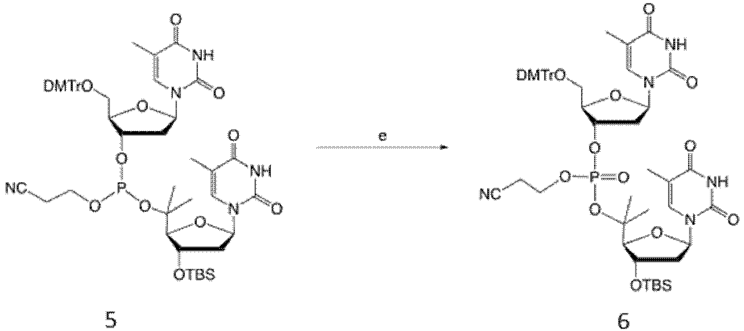

The compound 5 (3.48 g) obtained above was dissolved in acetonitrile (30 mL) and to the solution was added tert-butyl hydroperoxide (TBHP; 4.6 mL, 70% aqueous solution, 33.59 mmol), and the mixture was stirred at room temperature for an hour. After completion of the reaction, a saturated aqueous solution of sodium thiosulfate was added at 0°C, and extraction with ethyl acetate was performed. The extraction fraction was washed with water and saturated saline and then dried over anhydrous sodium sulfate, and the solvent was distilled away under reduced pressure. The resultant residue was purified by silica-gel column chromatography (SiO₂, methanol:chloroform = 1:20 (volume ratio)) to afford a compound 6 (3.24 g, yield: 76%, 2 steps) as a white solid.

Table 3 shows data on the properties of the obtained compound 6.

**[Table 3]**

| Physical property data of the obtained compound 6 |
|---|
| ¹H NMR (400 MHz, CDCl₃) δ 0.05-0.13 (m, 6H), 0.88 (s, 9H), 1.40 (s, 3H), 1.51 (s, 1.2H), 1.58 (s, 1.2H), 1.59 (s, 1.8H), 1.63 (s, 1.8H), 1.90 (s, 1.2H), 1.91 (d, *J* = 0.9 Hz, 1.8H), 2.04-2.26 (m, 2H), 2.37-2.49 (m, 1H), 2.53-2.70 (m, 2H), 2.76 (dd, *J* = 6.0, 6.0 Hz, 1H), 3.33-3.43 (m, 1 H), 3.45-3.52 (m, 0.4H), 3.53-3.57 (m, 0.6H), 3.60-3.64 (m, 0.4H), 3.67-3.70 (m, 0.6H), 3.79 (s, 3H), 3.80 (s, 3H), 4.04-4.18 (m, 1H), 4.19-4.32 (m, 2H), 4.43-4.51 (m, 1H), 5.12-5.18 (m, 1H), 6.21-6.29 (m, 1H), 6.37-6.46 (m, 1H), 6.83 (d, *J* = 8.7 Hz, 1.6H), 6.84 (d, *J* = 8.7 Hz, 2.4H), 7. 22-7.43 (m, 10H), 7.54-7.61 (m, 1H), 8.78 (brs, 0.4H), 8.83 (brs, 0.6H), 8.85 (brs, 0.6H), 8.95 (brs, 0.4H); ³¹P NMR (161.8 MHz, CDCl₃) δ -5.66, -5.52; HRMS (MALDI) Calculated for C₅₂H₆₆N₅O₁₄NaSiP [M+Na]⁺: 1066. 4005, Found: 1066.4032. |

### (1-6) Synthesis of Compound 7

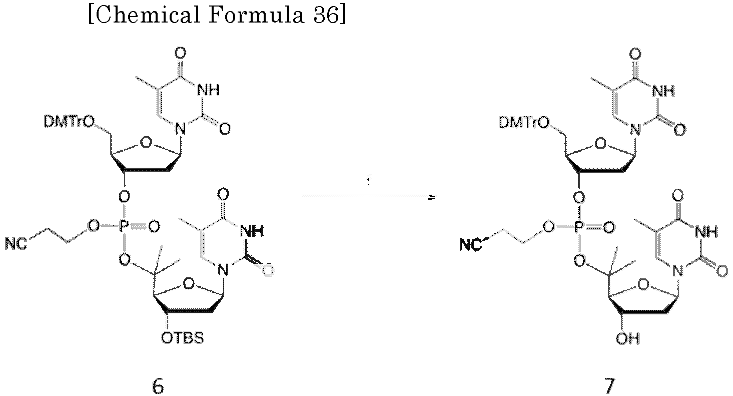

The compound 6 (0.64 g, 0.61 mmol) obtained above was dissolved in anhydrous tetrahydrofuran (6 mL), and to the solution was added at 0°C triethylamine trihydrofluoride (TEA•3HF; 1.0 mL, 6.13 mmol), followed by stirring for 15 minutes. Then, the mixture was stirred at room temperature for 41.5 hours. After completion of the reaction, the solution was diluted by adding ethyl acetate (30 mL), and then washed twice with 2% aqueous sodium bicarbonate and once with water. The aqueous layer was re-extracted with ethyl acetate, the organic layers were combined and washed once with saturated saline, and then dried over anhydrous sodium sulfate, and the solvent was distilled away under reduced pressure. The resultant residue was purified by silica-gel column chromatography (SiO₂, methanol:chloroform = 1:10 (volume ratio)) to afford a compound 7 (539 mg, yield: 95%) as a white solid.

Table 4 shows data on the properties of the obtained compound 7.

**[Table 4]**

| Physical property data of the obtained compound 7 |
|---|
| ¹H NMR (400 MHz, CDCl₃) δ 1.41 (s, 1.8H), 1.42 (d, *J* = 0.9 Hz, 1.2 H), 1.53 (s, 1.2H), 1.59 (s, 1.2H), 1.62 (s, 1.8H), 1.67 (s, 1.8H), 1.90 (d, *J* = 0.9 Hz, 1.2H), 1.90 (d, *J* = 0.9 Hz, 1.8H), 2.06-2.17 (m, 1H), 2.28-2.49 (m, 2H), 2.60-2.82 (m, 2H), 3.34-3.43 (m, 1H), 3.46-3.57 (m, 1H), 3.70-3.78 (m, 1H), 3.79 (s, 3H), 3.79(s, 3H), 4.09-4.32 (m, 3H), 4.52-4.62 (m, 1H), 5.07-5.18 (m, 1H), 6.37-6.46 (m, 2H), 6.84 (d, *J =* 9.2 Hz, 1.6H), 6.85 (d, *J* = 8.3 Hz, 2.4H), 7.20―7.40 (m, 10H), 7.55 (d, *J =* 0.9 Hz, 0.4H), 7.62 (d, *J =* 1.4 Hz, 0.6H), 9.37 (brs, 0.4H), 9.44 (brs, 0.6H), 9.60 (brs, 0.4H), 9.96 (brs, 0.6H); ³¹P NMR (161.8 MHz, C DCl₃) δ ―6.71, -5.90; HRMS (MALDI) Calculated for C₄₆H₅₂N₅O₁₄NaP [M+Na]⁺: 952.3141, Found: 952.3114. |

### (1-7) Synthesis of Compound 8

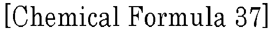

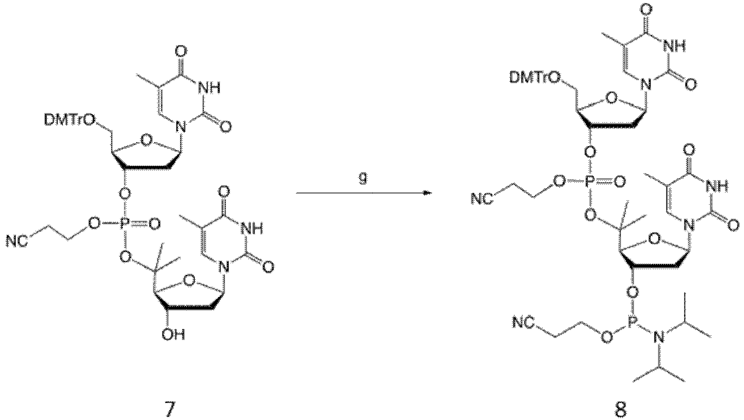

The compound 7 (3.98 g, 4.28 mmol) obtained above was azeotroped with anhydrous toluene. Then, to the solution were added sequentially under nitrogen stream anhydrous acetonitrile (43 mL), N,N-diisopropylethylamine (DIPEA; 2.2 mL, 12.94 mmol), and 2-cyanoethyl-N,N-diisopropyl phosphorochloridate (ⁱPr₂NP(Cl)OC₂H₄CN; 1.8 mL, 6.46 mmol), and the mixture was stirred at room temperature for 4 hours. After completion of the reaction, the mixture was diluted with ethyl acetate, washed with water and saturated saline, and then dried over anhydrous sodium sulfate, and the solvent was distilled away under reduced pressure. The resultant residue was purified by silica-gel column chromatography (diol-SiO₂, ethyl acetate:n-hexane = 1:1 → 1:2 (volume ratio)), and then, reprecipitation (hexane:chloroform = 20:1 (volume ratio)) was performed to afford a compound 8 (3.25 g, yield: 67%) as a white solid.

Table 5 shows data on the properties of the obtained compound 8.

**[Table 5]**

| Physical property data of the obtained compound 8 |
|---|
| ¹H NMR (400 MHz, CDCl₃) δ 1.13-1.22 (m, 12H), 1.37-1.41 (m, 3H), 1.49-1.67 (m, 6H), 1.89-1.95 (m, 3H), 2.07―2.24 (m, 1H), 2.30―2.68 (m, 7H), 2.72-2.81 (m, 1H), 3.35-3.43 (m, 1H), 3.45-3.75 (m, 5H), 3.76-3.97 (m, 2H), 3.79 (s, 3H), 3.80 (s, 3H), 4.02―4.34 (m, 4H), 4.62―4.70 (m, 1H), 5.13-5.22 (m, 1H), 6.27-6.35 (m, 1H), 6.37-6.47 (m, 1H), 6.83 (d, *J =* 8.7 Hz, 1.6H), 6.84 (d, *J=* 9.2 Hz, 2.4H), 7.21―7.40 (m, 10H), 7.53-7.59 (m, 1H), 8.01-8.16 (brs, 2H); ³¹P NMR (161.8 MHz, CDCl₃) δ -5.87, -5.47, -5.39, 148.3, 148.4, 149.2, 149.2; HRMS (MALDI) Calculated for C₅₅H₆₉N₇O₁₅NaP₂ [M+Na]⁺: 1152.4219, Found: 1152.4215. |

### (Example 2: Synthesis of 5'-Modified Nucleoside (2))

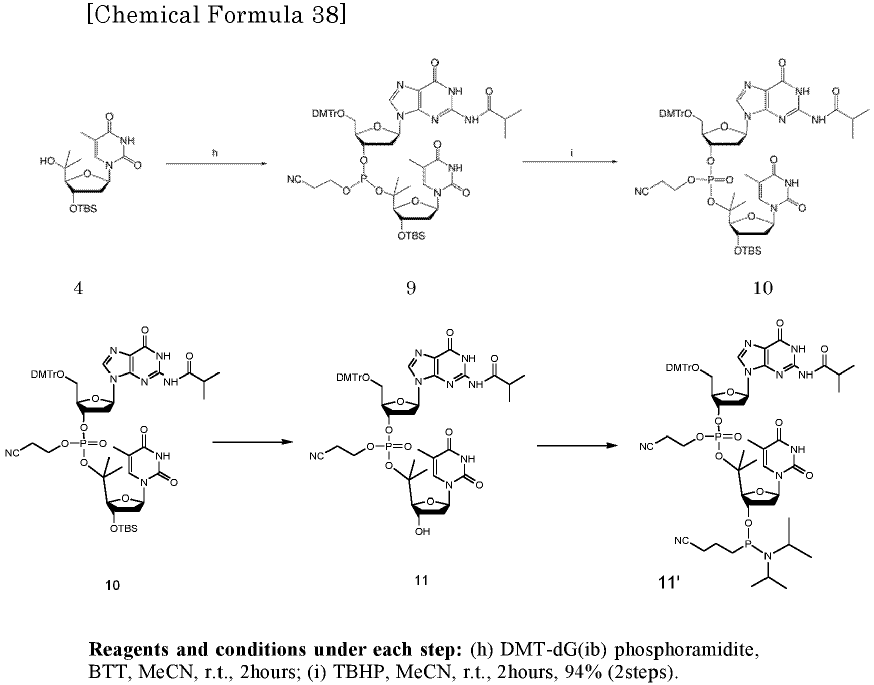

### (2-1) Synthesis of Compound 9

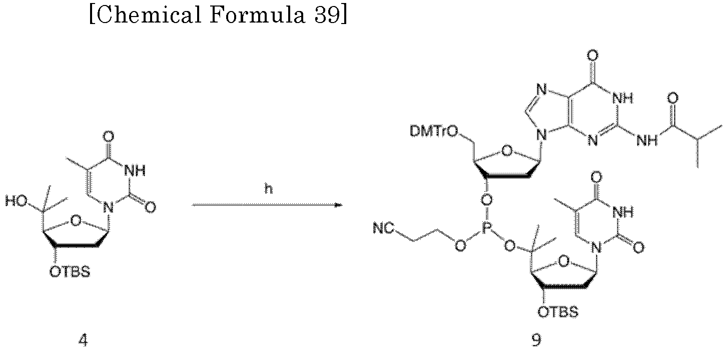

The compound 4 (494.7 mg, 1.29 mmol) obtained in (1-3) of Example 1 was azeotroped with anhydrous toluene and then dissolved in anhydrous acetonitrile (13 mL). To the solution were added sequentially under nitrogen stream 5'-(4,4'-dimethoxytrityl)-N-isobutyryl-2'-deoxyguanosine-3'-[(2-cyanoethyl )-(N,N-diisopropyl)]-phosphoramidite (DMT-dG(ib) phosphoramidite; 1.66 g, 1.98 mmol) and 5-(benzylthio)-1H-tetrazole (BTT; 372.6 mg, 1.94 mmol), and the mixture was stirred at room temperature for 2 hours. After completion of the reaction, water was added, and extraction with ethyl acetate was performed. The extraction fraction was washed with water and saturated saline and then dried over anhydrous sodium sulfate, and the solvent was distilled away under reduced pressure. A compound 9 (2.51 g; crude product) thus obtained was used for the next reaction without purification.

### (2-2) Synthesis of Compound 10

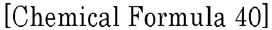

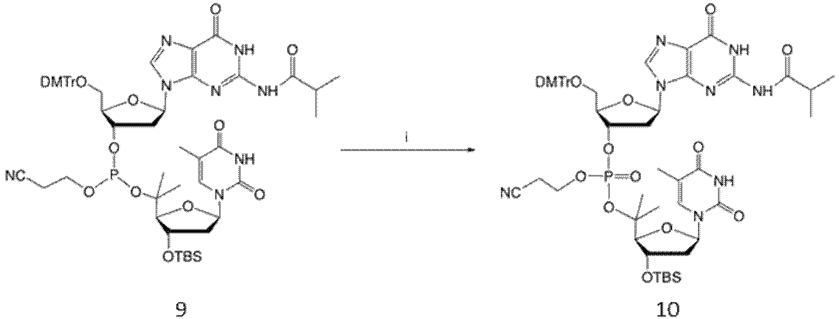

The compound 9 (2.51 g) obtained above was dissolved in acetonitrile (11 mL) and to the solution was added tert-butyl hydroperoxide (TBHP; 1.8 mL, 70% aqueous solution, 13.14 mmol), and the mixture was stirred at room temperature for 2 hours. After completion of the reaction, a saturated aqueous solution of sodium thiosulfate was added at 0°C, and extraction with ethyl acetate was performed. The extraction fraction was washed with water and saturated saline and then dried over anhydrous sodium sulfate, and the solvent was distilled away under reduced pressure. The resultant residue was purified by silica-gel column chromatography (SiO₂, methanol:chloroform = 1:18 →- 1:16) to afford a compound 10 (1.37 g, yield: 94%, 2 steps) as a white solid.

Table 6 shows data on the properties of the obtained compound 10.

**[Table 6]**

| Physical property data of the obtained compound 10 |
|---|
| ¹H NMR (400 MHz, CDCl₃) δ 0.08―0.14 (m, 6H), 0.83―0.91 (m, 9H), 1.12―1.30 (m, 6H), 1.46―1.68 (m, 6H), 2.01 (s, 1.8H), 2.15 (s, 1.2H), 2.18―2.92 (m, 7H), 3.24―3.44 (m, 2H), 3.57―3.67 (m, 1H), 3.78 (s, 6H), 4.05―4.20 (m, 1H), 4.23―4.36 (m, 2H), 4.60―4.76 (m, 1H), 5.23―5.32 (m, 1H), 5.87―5.95 (m, 0.6H), 6.10―6.23 (m, 1H), 6.31―6.39 (m, 0.4H), 6.81 (d, *J* = 11.6 Hz, 2.4H), 6.82 (d, *J* = 12.2 Hz, 1.6H), 6.97 (s, 0.6 H), 7.00 (s, 0.4H), 7.17―7.45 (m, 9H), 7.70 (s, 0.4H), 7.72 (s, 0.6H), 8. 34 (brs, 0.4H), 9.36 (brs, 0.6H), 10.17 (brs, 0.6H), 10.20 (brs, 0.4H), 1 2.21 (brs, 0.4H), 12.27 (brs, 0.6H); ³¹P NMR (161.8 MHz, CDCl₃) δ ―6. 22, ―5.66; HRMS (MALDI) Calculated for C₅₆H₇₁N₈O₁₄NaSiP [M+Na]⁺: 1161.4489, Found: 1161.4483. |

### (2-3) and (2-4) Synthesis of Compounds 11 and 11'

With the use of the compound 10 obtained above, a compound 11' can be obtained according to the following synthesis scheme, on the basis of the methods described in (1-6) and (1-7) of Example 1 above.

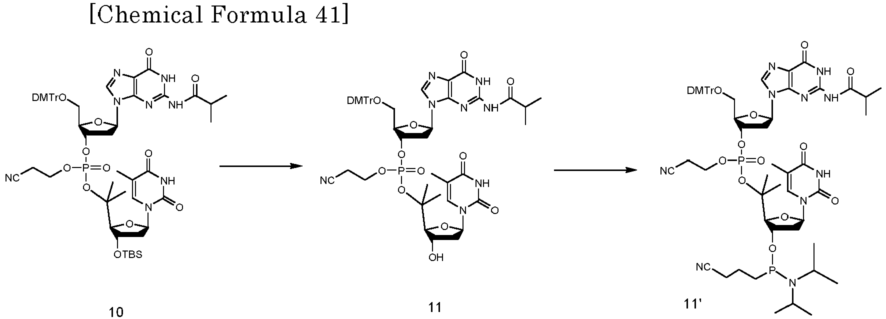

### (Example 3: Synthesis of 5'-Modified Nucleoside (3))

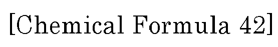

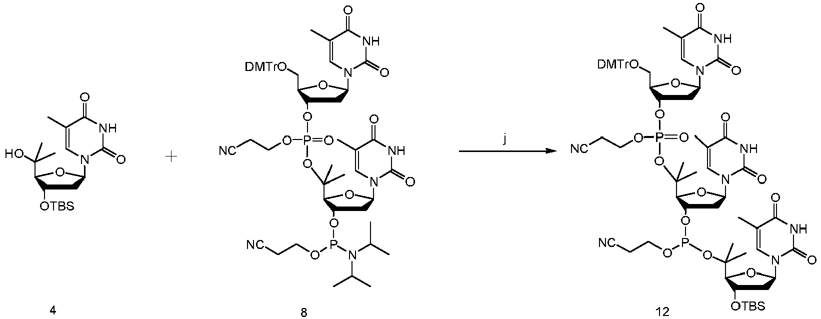

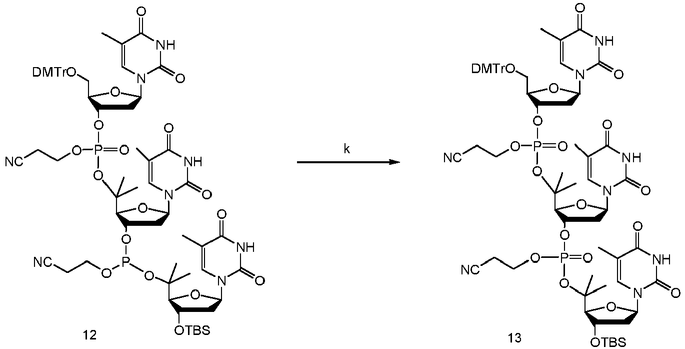

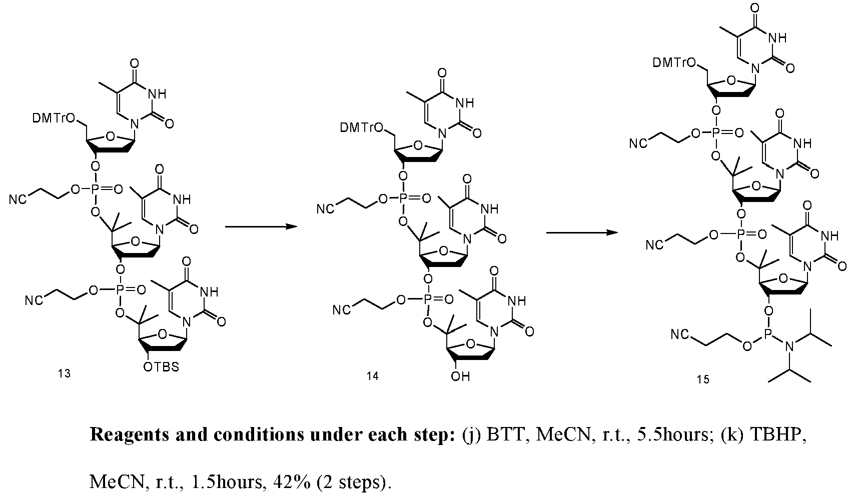

### (3-1) Synthesis of Compound 12

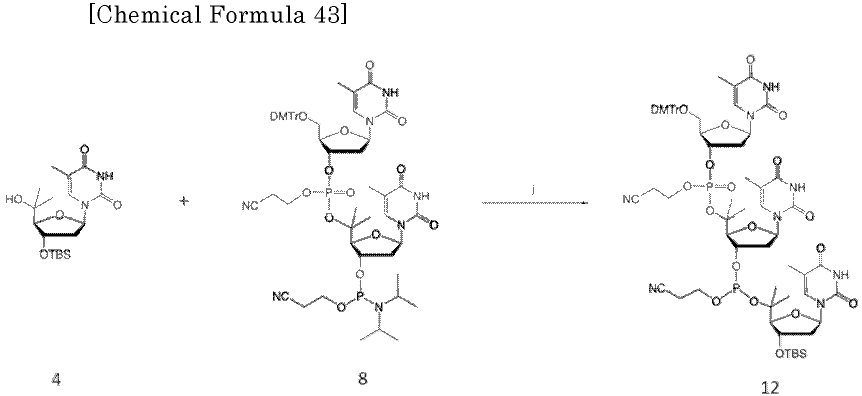

The compound 8 (80.5 mg, 0.071 mmol) obtained in (1-7) of Example 1 above was azeotroped with anhydrous toluene and then dissolved in anhydrous acetonitrile (0.5 mL). To the solution were added sequentially under nitrogen stream the compound 4 (21.9 mg, 0.057 mmol) obtained in (1-3) of Example 1 above and 5-(benzylthio)-1H-tetrazole (BTT; 17.1 mg, 0.089 mmol), and the mixture was stirred at room temperature for 5.5 hours. After completion of the reaction, water was added, and extraction with ethyl acetate was performed. The extraction fraction was washed with water and saturated saline and then dried over anhydrous sodium sulfate, and the solvent was distilled away under reduced pressure. A compound 12 (105.9 mg; crude product) thus obtained was used for the next reaction without purification.

### (3-2) Synthesis of Compound 13

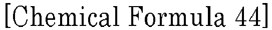

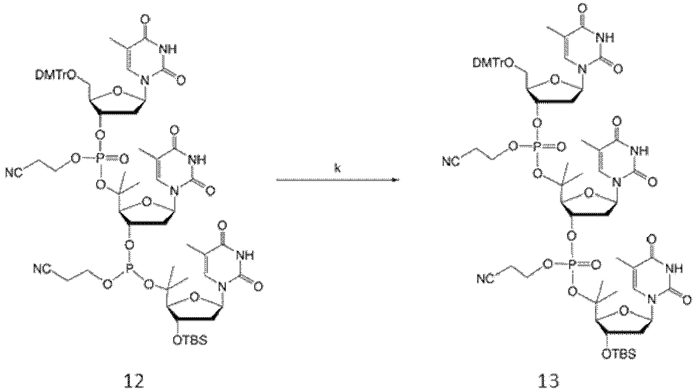

The compound 12 (105.9 mg) obtained above was dissolved in acetonitrile (0.45 mL). To the solution was added tert-butyl hydroperoxide (TBHP; 71.3 µL, 70% aqueous solution, 0.052 mmol), and the mixture was stirred at room temperature for 1.5 hours. After completion of the reaction, a saturated aqueous solution of sodium thiosulfate was added at 0°C, and extraction with ethyl acetate was performed. The extraction fraction was washed with water and saturated saline and then dried over anhydrous sodium sulfate, and the solvent was distilled away under reduced pressure. The resultant residue was purified by silica-gel column chromatography (SiO₂, methanol:chloroform = 1:12) to afford a compound 13 (33.1 mg, yield: 42%, 2 steps) as a white solid.

Table 7 shows data on the properties of the obtained compound 13.

**[Table 7]**

| Physical property data of the obtained compound 13 |
|---|
| ¹H NMR (400 MHz, CDCl₃) δ 0.07―0.12 (m, 6H), 0.88 (s, 5H), 0.88 (s, 4H), 1.39 (s, 1.5H), 1.41 (s, 1.5H), 1.49-1.71 (m, 12H), 1.88―1.97 (m, 6H), 2.13―2.27 (m, 3H), 2.40―2.69 (m, 4H), 2.73―2.82 (m, 3H), 3.3 4―3.42 (m, 1H), 3.47―3.58 (m, 1H), 3.68―3.74 (m, 1H), 3.79 (s, 3H), 3. 80 (s, 3H), 3.85―3.98 (m, 1H), 4.04―4.37 (m, 5H), 4.42―4.52 (m, 1H), 5.12―5.24 (m, 2H), 6.13―6.45 (m, 3H), 6.84 (d, *J* = 8.7 Hz, 4/3H), 6.85 (d, *J* = 8.7 Hz, 8/3H), 7.20―7.43 (m, 11H), 7.53―7.58 (m, 1H), 8.31―8. 74 (m, 3H); ³¹P NMR (161.8 MHz, CDCl₃) δ ―6.36, ―6.17, ―5.85, ―5.7 6; HRMS (MALDI) Calculated for C₆₇H₈₆N₈O₂₁NaSiP₂ [M+Na]⁺: 1451.50 44, Found: 1451.5037. |

### (3-3) and (3-4) Synthesis of Compounds 14 and 15

With the use of the compound 13 obtained above, a compound 15 can be obtained according to the following synthesis scheme, on the basis of the methods described in (1-6) and (1-7) of Example 1 above.

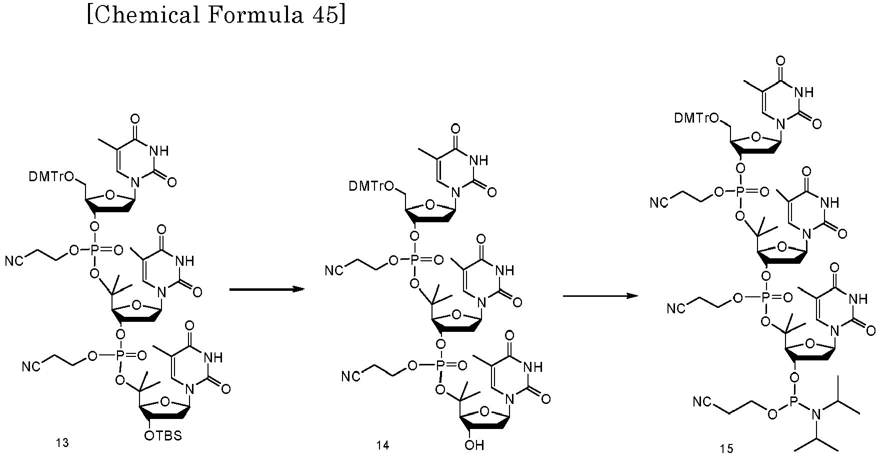

### (Example 4: Synthesis of 5'-Modified Nucleoside (4))

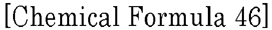

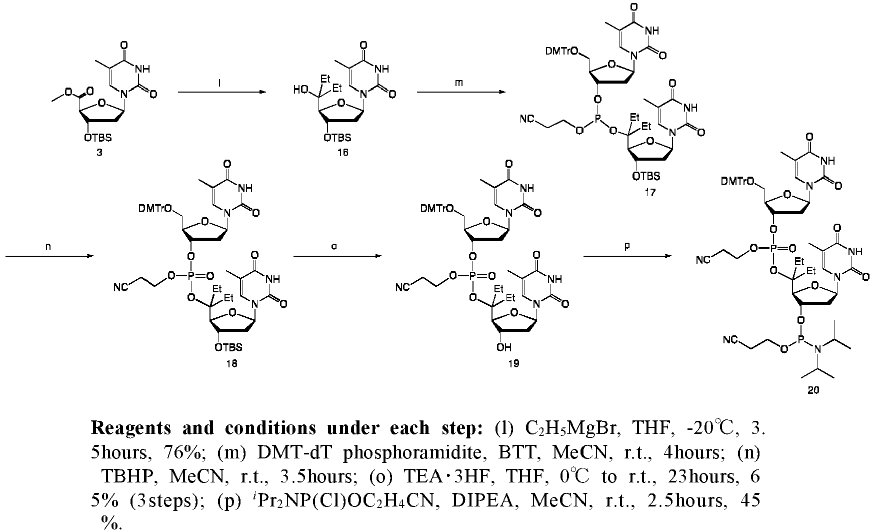

### (4-1) Synthesis of Compound 16

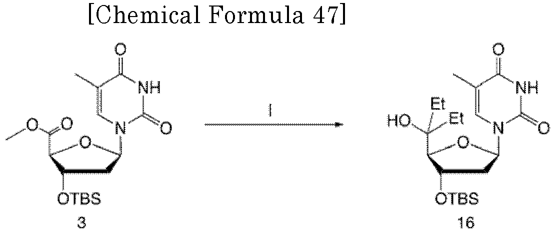

The compound 3 (446 mg, 1.16 mmol) obtained above was azeotroped with anhydrous toluene and then dissolved in anhydrous tetrahydrofuran (12 mL). To the solution was slowly added dropwise at ―20°C under nitrogen stream ethylmagnesium bromide (C₂H₅MgBr; 4.6 mL, 4.6 mmol, 1 M in THF), and the mixture was stirred at the same temperature for 3.5 hours. After completion of the reaction, an aqueous solution of ammonium chloride was added, and extraction with ethyl acetate was performed. The extraction fraction was washed with saturated aqueous sodium bicarbonate and saturated saline and then dried over anhydrous sodium sulfate, and the solvent was distilled away under reduced pressure. The resultant residue was purified by silica-gel column chromatography (SiO₂, ethyl acetate:n-hexane = 1:1) to afford a compound 16 (364 mg, 76%) as a white solid.

Table 8 shows data on the properties of the obtained compound 16.

**[Table 8]**

| Physical property data of the obtained compound 16 |
|---|
| ¹H NMR (300 MHz, CDCl₃) δ 0.09 (s, 6H), 0.87 (t, *J* = 7.6 Hz, 3H), 0.90 (s, 9H), 0.92 (t, *J* = 7.6 Hz, 3H), 1.51―1.70 (m, 4H), 1.92 (s, 3 H), 2.09 (ddd, *J* = 2.1, 5.5, 13.0 Hz, 1H), 2.32 (s, 1H), 2.40 (ddd, *J* = 6.2, 8.3, 14.4 Hz 1H), 3.81 (d, *J* = 2.7 Hz, 1H), 4.55 (ddd, *J* =2.8, 2. 8, 6.2 Hz, 1H), 6.06 (dd, *J* = 6.2, 8.3 Hz, 1H), 7.38 (d, *J* = 1.4 Hz, 1 H), 7.99 (brs, 1H); ¹³C NMR (75.57 MHz, CDCl₃) δ ―4.9, ―4.3, 7.4, 8.1, 12.5, 17.7, 25.6, 26.5, 27.4, 40.3, 71.6, 75.4, 86.9, 90.7, 111.0, 13 7.6, 150.4, 163.9; HRMS (MALDI) Calculated for C₂₀H₃₆N₂O₅NaSi [M+ Na]⁺: 435.2286, Found: 435.2283. |

### (4-2) Synthesis of Compound 17

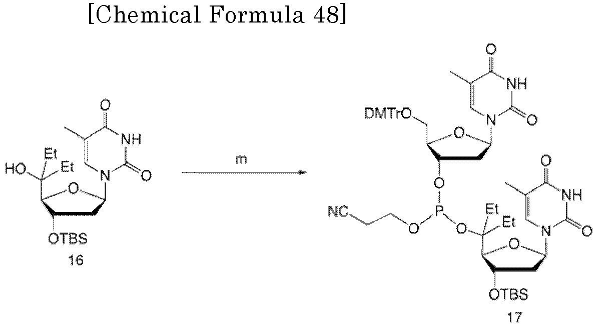

The compound 16 (283 mg, 0.69 mmol) obtained above was azeotroped with anhydrous toluene and then dissolved in anhydrous acetonitrile (7 mL). To the solution were added sequentially under nitrogen stream 5'-(4,4'-dimethoxytrityl) -thymidine-3'-[(2-cyanoethyl)-(N,N-diisopropyl)]-phosphoramidite (DMT-dT phosphoramidite; 840.3 mg, 1.13 mmol) and 5-(benzylthio)-1H-tetrazole (BTT; 198.9 mg, 1.03 mmol), and the mixture was stirred at room temperature for 4 hours. After completion of the reaction, water was added, and extraction with ethyl acetate was performed. The extraction fraction was washed with water and saturated saline and then dried over anhydrous sodium sulfate, and the solvent was distilled away under reduced pressure to afford a compound 17 (1.22 g) as a crude product. The compound 17 was used for the next reaction without purification.

### (4-3) Synthesis of Compound 18

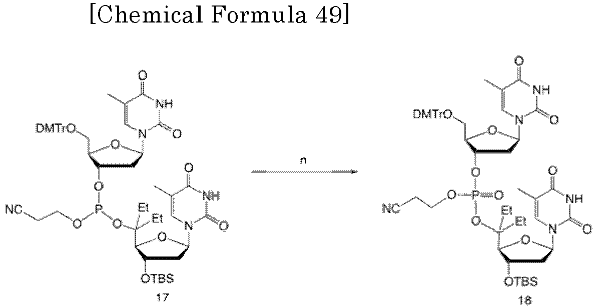

The compound 17 (1.22 g) obtained above was dissolved in acetonitrile (6.2 mL). To the solution was added tert-butyl hydroperoxide (TBHP; 0.7 mL, 70% aqueous solution, 5.11 mmol), and the mixture was stirred at room temperature for 3.5 hours. After completion of the reaction, a saturated aqueous solution of sodium thiosulfate was added at 0°C, and extraction with ethyl acetate was performed. The extraction fraction was washed with water and saturated saline and then dried over anhydrous sodium sulfate, and the solvent was distilled away under reduced pressure. The resultant residue was briefly purified by silica-gel column chromatography (SiO₂, methanol:chloroform = 0:1 →- 1:30) to afford a compound 18 (979 mg) as a crude product.

Table 9 shows data on the properties of the obtained compound 18.

**[Table 9]**

| Physical property data of the obtained compound 18 |
|---|
| HRMS (FAB) Calculated for C₅₄H₇₀N₅O₁₄SiP [M]⁺: 1071.4426, Found: 10 71.4403. |

### (4-4) Synthesis of Compound 19

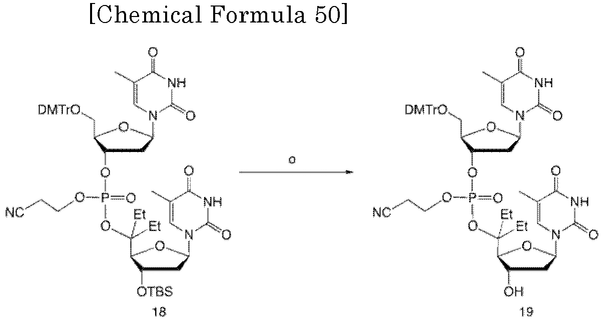

The compound 18 (979 mg) obtained above was dissolved in anhydrous tetrahydrofuran (6.9 mL). To the solution was added at 0°C triethylamine trihydrofluoride (TEA•3HF; 1.1 mL, 6.61 mmol), and then, the mixture was stirred at room temperature for 16.5 hours. Since the reaction was not completed, triethylamine trihydrofluoride (TEA•3HF; 0.6 mL, 3.61 mmol) was further added at 0°C, and then, the mixture was stirred at room temperature for 6.5 hours. After completion of the reaction, the solution was diluted by adding ethyl acetate (30 mL), and then washed twice with 2% aqueous sodium bicarbonate and once with water. The aqueous layer was re-extracted with ethyl acetate, the organic layers were combined and washed once with saturated saline, and then dried over anhydrous sodium sulfate, and the solvent was distilled away under reduced pressure. The resultant residue was purified by silica-gel column chromatography (SiO₂, methanol:chloroform = 0:1 →- 1:30) to afford a compound 19 (425 mg, 65% (3 or more steps)) as a white solid.

Table 10 shows data on the properties of the obtained compound 19.

**[Table 10]**

| Physical property data of the obtained compound 19 |
|---|
| ¹H NMR (400 MHz, CDCl₃) δ 0.99 (t, *J* = 7.8 Hz, 3H), 1.05 (t, *J* = 7.4 Hz, 3H), 1.39―1.44 (m, 3H), 1.81―2.85 (m, 14H), 3.36―3.43 (m, 1 H), 3.54 (dd, *J* = 2.3, 13.7 Hz, 1H), 3.79 (s, 6H), 3.84―4.33 (m, 4H), 4.64―4.73 (m, 1H), 5.10―5.18 (m, 1H), 6.21-6.31 (m, 1H), 6.35―6.43 (m, 1H), 6.83 (d, *J* = 8.7 Hz, 1H), 6.85 (d, *J* = 8.7 Hz, 3H), 7.10―7.63 (m, 11H), 8.20―8.62 (m, 2H); HRMS (FAB) Calculated for C₄₈H₅₆N₅O₁₄ P [M]⁺: 957.3561, Found: 957.3550. |

### (4-5) Synthesis of Compound 20

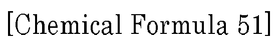

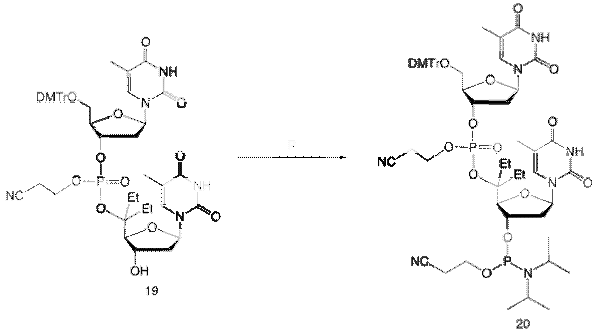

The compound 19 (393 mg, 0.41 mmol) obtained above was azeotroped with anhydrous toluene. Then, to the solution were added sequentially under nitrogen stream anhydrous acetonitrile (4.2 mL), N,N-diisopropylethylamine (DIPEA; 0.25 mL, 1.47 mmol), and 2-cyanoethyl-N,N-diisopropyl phosphorochloridate (ⁱPr₂NP(Cl)OC₂H₄CN; 0.17 mL, 0.61 mmol), and the mixture was stirred at room temperature for 2.5 hours. The solvent was partially distilled away under reduced pressure, the resultant residue was purified by silica-gel column chromatography (SiO₂, methanol:chloroform = 0:1 →- 1:35), and then, reprecipitation (pentane:chloroform = 20:1) was performed to afford a compound 20 (215 mg, 45%) as a white solid.

Table 11 shows data on the properties of the obtained compound 20.

**[Table 11]**

| Physical property data of the obtained compound 20 |
|---|
| ¹H NMR (400 MHz, CDCl₃) δ 0.97 (t, *J* = 7.4 Hz, 3H), 1.04 (t, *J* = 7.3 Hz, 3H), 1.12―1.35 (m, 12H), 1.40 (s, 3H), 1.74―2.27 (m, 4H), 1.91 (s, 3H), 2.28―2.85 (m, 8H), 3.33―3.67 (m, 4H), 3.80 (s, 6H), 3.83―4.38 (m, 6H), 4.93―5.08 (m, 1H), 5.08―5.22 (m, 1H), 6.25―6.36 (m, 1H), 6. 36―6.45 (m, 1H), 6.84 (d, *J* = 8.7 Hz, 4H), 7.18―7.40 (m, 10H), 7.54 (s, 1H), 8.06―8.22 (brs, 2H); ³¹P NMR (400 MHz, CDCl₃) δ ―7.54, ―7. 14. 139.7, 139.6; HRMS (FAB) Calculated for C₅₇H₇₃N₇O₁₅NaP₂ [M+Na] ⁺: 1180.4535, Found: 1180.4552. |

### (Example 5: Synthesis and Purification of Oligonucleotide)

An oligonucleotide was synthesized in the following manner using the compound 8 produced in Example 1 as an amidite block. Compounds other than the compound 8 constituting the oligonucleotide were purchased from Proligo unless otherwise stated.

The compound 8 produced in Example 1 and commercially available phosphoramidites, dG(iBu), dC(Bz), and T, were used as the compounds constituting the antisense oligonucleotide. A 0.1 M anhydrous acetonitrile solution containing these amidite blocks was prepared and fed into nS-8 Oligonucleotides Synthesizer manufactured by GeneDesign, Inc., and oligo synthesis was performed trityl-on, on a 0.2 µmol scale. A 5-ethylthio-1H-tetrazole (ETT) activator (0.25 M acetonitrile solution) was used as an activator, and the synthesis was performed according to an ordinary phosphoramidite method.

After completion of the synthesis, the product was treated with a 28% aqueous solution of ammonia at room temperature for 1.5 hours, thus cleaved from the column support, and subsequently allowed to stand at 55°C for 24 hours to thereby deprotect the base moiety and deprotect the phosphate diester moiety. Then, the oligonucleotide was purified on a simplified reverse-phase column (Sep-Pak (registered trademark) Plus C18 Environmental Cartridges manufactured by Waters) and further purified by reverse-phase HPLC.

The composition of the purified oligonucleotide was determined by MALDI-TOF-MS. For this measurement, first, a matrix (1 µL) obtained by mixing an aqueous solution of 3-hydroxypicolinic acid (10 mg/mL) and an aqueous solution of diammonium citrate (1 mg/mL) in a volume ratio of 1:1 was dried on an AnchorChip. An aqueous solution of oligonucleotide (approximately 50 µM, 1 µL) was placed on the AnchorChip and then dried again. After that, MALDI-TOF-MS was performed. The molecular weight was measured in a negative mode, and oligothymidylic acids (7-mer, 9-mer, 11-mer, and 13-mer) were used as external standards. Also, the synthesized oligonucleotide was quantified by measuring ultraviolet absorption at 260 nm using an absorbance measurement apparatus (SHIMADZU UV-1800 manufactured by Shimadzu Corporation).

### (Example 6: Assessment of Double-Strand Forming Ability)

Oligonucleotides having the following sequences were synthesized and purified in a manner similar to that described in Example 5. 5'-d(GCGTTXTTTGCT)-3' (SEQ ID Nos. 1 and 2)
(1) X = thymidine (T) (SEQ ID No. 1)
(2) X = 5'-dimethylthymidine (5'-diMe-T) (SEQ ID No. 2)

During synthesis of the oligonucleotide having the sequence (2) above, the compound 8 was used to arrange thymidine (T) at the fifth position, and 5'-dimethylthymidine (5'-diMe-T) at the sixth position ("X"), in the sequence of the oligonucleotide when counted from the 5' side.

A single-stranded oligo RNA 5'-r(AGCAAAAAACGC)-3' (SEQ ID No. 3) and a single-stranded oligo DNA 5'-d(AGCAAAAAACGC)-3' (SEQ ID No. 4) were used as target strands, and the double-strand forming ability (binding affinity) of each oligonucleotide was examined.

The double-strand forming ability of the oligonucleotides was examined by subjecting the different types of oligonucleotides and the target strands to an annealing treatment to form double strands, and then measuring their Tₘ values. More specifically, a mixed liquid of each oligonucleotide (final concentration: 4 µM) and a phosphate buffer (10 mM, pH 7.2, 130 µL) containing sodium chloride (final concentration: 100 mM) was bathed in boiled water and then slowly cooled to room temperature. After that, the mixed liquid was cooled to 5°C under nitrogen stream before starting the measurement. The temperature was raised to 90°C at a rate of 0.5°C/min while absorbance at 260 nm was plotted at intervals of 0.5°C. The Tₘ value was calculated using a median method, and a mean value of three independent measurements was adopted. Table 12 below shows the results. In Table 12, the results with respect to the single-stranded oligo-RNA are indicated by "ssRNA", the results with respect to the single-stranded oligo-DNA are indicated by "ssDNA", and the Tₘ for each oligonucleotide and the Tₘ temperature change ("ΔTₘ/mod.") per artificially modified nucleic acid base are shown.

**[Table 12]**

| | ssRNA | | ssDNA | |
|---|---|---|---|---|
| sequence (5'-3') | *T*ₘ (°C) | Δ*T*ₘ/mod. (°C) | *T*ₘ (°C) | Δ*T*ₘ/mod. (°C) |
| d(GCGTTTTTTGCT) | 47.4 | - | 51.6 | - |
| d(GCGTTXTTTGCT) | 46.5 | -0.9 | 50.4 | -1.2 |

| | | | | |
|---|---|---|---|---|
| X = **5'-dimethylthymidine** | | | | |

In the case of the 5'-dimethylthymidine-containing oligonucleotide synthesized using the compound 8 (sequence (2) above), for both the single-stranded oligo-DNA and the single-stranded oligo-RNA, the Tₘ values decreased only slightly compared with those of the naturally occurring oligonucleotide (sequence (1) above), which means that the binding affinity was not impaired.

### (Example 7: Assessment of Nuclease-Resistant Ability)

Oligonucleotides having the following 10-mer sequences were synthesized and purified in a manner similar to that described in Example 5, and used as test oligonucleotides. 5'-TTTTTTTTTX-3'
(3) X = phosphorothioate thymidine (ps-T) (phosphorothioate bond (i.e., phosphate groups at the binding sites between nucleotides were sulfurized (substituted by S))
(4) X = 5'-dimethylthymidine (5'-diMe-T)

During synthesis of the oligonucleotide having the sequence (4) above, the compound 8 was used to arrange thymidine (T) at the ninth position, and 5'-dimethylthymidine (5'-diMe-T) at the tenth position ("X"), in the sequence of the oligonucleotide when counted from the 5' side.

To a 50 mM tris-hydrochloric acid buffer (pH 8.0) containing 7.5 µM test oligonucleotide and 10 mM magnesium chloride was added 3'-exonuclease (Crotalus adamanteus venom phosphodiesterase, CAVP) with a concentration of 2.5 µg/ mL, and the mixture was incubated at 37°C. At the start of the incubation (0 minutes) and 2.5, 5, 10, 20, and 40 minutes after the start of the incubation, a 10-µL aliquot was taken from the specimen and analyzed by reverse-phase HPLC to calculate the percentages of uncleaved oligonucleotides. Moreover, the assessment was derived from three independent measurements.

FIG. 1 shows the results. In FIG. 1, solid-black rhombuses indicate the results with respect to the sequence (3) above, and solid-black squares indicate the results with respect to the sequence (4) above. As is clear from FIG. 1, in the present example, the residual ratio of uncleaved oligonucleotides in the phosphorothioated (PS) oligo (sequence (3) above) at 40 minutes after the nuclease treatment decreased to approximately 20% or less, whereas the 5'-dimethylthymidine-containing oligonucleotide (sequence (4) above) synthesized using the compound 8 was not readily degraded, with approximately 80% remaining uncleaved even at 40 minutes after the nuclease treatment. Thus, it was confirmed that, a higher level of nuclease resistance can be acquired by 5'-dimethyl modification than by phosphorothioate modification.

### (Example 8: Assessment of RNase H Activity)

Oligonucleotides having the following sequences were synthesized and purified in a manner similar to that described in Example 5, and used as test oligonucleotides:
(5) 5'-GₘCₘGₘttttttttGₘCₘUₘ-3' (SEQ ID No. 5)
(6) 5'-GₘCₘGₘtttXtttXGₘCₘUₘ-3' (SEQ ID No. 6)
(7) 5'-GₘCₘGₘtXttXttXGₘCₘUₘ-3' (SEQ ID No. 7)
(8) 5'-GₘCₘGₘUₘUₘUₘUₘUₘUₘUₘUₘGₘCₘUₘ-3' (SEQ ID No. 8)
where Gₘ, Cₘ, and Uₘ: 2'-O-methyl modification,
t = thymidine, and
X = 5'-dimethylthymidine (5'-diMe-T).

During synthesis of the oligonucleotides having the sequences (6) and (7) above, the compound 8 was used to arrange thymidine (t) and 5'-dimethylthymidine (5'-diMe-T) side-by-side at the "tX" positions in the respective sequences (the sixth and seventh positions as well as the tenth and eleventh positions in the sequence (6), and the fourth and fifth positions, the seventh and eighth positions, as well as the tenth and eleventh positions in the sequence (7), when counted from the 5' side).

Each test oligonucleotide (60 pmol) and a fluorescein-labeled complementary strand RNA (5'-FAM-r(AGCAAAAAAAACGC)-3') (SEQ ID No. 9) (300 pmol) were dissolved in a mixed solution containing a 50 mM tris-hydrochloric acid buffer (pH 8.0), 3 mM magnesium chloride, and 10 mM dithiothreitol. The specimens were each heated to 65°C and then slowly cooled to room temperature. 3.0 units of RNase H derived from Escherichia coli was added to each specimen, followed by incubation at 37°C. After 40 minutes, a formamide/0.1 M ethylenediaminetetraacetic acid mixed solution (formamide:0.1 M ethylenediaminetetraacetic acid = 14:1 (volume ratio)) was added in an amount that was three times the volume of the specimen to quench the reaction. Cleavage products were analyzed by 20% denaturing polyacrylamide gel electrophoresis. Note that the cleavage ratio was calculated for each sequence based on the fluorescence intensity ratio between bands, with the residual ratio of the sequence (8) being taken as 100%. Table 13 below shows the results.

**[Table 13]**

| | Sequence (5) | Sequence (6) | Sequence (7) |
|---|---|---|---|
| Cleavage Ratio | 96% | 95% | 59% |

The oligonucleotide (sequence (6)) in which 5'-dimethylthymidine was introduced every three thymidines showed a cleavage ratio comparable to that of the oligonucleotide (sequence (5)) that did not contain 5'-dimethylthymidine. Meanwhile, the oligonucleotide (sequence (7)) in which 5'-dimethylthymidine was introduced every two thymidines showed a cleavage ratio somewhat lower than that of the oligonucleotide (sequence (5)) that did not contain 5'-dimethylthymidine, but cleavage was observed. Thus, it was confirmed that 5'-dimethyl modification can also be applied to RNase H-inducible antisense oligonucleotides.

### (Example 9: Assessment of Antisense Activity and Hematotoxicity)

Oligonucleotides having the following sequences were synthesized and purified in a manner similar to that described in Example 5, and used as test oligonucleotides. Two types of test oligonucleotide sequences were designed as antisense oligonucleotides of NR3C1:
ASO1: 5'-G^T^^{m}C^t^c^t^t^t^a^c^c^T^G^G-3' (SEQ ID No. 10)
ASO2: 5'-G^T^^{m}C^t^c^tX^t^a^c^c^T^G^G-3' (SEQ ID No. 11)
ASO3: 5'-A^G^G^t^g^c^t^t^t^g^g^T^^{m}C^T-3' (SEQ ID No. 12)
ASO4: 5'-A^G^G^t^g^c^t^tX^g^g^T^^{m}C^T-3' (SEQ ID No. 13)
where a, g, c, and t = DNA,
A = LNA-A, G = LNA-G, ^{m}C = LNA-5-methylcytosine, T = LNA-T,
X = 5'-dimethylthymidine (5'-diMe-T), and
^ = phosphorothioate bond (i.e., phosphate groups at the binding sites between nucleotides were sulfurized (substituted by S)).

The above-described test oligonucleotides (20 mg/kg) were administered to the tail vein of six-week-old mice (C57BL/6NCrl, male). Saline was administered to control mice. After 96 hours, blood was collected under inhalation anesthesia (isoflurane), and the mice were exsanguinated. After that, livers were collected to measure the liver weight and extract RNA (phenol-chloroform extraction after homogenization with TRIzol). The activities of aspartate transaminase (AST) and alanine transaminase (ALT) in blood were measured using an automated analyzer (JCA-BM6070 manufactured by JEOL Ltd.). In addition, the mRNA expression levels of a target gene NR3C1 and a housekeeping gene GAPDH were measured by real-time PCR (kit used: One Step SYBR PrimeScript RT-PCR Kit (manufactured by Takara Bio Inc.), primer sequences: NR3C1 forward (actgtccagcatgccgctat) (SEQ ID No. 14), NR3C1 reverse (gcagtggcttgctgaattcc) (SEQ ID No. 15), GAPDH forward (gtgtgaacggatttggccgt) (SEQ ID No. 16), and GAPDH reverse (gacaagcttcccattctcgg) (SEQ ID No. 17)).

FIGS. 2 to 5 show the results. FIGS. 2 and 3 are graphs showing the abundances of mRNA of the target gene NR3C1 in mouse livers in the cases where the test oligonucleotides were administered and in the case where saline was administered, and relative values of the mRNA abundance are shown, where the mRNA abundance in the case where saline was administered is taken as 100 (FIG. 2: ASO1 and ASO2; and FIG. 3: ASO3 and ASO4). FIGS. 4 and 5 are graphs showing the activities of aspartate transaminase (AST) and alanine transaminase (ALT) in blood in the cases where the test oligonucleotides were administered and in the case where saline was administered, and relative values of the AST and ALT are shown, where the ALT value and the AST value in the case where the oligonucleotide that did not contain 5'-dimethylthymidine (ASO1 or ASO3) was administered are taken as 100 (FIG. 4: ASO1 and ASO2; and FIG. 5: ASO3 and ASO4).

As shown in FIGS. 2 and 3, ASO1 and ASO2, as well as ASO3 and ASO4, comparably suppressed the RNA expression level when compared with that of the control to which saline was administered. Therefore, the oligonucleotides containing 5'-dimtehylthymidine had substantially comparable antisense activities, compared with the oligonucleotides that did not contain 5'-dimethylthymidine. As shown in FIGS. 4 and 5, ASO2 and ASO4 in which 5'-dimethylthymidine was used significantly reduced the AST and ALT values, which are indicators of hematotoxicity, compared with ASO1 and ASO3 in which 5'-dimethylthymidine was not used. Thus, it was confirmed that 5'-dimethyl modification can reduce hematotoxicity without impairing antisense activities of antisense oligonucleotides.

### (Example 10: Assessment of Nuclease-Resistant Ability of 5'-Diethyl-Modified Nucleic Acid)

Oligonucleotides having the following 10-mer sequences were synthesized and purified in a manner similar to that described in Example 5, except that diethylthymidine (5'-diEt-T) of the compound 20 was used as the compound represented by X, and the resultant oligonucleotides were used as test oligonucleotides.
5'-TTTTTTTTTX-3'
(9) X = phosphorothioate thymidine (ps-T) (phosphorothioate bond (i.e., phosphate groups at the binding sites between nucleotides were sulfurized (substituted by S))
(10) X = 5'-dimethylthymidine (5'-diMe-T)
(11) X = 5'-diethylthymidine (5'-diEt-T)

During synthesis of the oligonucleotide having the sequence (10) or (11) above, the compound 8 or 20 was used to arrange thymidine (T) at the ninth position, and 5'-dimethylthymidine (5'-diMe-T) or 5'-diethylthymidine (5'-diEt-T) at the tenth position ("X"), in the sequence of the oligonucleotide when counted from the 5' side.

To a 50 mM tris-hydrochloric acid buffer (pH 8.0) containing 7.5 µM test oligonucleotide and 10 mM magnesium chloride was added 3'-exonuclease (Crotalus adamanteus venom phosphodiesterase, CAVP) with a concentration of 3.0 µg/ mL, and the mixture was incubated at 37°C. At the start of the incubation (0 minutes) and 2.5, 5, 10, 20, and 40 minutes after the start of the incubation, a 10-µM aliquot was taken from the specimen and analyzed by reverse-phase HPLC to calculate the percentages of uncleaved oligonucleotides. Moreover, the assessment was derived from three independent measurements.

FIG. 6 shows the results. In FIG. 6, solid-black circles indicates the results with respect to the sequence (9) above (PS), solid-black triangles indicate the results with respect to the sequence (10) above (5'diMeT), and solid-white rhombuses indicate the results with respect to the sequence (11) above (5'diEtT). As is clear from FIG. 6, in the present example, the residual ratio of uncleaved oligonucleotides in the phosphorothioated (PS) oligo (sequence (9) above) decreased to approximately 20% or less at 20 minutes after the nuclease treatment and thereafter, whereas the 5'-diethylthymidine-containing oligonucleotide (sequence (11) above) synthesized using the compound 20 was not readily degraded as is the case with the 5'-dimethylthymidine-containing oligonucleotide (sequence (10) above) synthesized using the compound 8, with approximately 60% remaining uncleaved even at 40 minutes after the nuclease treatment. Thus, it was confirmed that, a higher level of nuclease resistance can be acquired not only by 5'-dimethyl modification but also by 5'-diethyl modification than by phosphorothioate modification.

(Example 11: Assessment of Double-Strand Forming Ability of 5'-Diethyl-Modified Nucleic Acid)

Oligonucleotides having the following sequences were synthesized and purified in a manner similar to that described in Example 5, except that diethylthymidine (5'-diEt-T) of the compound 20 was used as the compound represented by X, and the resultant oligonucleotides were used as test oligonucleotides:
(12) 5'-d(GCGTTTTTTGCT)-3' (SEQ ID No. 1)
(13) 5'-d(GCGTTXTTTGCT)-3' (SEQ ID No. 18)
(14) 5'-d(GCGTTXTXTGCT)-3' (SEQ ID No. 19)
where X in the sequences (13) and (14) is 5'-diethylthymidine (5'-diEt-T).

During synthesis of the oligonucleotide having the sequence (13) above, the compound 20 was used to arrange thymidine (T) at the fifth position, and 5'-diethylthymidine (5'-diEt-T) at the sixth position ("X"), in the sequence of the oligonucleotide when counted from the 5' side. For the oligonucleotide having the sequence (14) above, thymidine (T) was arranged at the fifth and seventh positions, and 5'-diethylthymidine (5'-diEt-T) was arranged at the sixth and eighth positions ("X"), when counted from the 5' side.

The single-stranded oligo RNA 5'-r(AGCAAAAAACGC)-3' (SEQ ID No. 3) and the single-stranded oligo DNA 5'-d(AGCAAAAAACGC)-3' (SEQ ID No. 4) were used as target strands, and the double-strand forming ability (binding affinity) of each oligonucleotide was examined.

The double-strand forming ability of the oligonucleotides was assessed in a manner similar to that of Example 6 above. Table 14 below shows the results. In Table 14, the results with respect to the single-stranded oligo-RNA are indicated by "ssRNA", the results with respect to the single-stranded oligo-DNA are indicated by "ssDNA", and the Tₘ for each oligonucleotide and the Tₘ temperature change ("ΔTₘ/mod.") per artificially modified nucleic acid base are shown.

**[Table 14]**

| | ssRNA | | ssDNA | |
|---|---|---|---|---|
| sequence (5'-3') | *T*ₘ (°C) | Δ*T*ₘ/mod. (°C) | *T*ₘ (°C) | Δ*T*ₘ/mod. (°C) |
| d(GCGTTTTTTGCT) | 45.8 | ― | 49.8 | ― |
| d(GCGTTXTTTGCT) | 43.8 | ―2.0 | 47.8 | ―2.0 |
| d(GCGTTXTXTGCT) | 43.0 | ―1.4 | 43.6 | ―3.1 |

| | | | | |
|---|---|---|---|---|
| **X = 5'-diethylthymidine** | | | | |

In the cases of the 5'-diethylthymidine-containing oligonucleotides synthesized using the compound 20 (sequences (13) and (14) above), for both the single-stranded oligo-DNA and the single-stranded oligo-RNA, the Tₘ values decreased only slightly compared with those of the naturally occurring oligonucleotide (sequence (12) above), which means that the binding affinity was not impaired. When the amount of 5'-diethylthymidine was increased, the double-bond forming ability particularly for RNA was not impaired.

### Industrial Applicability

According to the present invention, provided are a novel 5'-modified nucleoside that is usable as a substitute for a phosphorothioate-modified nucleic acid, and a nucleotide using the 5'-modified nucleoside. The 5'-modified nucleoside of the present invention also has excellent industrial productivity because a diastereomer separation step is not involved in the production process thereof. An oligonucleotide obtained using the 5'-modified nucleoside of the present invention is useful as, for example, materials for nucleic acid drugs.

## Claims

1. A compound represented by a formula (I) below or a salt thereof: 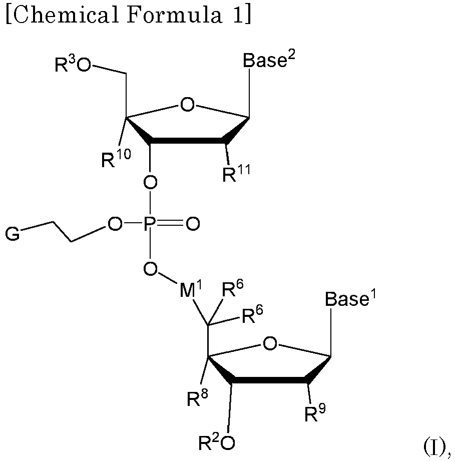 wherein Base¹ and Base² each independently represent a purin-9-yl group that may have any one or more substituents selected from an a group, or a 2-oxo-1,2-dihydropyrimidin-1-yl group that may have any one or more substituents selected from the α group, the α group consisting of a hydroxy group, a hydroxy group protected by a protecting group for nucleic acid synthesis, a C₁ to C₆ linear alkyl group, a C₁ to C₆ linear alkoxy group, a mercapto group, a mercapto group protected by a protecting group for nucleic acid synthesis, a C₁ to C₆ linear alkylthio group, an amino group, a C₁ to C₆ linear alkylamino group, an amino group protected by a protecting group for nucleic acid synthesis, and halogen atoms;
G is a cyano group or a nitro group;
R² and R³ each independently represent a hydrogen atom, a hydroxy group protecting group for nucleic acid synthesis, a C₁ to C₇ alkyl group that may form a branch or a ring, a C₂ to C₇ alkenyl group that may form a branch or a ring, a C₃ to C₁₀ aryl group that may have any one or more substituents selected from the a group and that may contain a heteroatom, an aralkyl group with a C₃ to C₁₂ aryl moiety that may have any one or more substituents selected from the a group and that may conatin a heteroatom, an acyl group that may have any one or more substituents selected from the a group, a silyl group that may have any one or more substituents selected from the a group, a phosphate group that may have any one or more substituents selected from the a group, a phosphate group protected by a protecting group for nucleic acid synthesis, or -P(R⁴)R⁵, wherein R⁴ and R⁵ each independently represent a hydroxy group, a hydroxy group protected by a protecting group for nucleic acid synthesis, a mercapto group, a mercapto group protected by a protecting group for nucleic acid synthesis, an amino group, a C₁ to C₆ alkoxy group, a C₁ to C₆ alkylthio group, a C₁ to C₆ cyanoalkoxy group, or a dialkylamino group having a C₁ to C₆ alkyl group;
R⁶ is a C₁ or C₂ alkyl group atoms that may be substituted with a halogen atom;
R⁸ is a hydrogen atom, and R⁹ is a hydrogen atom or a halogen atom; a C₁ to C₆ linear alkoxy group that may be substituted with a C₁ to C₆ linear alkoxy group; or -OR¹², wherein R¹² is a hydrogen atom or a hydroxy group protecting group for nucleic acid synthesis, or
R⁸ and R⁹ together represent a divalent group represented by a formula below: 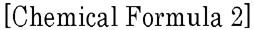 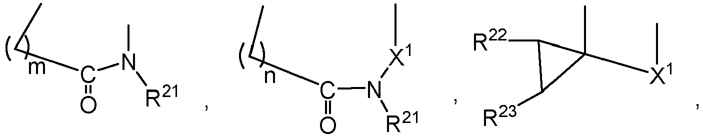 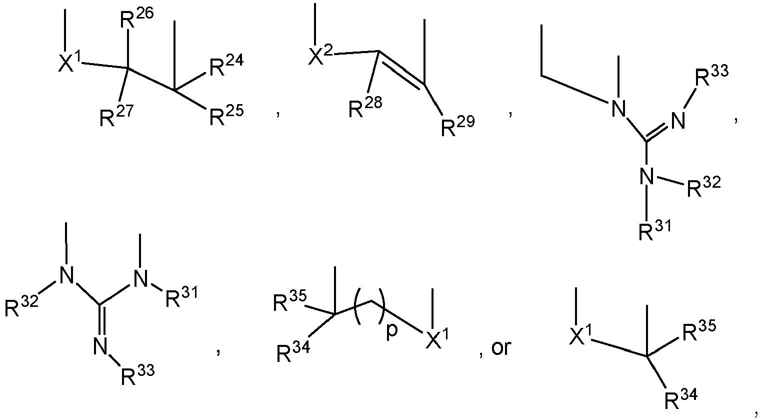 wherein R²¹ is a hydrogen atom, a C₁ to C₆ alkyl group that may form a branch or a ring, a C₂ to C₆ alkenyl group that may form a branch or a ring, a C₃ to C₁₀ aryl group that may have any one or more substituents selected from the a group and that may contain a heteroatom, an aralkyl group with a C₃ to C₁₂ aryl moiety that may have any one or more substituents selected from the a group and that may contain a heteroatom, or an amino group protecting group for nucleic acid synthesis;
R²² and R²³ are each independently a hydrogen atom, a C₁ to C₆ alkyl group that may be substituted with a C₃ to C₁₂ aryl group that may contain a heteroatom, and that may may be branched or form a ring, or an aralkyl group with a C₃ to C₁₂ aryl moiety that may contain a heteroatom, or
R²² and R²³ together represent -(CH₂)_{q1}-, wherein q1 is an integer from 2 to 5;
R²⁴ and R²⁵ are each independently a group selected from the group consisting of a hydrogen atom, a hydroxy group, a C₁ to C₆ alkyl group that may be buranched or form a ring, a C₁ to C₆ alkoxy group that may be branched or form a ring, an amino group, and an amino group protected by a protecting group for nucleic acid synthesis, or
R²⁴ and R²⁵ together represent =C(R³⁶)R³⁷, wherein R³⁶ and R³⁷ each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R²⁶ and R²⁷ are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁸ is a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁹ is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R³¹, R³², and R³³ are each independently a hydrogen atom, a C₁ to C₆ alkyl group that may be branched or form a ring, or an amino group protecting group for nucleic acid synthesis;
R³⁴ and R³⁵ are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
m is an integer from 0 to 2;
n is an integer of 0 or 1;
p is an integer of 0 or 1;
X¹ is an oxygen atom, a sulfur atom, or an amino group; and
X² is an oxygen atom or a sulfur atom;
R¹⁰ is a hydrogen atom, and R¹¹ is a hydrogen atom or a halogen atom; a C₁ to C₆ linear alkoxy group that may be substituted with a C₁ to C₆ linear alkoxy group; or -OR^{12b}, wherein R^{12b} is a hydrogen atom or a hydroxy group protecting group for nucleic acid synthesis, or
R¹⁰ and R¹¹ together represent a divalent group represented by a formula below: 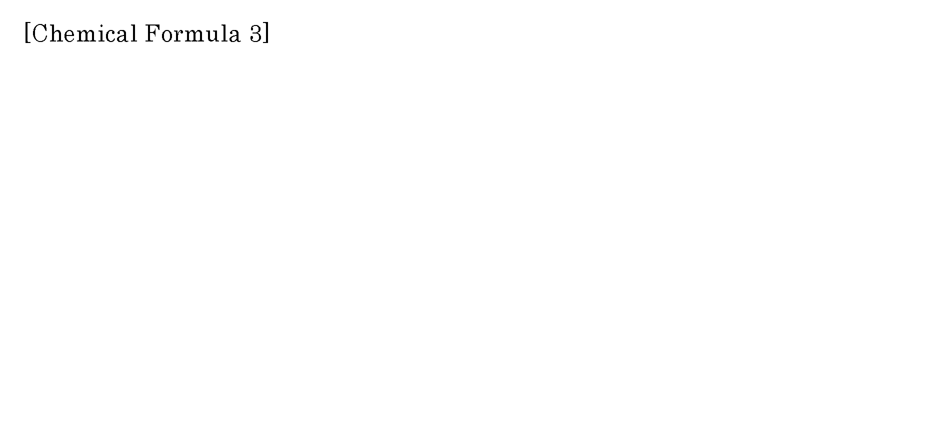 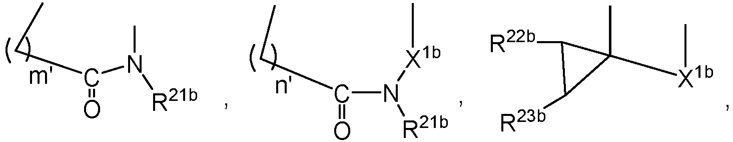 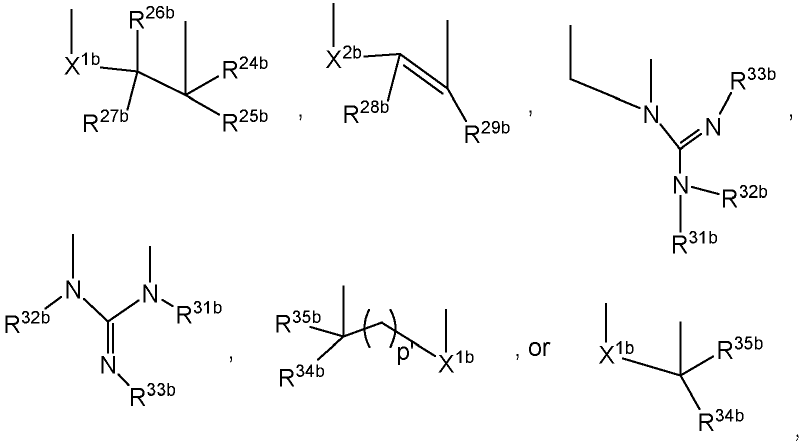
wherein R^{21b} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the a group and that may have a heteroatom, an aralkyl group with a C₃ to C₁₂ aryl moiety that may have any one or more substituents selected from the a group and that may have a heteroatom, or an amino group protecting group for nucleic acid synthesis;
R^{22b} and R^{23b} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22b} and R^{23b} together represent -(CH₂)_{q2}-, wherein q2 is an integer from 2 to 5;
R^{24b} and R^{25b} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24b} and R^{25b} together represent =C(R^{36b})R^{37b}, wherein R^{36b} and R^{37b} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26b} and R^{27b} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28b} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29b} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31b}, R^{32b}, and R^{33b} are each independently a hydrogen atom, a C₁ to C₆ alkyl group that may be branched or form a ring, or an amino group protecting group for nucleic acid synthesis;
R^{34b} and R^{35b} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
m' is an integer from 0 to 2;
n' is an integer of 0 or 1;
p' is an integer of 0 or 1;
X^{1b} is an oxygen atom, a sulfur atom, or an amino group; and
X^{2b} is an oxygen atom or a sulfur atom; and
M¹ represents a single bond or a formula below: 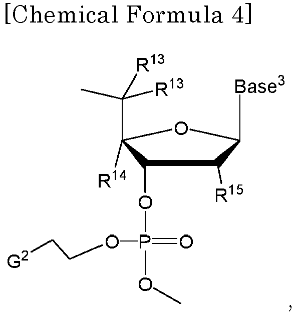 wherein Base³ represents a purin-9-yl group that may have any one or more substituents selected from the a group, or a 2-oxo-1,2-dihydropyrimidin-1-yl group that may have any one or more substituents selected from the a group;
G² is a cyano group or a nitro group;
R¹³ is a C₁ or C₂ alkyl group that may be substituted with a halogen atom;
R¹⁴ is a hydrogen atom, and R¹⁵ is a hydrogen atom or a halogen atom; a C₁ to C₆ linear alkoxy group that may be substituted with a C₁ to C₆ linear alkoxy group; or -OR^{12c}, wherein R^{12c} is a hydrogen atom or a hydroxy group protecting group for nucleic acid synthesis, or
R¹⁴ and R¹⁵ together represent a divalent group represented by a formula below: 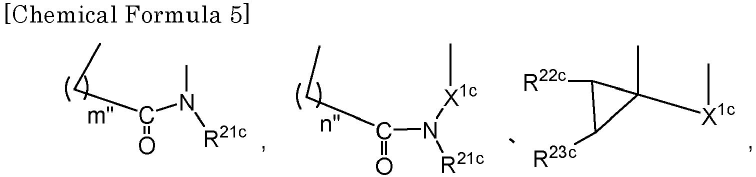 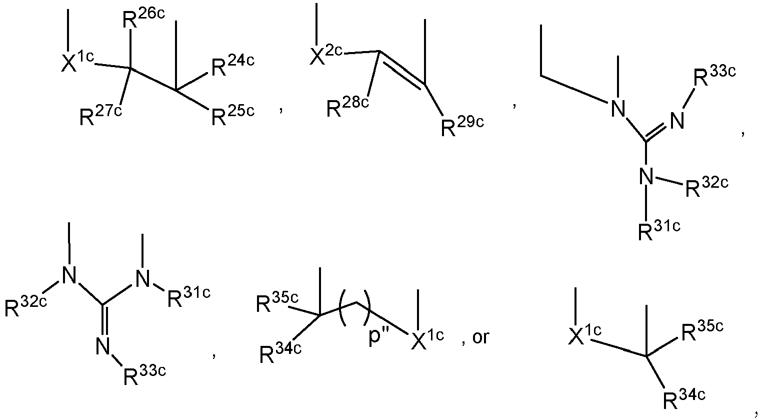 wherein R^{21c} is a hydrogen atom, a C₁ to C₆ alkyl group that may be branched or form a ring, a C₂ to C₆ alkenyl group that may be branched or form a ring, a C₃ to C₁₂ aryl group that may have any one or more substituents selected from the α group and that may contain a heteroatom, an aralkyl group with a C₃ to C₁₂ aryl moiety that may have any one or more substituents selected from the a group and taht may contain a heteroatom, or an amino group protecting group for nucleic acid synthesis;
R^{22c} and R^{23c} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22c} and R^{23c} together represent -(CH₂)_{q3}-, wherein q3 is an integer from 2 to 5;
R^{24c} and R^{25c} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24c} and R^{25c} together represent =C(R^{36c})R^{37c}, wherein R^{36c} and R^{37c} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26c} and R^{27c} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28c} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29c} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31c}, R^{32c}, and R^{33c} are each independently a hydrogen atom, a C₁ to C₆ alkyl group that may be branched or form a ring, or an amino group protecting group for nucleic acid synthesis;
R^{34c} and R^{35c} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
m" is an integer from 0 to 2;
n" is an integer of 0 or 1;
p" is an integer of 0 or 1;
X^{1c} is an oxygen atom, a sulfur atom, or an amino group; and
X^{2c} is an oxygen atom or a sulfur atom.

2. The compound or salt thereof according to claim 1, wherein M¹ in the formula (I) is a single bond.

3. The compound or salt thereof according to claim 2, wherein the Base¹ and the Base ² in the formula (I) are each independently a 6-aminopurin-9-yl group, a 2,6-diaminopurin-9-yl group, a 2-amino-6-chloropurin-9-yl group, a 2-amino-6-fluoropurin-9-yl group, a 2-amino-6-bromopurin-9-yl group, a 2-amino-6-hydroxypurin-9-yl group, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl group, a 4-amino-2-oxo-5-fluoro-1,2-dihydropyrimidin-1-yl group, a 4-amino-2-oxo-5-chloro-1,2-dihydropyrimidin-1-yl group, a 2-oxo-4-methoxy-1,2-dihydropyrimidin-1-yl group, a 2-oxo-4 mercapto-1,2-dihydropyrimidin-1-yl group, a 2-oxo-4-hydroxy-1,2-dihydropyrimidin-1-yl group, a 2-oxo-4-hydroxy-5-methyl-1,2-dihydropyrimidin-1-yl group, or a 4-amino-5-methyl-2-oxo-1,2-dihydropyrimidin-1-yl group.

4. The compound or salt thereof according to claim 2, wherein the Base¹ and the Base² in the formula (I) are each independently a group selected from the group consisting of formulae below:

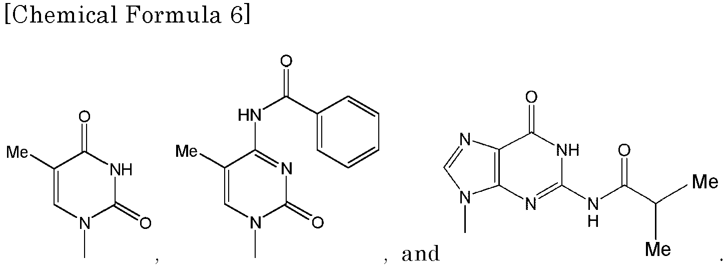

5. The compound or salt thereof according to any one of claims 2 to 4, wherein R⁶ in the formula (I) is a methyl group or an ethyl group.

6. The compound or salt thereof according to any one of claims 2 to 5, wherein R⁸, R⁹, R¹⁰, and R¹¹ in the formula (I) are all hydrogen atoms.

7. An oligonucleotide containing at least one nucleoside structure represented by a formula (II) below or a pharmacologically acceptable salt thereof: 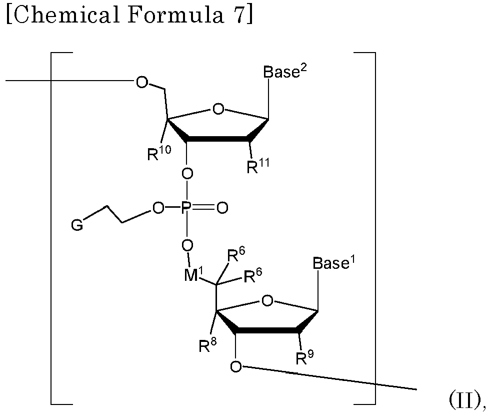 wherein Base¹ and Base² each independently represent a purin-9-yl group that may have any one or more substituents selected from an a group, or a 2-oxo-1,2-dihydropyrimidin-1-yl group that may have any one or more substituents selected from the a group, the a group consisting of a hydroxy group, a hydroxy group protected by a protecting group for nucleic acid synthesis, a C₁ to C₆ linear alkyl group, a C₁ to C₆ linear alkoxy group, a mercapto group, a mercapto group protected by a protecting group for nucleic acid synthesis, a C₁ to C₆ linear alkylthio group, an amino group, a C₁ to C₆ linear alkylamino group, an amino group protected by a protecting group for nucleic acid synthesis, and halogen atoms;
G is a cyano group or a nitro group;
R⁶ is a C₁ or C₂ alkyl group that may be substituted with a halogen atom;
R⁸ is a hydrogen atom, and R⁹ is a hydrogen atom or a halogen atom; a C₁ to C₆ linear alkoxy group that may be substituted with a C₁ to C₆ linear alkoxy group; or -OR¹², wherein R¹² is a hydrogen atom or a hydroxy group protecting group for nucleic acid synthesis, or
R⁸ and R⁹ together represent a divalent group represented by a formula below: 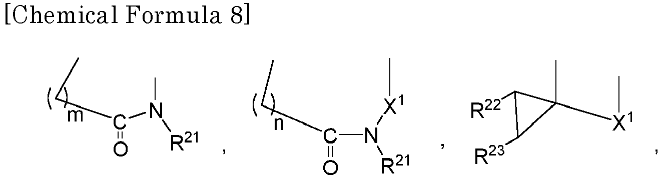 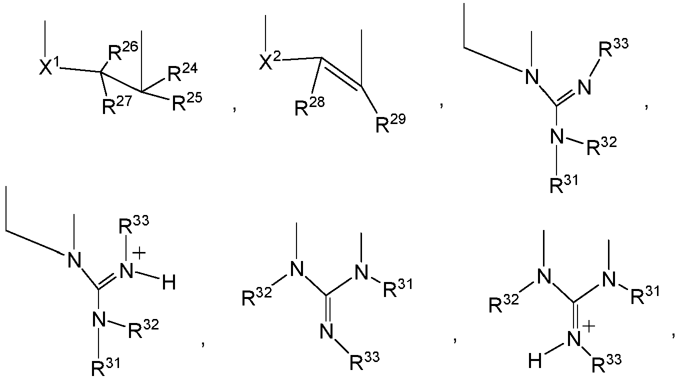 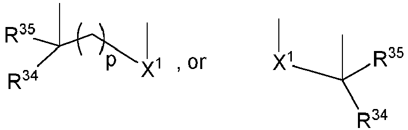 wherein R²¹ is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the a group and that may contain a heteroatom, an aralkyl group with a C₃ to C₁₂ aryl moiety that may have any one or more substituents selected from the a group and that may contain a heteroatom, or an amino group protecting group for nucleic acid synthesis;
R²² and R²³ are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R²² and R²³ together represent -(CH₂)_{q1}-, wherein q1 is an integer from 2 to 5;
R²⁴ and R²⁵ are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R²⁴ and R²⁵ together represent =C(R³⁶)R³⁷, wherein R³⁶ and R³⁷ each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R²⁶ and R²⁷ are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁸ is a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁹ is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R³¹, R³², and R³³ are each independently a hydrogen atom, a C₁ to C₆ alkyl group that may be branched or form a ring, or an amino group protecting group for nucleic acid synthesis;
R³⁴ and R³⁵ are each independently a hydrogen atom, a hydroxy group, a C₁ to C₆ alkyl group that may be branche or form a ring, a C₁ to C₆ alkoxy group that may be branched or form a ring, an amino group, or an amino group protected by a protecting group for nucleic acid synthesis;
m is an integer from 0 to 2;
n is an integer of 0 or 1;
p is an integer of 0 or 1;
X¹ is an oxygen atom, a sulfur atom, or an amino group; and
X² is an oxygen atom or a sulfur atom;
R¹⁰ is a hydrogen atom, and R¹¹ is a hydrogen atom or a halogen atom; a C₁ to C₆ linear alkoxy group that may be substituted with a C₁ to C₆ linear alkoxy group; or -OR^{12b}, wherein R^{12b} is a hydrogen atom or a hydroxy group protecting group for nucleic acid synthesis, or
R¹⁰ and R¹¹ together represent a divalent group represented by a formula below: 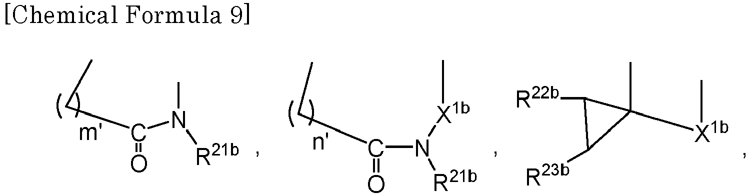 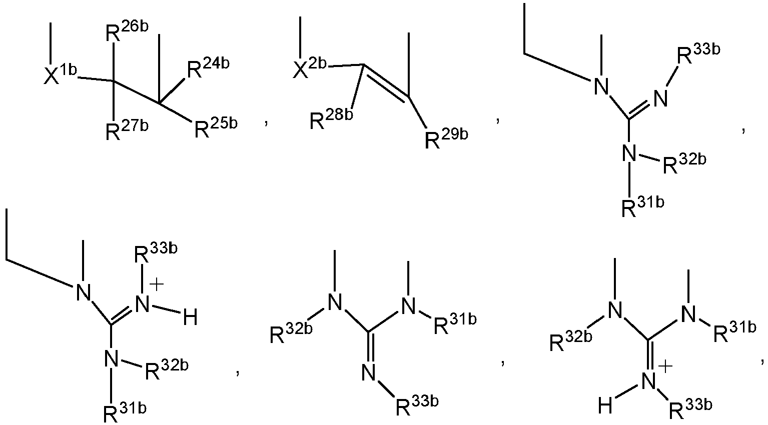 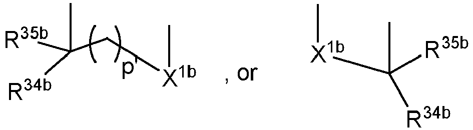 wherein R^{21b} is a hydrogen atom, a C₁ to C₆ alkyl group hat may be branched or form a ring, a C₂ to C₆ alkenyl group that may be branched or form a ring, a C₃ to C₁₀ aryl group that may have any one or more substituents selected from the a group and that may contain a heteroatom, an aralkyl group with a C₃ to C₁₂ aryl moiety that may have any one or more substituents selected from the a group and that may contain a heteroatom, or an amino group protecting group for nucleic acid synthesis;
R^{22b} and R^{23b} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22b} and R^{23b} together represent -(CH₂)_{q2}-, wherein q2 is an integer from 2 to 5;
R^{24b} and R^{25b} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24b} and R^{25b} together represent =C(R^{36b})R^{37b}, wherein R^{36b} and R^{37b} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26b} and R^{27b} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28b} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29b} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31b}, R^{32b}, and R^{33b} are each independently a hydrogen atom, a C₁ to C₆ alkyl group that may be branched or form a ring, or an amino group protecting group for nucleic acid synthesis;
R^{34b} and R^{35b} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
m' is an integer from 0 to 2;
n' is an integer of 0 or 1;
p' is an integer of 0 or 1;
X^{1b} is an oxygen atom, a sulfur atom, or an amino group; and
X^{2b} is an oxygen atom or a sulfur atom; and
M¹ represents a single bond or a formula below: 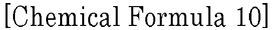 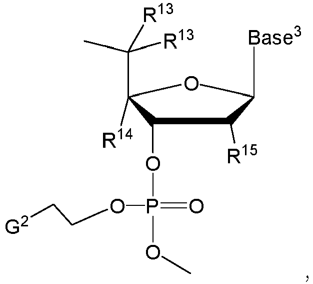 wherein Base³ represents a purin-9-yl group that may have any one or more substituents selected from the a group, or a 2-oxo-1,2-dihydropyrimidin-1-yl group that may have any one or more substituents selected from the a group;
G² is a cyano group or a nitro group;
R¹³ is a C₁ or C₂ alkyl group that may be substituted with a halogen atom;
R¹⁴ is a hydrogen atom, and R¹⁵ is a hydrogen atom or a halogen atom; a C₁ to C₆ linear alkoxy group that may be substituted with a C₁ to C₆ linear alkoxy group; or -OR^{12c}, wherein R^{12c} is a hydrogen atom or a hydroxy group protecting group for nucleic acid synthesis, or
R¹⁴ and R¹⁵ together represent a divalent group represented by a formula below: 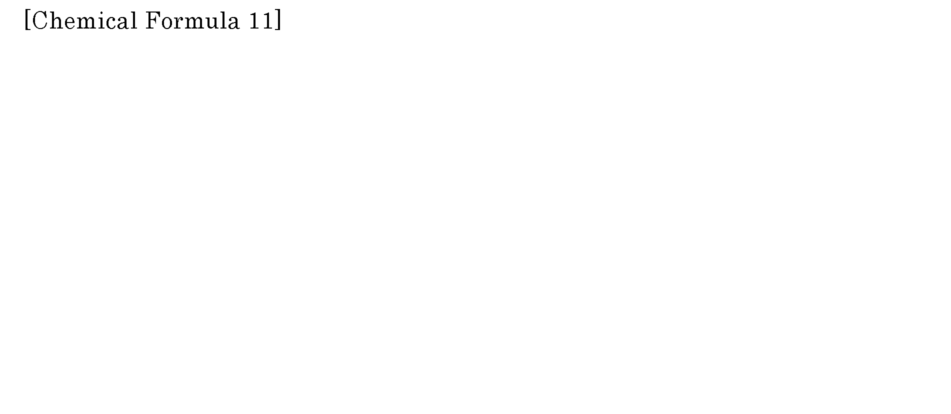 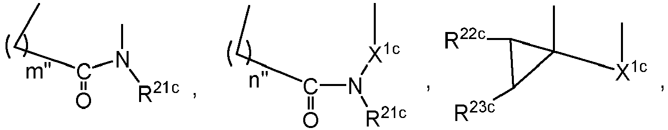 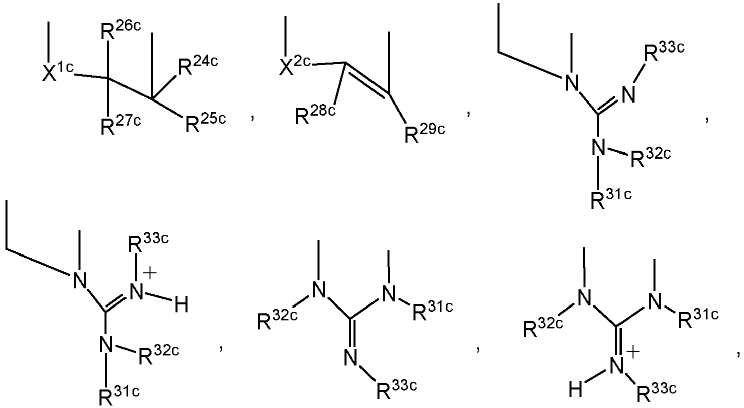 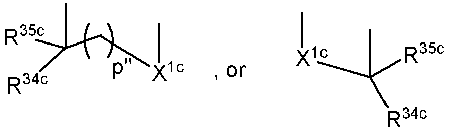 wherein R^{21c} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the a group and that may contain a heteroatom, an aralkyl group with a C₃ to C₁₂ aryl moiety that may have any one or more substituents selected from the a group and that may contain a heteroatom, or an amino group protecting group for nucleic acid synthesis;
R^{22c} and R^{23c} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety and that may contain a heteroatom, or
R^{22c} and R^{23c} together represent -(CH₂)_{q3}-, wherein q3 is an integer from 2 to 5;
R^{24c} and R^{25c} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R24c and R^{25c} together represent =C(R^{36c})R^{37c}, wherein R^{36c} and R^{37c} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26c} and R^{27c} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28c} is a hydrogen atom, a C₁ to C₆ alkyl group that may be branche or form a ring, a C₁ to C₆ alkoxy group that may be branched or form a ring, or a C₁ to C₆ linear or branched alkylthio group;
R^{29c} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31c}, R^{32c}, and R^{33c} are each independently a hydrogen atom, a C₁ to C₆ alkyl group that may be branched or form a ring, or an amino group protecting group for nucleic acid synthesis;
R^{34c} and R^{35c} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
m" is an integer from 0 to 2;
n" is an integer of 0 or 1;
p" is an integer of 0 or 1;
X^{1c} is an oxygen atom, a sulfur atom, or an amino group; and
X^{2c} is an oxygen atom or a sulfur atom.

8. The oligonucleotide or pharmacologically acceptable salt thereof according to claim 7, wherein M¹ in the formula (II) is a single bond.

9. The oligonucleotide or pharmacologically acceptable salt thereof according to claim 8, wherein R⁶ in the formula (II) is a methyl group or an ethyl group.

10. The oligonucleotide or pharmacologically acceptable salt thereof according to claim 8 or 9, wherein R⁸, R⁹, R¹⁰, and R¹¹ in the formula (II) are all hydrogen atoms.

11. A method for producing the oligonucleotide or pharmacologically acceptable salt thereof according to claim 7, which comprises:
synthesizing an oligonucleotide using a compound represented by a formula (I) below or a pharmacologically acceptable salt thereof: 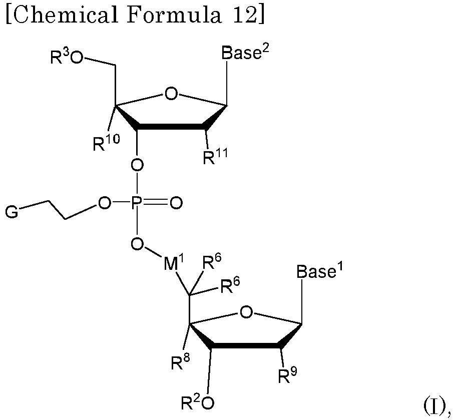 wherein Base¹ and Base² each independently represent a purin-9-yl group that may heve any one or more substituents selected from an a group, or a 2-oxo-1,2-dihydropyrimidin-1-yl group that may have any one or more substituents selected from the α group, the a group consisting of a hydroxy group, a hydroxy group protected by a protecting group for nucleic acid synthesis, a C₁ to C₆ linear alkyl group, a C₁ to C₆ linear alkoxy group, a mercapto group, a mercapto group protected by a protecting group for nucleic acid synthesis, a C₁ to C₆ linear alkylthio group, an amino group, a C₁ to C₆ linear alkylamino group, an amino group protected by a protecting group for nucleic acid synthesis, and halogen atoms;
G is a cyano group or a nitro group;
R² and R³ each independently represent a hydrogen atom, a hydroxy group protecting group for nucleic acid synthesis, a C₁ to C₇ alkyl group that may be branched or form a ring, a C₂ to C₇ alkenyl group that may be branched or form a ring, a C₃ to C₁₀ aryl group that may have any one or more substituents selected from the a group and that may contain a heteroatom, an aralkyl group with a C₃ to C₁₂ aryl moiety that may have any one or more substituents selected from the a group and that may contain a heteroatom, an acyl group that may have any one or more substituents selected from the a group, a silyl group that may have any one or more substituents selected from the a group, a phosphate group that may have any one or more substituents selected from the a group, a phosphate group protected by a protecting group for nucleic acid synthesis, or -P(R⁴)R⁵, wherein R⁴ and R⁵ each independently represent a hydroxy group, a hydroxy group protected by a protecting group for nucleic acid synthesis, a mercapto group, a mercapto group protected by a protecting group for nucleic acid synthesis, an amino group, a C₁ to C₆ alkoxy group, a C₁ to C₆ alkylthio group, a C₁ to C₆ cyanoalkoxy group, or a dialkylamino group having a C₁ to C₆ alkyl group;
R⁶ is a C₁ or C₂ alkyl group that may be subtituted with a halogen atom;
R⁸ is a hydrogen atom, and R⁹ is a hydrogen atom or a halogen atom; a C₁ to C₆ linear alkoxy group that may be substituted with a C₁ to C₆ linear alkoxy group; or -OR¹², wherein R¹² is a hydrogen atom or a hydroxy group protecting group for nucleic acid synthesis, or
R⁸ and R⁹ together represent a divalent group represented by a formula below: 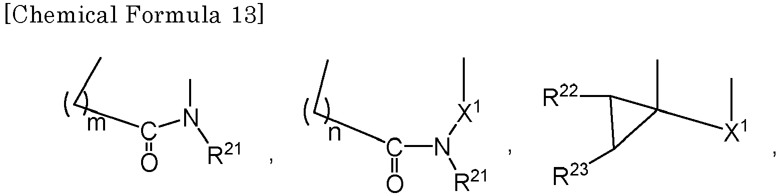 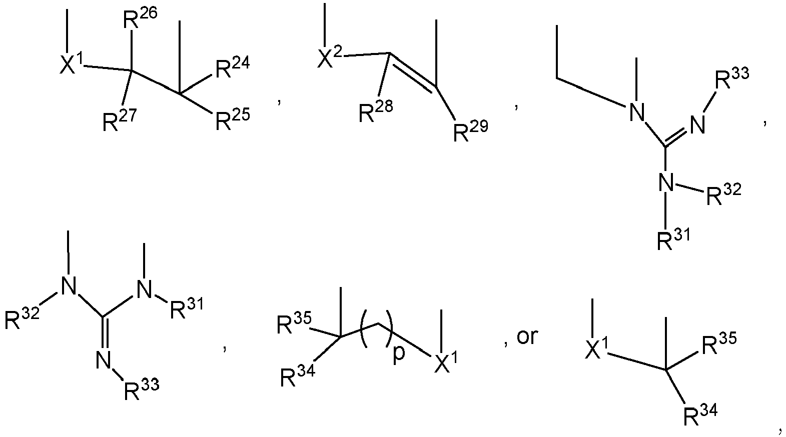 wherein R²¹ is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the a group and that may contain a heteroatom, an aralkyl group with a C₃ to C₁₂ aryl moiety that may have any one or more substituents selected from the a group and that may contain a heteroatom, or an amino group protecting group for nucleic acid synthesis;
R²² and R²³ are each independently a hydrogen atom, a C₁ to C₆ alkyl group that may be substituted with a C₃ to C₁₂ aryl group that conatain a heteroatom, and that may be branched or form a a ring, or an aralkyl group with a C₃ to C₁₂ aryl moiety that may contain a heteroatom, or
R²² and R²³ together represent -(CH₂)_{q1}-, wherein q1 is an integer from 2 to 5;
R²⁴ and R²⁵ are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R²⁴ and R²⁵ together represent =C(R³⁶)R³⁷, wherein R³⁶ and R³⁷ each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R²⁶ and R²⁷ are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁸ is a hydrogen atom, a C₁ to C₆ alkyl group that may be branched or form a ring, a C₁ to C₆ alkoxy group that may be branched or form a ring, or a C₁ to C₆ linear or branched alkylthio group;
R²⁹ is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R³¹, R³², and R³³ are each independently a hydrogen atom, a C₁ to C₆ alkyl group that may be branched or form a ring, or an amino group protecting group for nucleic acid synthesis;
R³⁴ and R³⁵ are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
m is an integer from 0 to 2;
n is an integer of 0 or 1;
p is an integer of 0 or 1;
X¹ is an oxygen atom, a sulfur atom, or an amino group; and
X² is an oxygen atom or a sulfur atom;
R¹⁰ is a hydrogen atom, and R¹¹ is a hydrogen atom or a halogen atom; a C₁ to C₆ linear alkoxy group that may be substituted with a C₁ to C₆ linear alkoxy group; or -OR^{12b}, wherein R^{12b} is a hydrogen atom or a hydroxy group protecting group for nucleic acid synthesis, or
R¹⁰ and R¹¹ together represent a divalent group represented by a formula below: 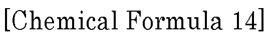 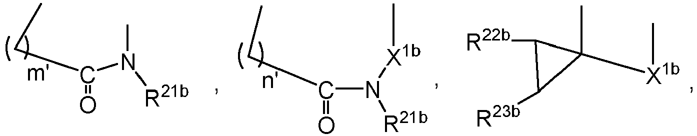 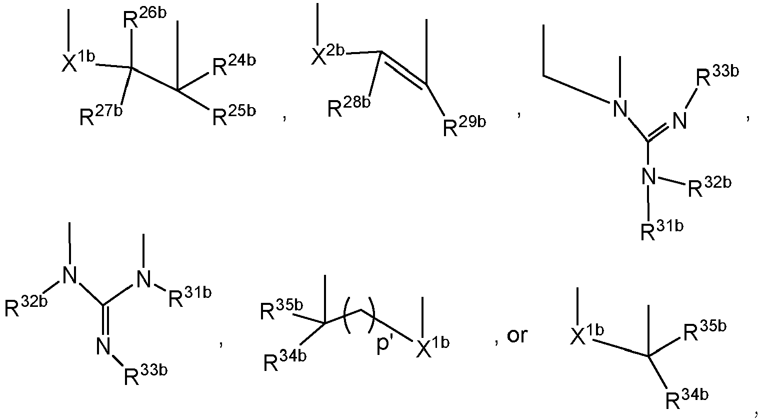
wherein R^{21b} is a hydrogen atom, a C₁ to C₆ alkyl group that may be branched or form a ring, a C₂ to C₆ n alkenyl group that may be branched or form a ring, a C₃ to C₁₀ aryl group that may have any one or more substituents selected from the a group and that may contain a heteroatom, an aralkyl group with a C₃ to C₁₂ aryl moiety that may have any one or more substituents selected from the a group and that may contain a heteroatom, or an amino group protecting group for nucleic acid synthesis;
R^{22b} and R^{23b} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22b} and R^{23b} together represent -(CH₂)_{q2}-, wherein q2 is an integer from 2 to 5;
R^{24b} and R^{25b} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24b} and R^{25b} together represent =C(R^{36b})R^{37b}, wherein R^{36b} and R^{37b} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26b} and R^{27b} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28b} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29b} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31b}, R^{32b}, and R^{33b} are each independently a hydrogen atom, a C₁ to C₆ alkyl group that may be branched or form a ring, or an amino group protecting group for nucleic acid synthesis;
R^{34b} and R^{35b} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
m' is an integer from 0 to 2;
n' is an integer of 0 or 1;
p' is an integer of 0 or 1;
X^{1b} is an oxygen atom, a sulfur atom, or an amino group; and
X^{2b} is an oxygen atom or a sulfur atom; and
M¹ represents a single bond or a formula below: 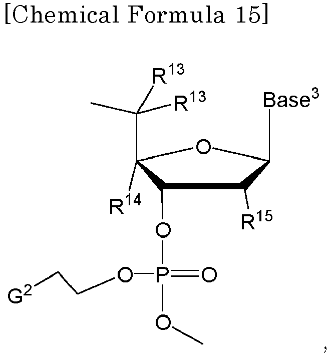 wherein Base³ represents a purin-9-yl group that may have any one or more substituents selected from the a group, or a 2-oxo-1,2-dihydropyrimidin-1-yl group that may have any one or more substituents selected from the a group;
G² is a cyano group or a nitro group;
R¹³ is a C₁ or C₂ alkyl group that may be substituted with a halogen atom;
R¹⁴ is a hydrogen atom, and R¹⁵ is a hydrogen atom or a halogen atom; a C₁ to C₆ linear alkoxy group that may be substituted with a C₁ to C₆ linear alkoxy group; or -OR^{12c}, wherein R^{12c} is a hydrogen atom or a hydroxy group protecting group for nucleic acid synthesis, or
R¹⁴ and R¹⁵ together represent a divalent group represented by a formula below: 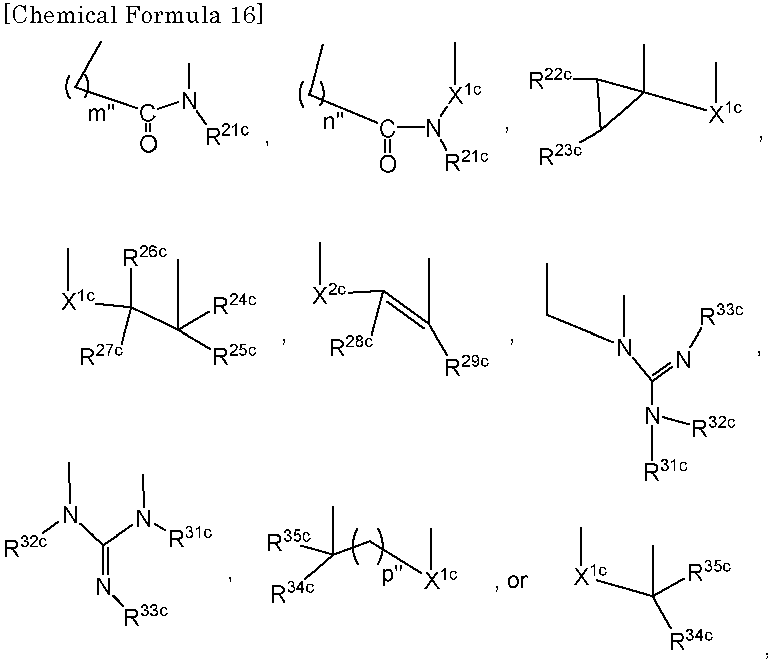 wherein R^{21c} is a hydrogen atom, a C₁ to C₆ alkyl group that may be branched or form a ring, a C₂ to C₆ alkenyl group that may be branched or form a ring, a C₃ to C₁₀ aryl group that may have any one or more substituents selected from the a group and that may contain a heteroatom, an aralkyl group with a C₃ to C₁₂ aryl moiety that may have any one or more substituents selected from the a group and that may contain a heteroatom, or an amino group protecting group for nucleic acid synthesis;
R^{22c} and R^{23c} are each independently a hydrogen atom, a C₁ to C₆ alkyl group that may be substituted with a C₃ to C₁₂ aryl group that may contain a heteroatom, and that may be branched or form a ring, or an aralkyl group with a C₃ to C₁₂ aryl moiety that may contain a heteroatom, or
R^{22c} and R^{23c} together represent -(CH₂)_{q3}-, wherein q3 is an integer from 2 to 5;
R^{24c} and R^{25c} are each independently a group selected from the group consisting of a hydrogen atom, a hydroxy group, a C₁ to C₆ alkyl group that may be branched or form a ring, a C₁ to C₆ alkoxy group that may be branched or form a ring, an amino group, and an amino group protected by a protecting group for nucleic acid synthesis, or
R^{24c} and R^{25c} together represent =C(R^{36c})R^{37c}, wherein R^{36c} and R^{37c} each independently represent a hydrogen atom, a hydroxy group, a hydroxy group protected by a protecting group for nucleic acid synthesis, a mercapto group, a mercapto group protected by a protecting group for nucleic acid synthesis, an amino group, a C₁ to C₆ linear or branched alkoxy group, a C₁ to C₆ linear or branched alkylthio group, a C₁ to C₆ cyanoalkoxy group, or a C₁ to C₆ linear or branched alkylamino group;
R^{26c} and R^{27c} are each independently a hydrogen atom, a C₁ to C₆ alkyl group that may be branched or form a ring, a C₁ to C₆ alkoxy group that may be branched or form a ring, or a C₁ to C₆ linear or branched alkylthio group;
R^{28c} is a hydrogen atom, a C₁ to C₆ alkyl group that may be branched or form a ring, a C₁ to C₆ alkoxy group that may be branched or form a ring, or a C₁ to C₆ linear or branched alkylthio group;
R^{29c} is a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
R^{31c}, R^{32c}, and R^{33c} are each independently a hydrogen atom, a C₁ to C₆ alkyl group that may be branched or form a ring, or an amino group protecting group for nucleic acid synthesis;
R^{34c} and R^{35c} are each independently a hydrogen atom, a hydroxy group, a C₁ to C₆ alkyl group that may be branched or form a ring, a C₁ to C₆ alkoxy group that may be branched or form a ring, an amino group, or an amino group protected by a protecting group for nucleic acid synthesis;
m" is an integer from 0 to 2;
n" is an integer of 0 or 1;
p" is an integer of 0 or 1;
X^{1c} is an oxygen atom, a sulfur atom, or an amino group; and
X^{2c} is an oxygen atom or a sulfur atom.


SEQUENCE LISTING

<110>  Osaka University

<120>  5′ position modified nucleoside and nucleotide using the same

<130>  P2-19O07103

<150>  JP 2019-23514
<151>  2019-02-13

<160>  19

<170>  PatentIn version 3.5

<210>  1
<211>  12
<212>  DNA
<213>  Artificial Sequence

<220>
<223>  Sequence (1)

<400>  1
gcgtttttg ct                                                          12


<210>  2
<211>  12
<212>  DNA
<213>  Artificial Sequence

<220>
<223>  Sequence (2)


<220>
<221>  misc_feature
<222>  (6)..(6)
<223>  t = 5′-dimethyl-thymidine (5′-diMe-T)

<400>  2
gcgtttttg ct                                                          12


<210>  3
<211>  12
<212>  RNA
<213>  Artificial Sequence

<220>
<223>  Target oligoRNA

<400>  3
   agcaaaaaac gc                                                          12


<210>  4
<211>  12
<212>  DNA
<213>  Artificial Sequence

<220>

<223>  Target oligoDNA

<400>  4
agcaaaaaac gc                                                          12


<210>  5
<211>  14
<212>  DNA
<213>  Artificial Sequence

<220>
<223>  Sequence (5)


<220>
<221>  misc_feature
<222>  (1)..(3)
<223>  2′-o-methyl modified

<220>
<221>  misc_feature
<222>  (12)..(14)
<223>  2′-o-methyl modified

<400>  5
gcgttttttt tgcu                                                        14


<210>  6
<211>  14
<212>  DNA
<213>  Artificial Sequence

<220>
<223>  Sequence (6)


<220>
<221>  misc_feature
<222>  (1)..(3)
<223>  2′-o-methyl modified

<220>
<221>  misc_feature
<222>  (7)..(7)
<223>  t = 5′-dimethyl-thymidine  (5′-diMe-T)

<220>
<221>  misc_feature
<222>  (11)..(11)
<223>  t = 5′-dimethyl-thymidine  (5′-diMe-T)

<220>
<221>  misc_feature
<222>  (12)..(14)
<223>  2′-o-methyl modified

<400>  6
gcgttttttt tgcu                                                        14


<210>  7

<211>  14
<212>  DNA
<213>  Artificial Sequence

<220>
<223>  Sequence (7)


<220>
<221>  misc_feature
<222>  (1)..(3)
<223>  2'-o-methyl modified

<220>
<221>  misc_feature
<222>  (5)..(5)
<223>  t = 5'-dimethyl-thymidine (5'-diMe-T)

<220>
<221>  misc_feature
<222>  (8)..(8)
<223>  t = 5'-dimethyl-thymidine (5'-diMe-T)

<220>
<221>  misc_feature
<222>  (11)..(11)
<223>  t = 5'-dimethyl-thymidine (5'-diMe-T)

<220>
<221>  misc_feature
<222>  (12)..(14)
<223>  2'-o-methyl modified

<400>  7
gcgttttttt tgcu                                                          14


<210>  8
<211>  14
<212>  DNA
<213>  Artificial Sequence

<220>
<223>  Sequence (8)


<220>
<221>  misc_feature
<222>  (1)..(14)
<223>  2'-o-methyl modified

<400>  8
gcguuuuuuu ugcu                                                          14


<210>  9
<211>  14
<212>  RNA
<213>  Artificial Sequence

<220>
<223>  complementary RNA to Sequences (5) to (8)

<400>  9
agcaaaaaaa acgc                                                         14


<210>  10
<211>  14
<212>  DNA
<213>  Artificial Sequence

<220>
<223>  ASO1


<220>
<221>  misc_feature
<222>  (1)..(1)
<223>  g = LNA-g

<220>
<221>  misc_feature
<222>  (2)..(2)
<223>  t = LNA-t

<220>
<221>  misc_feature
<222>  (3)..(3)
<223>  c = LNA-5-methylcytosine

<220>
<221>  misc_feature
<222>  (12)..(12)
<223>  t = LNA-t

<220>
<221>  misc_feature
<222>  (13)..(14)
<223>  g = LNA-g

<400>  10
gtctctttac ctgg                                                         14


<210>  11
<211>  14
<212>  DNA
<213>  Artificial Sequence

<220>
<223>  ASO2


<220>
<221>  misc_feature
<222>  (1)..(1)
<223>  g = LNA-g

<220>
<221>  misc_feature
<222>  (2)..(2)
<223>  t =  LNA-t

<220>
<221>  misc_feature

<222>   (3)..(3)
<223>   c = LNA-5-methylcytosine

<220>
<221>   misc_feature
<222>   (7)..(7)
<223>   t = 5′-dimethyl-thymidine (5′-diMe-T)

<220>
<221>   misc_feature
<222>   (12)..(12)
<223>   t =  LNA-t

<220>
<221>   misc_feature
<222>   (13)..(14)
<223>   g = LNA-g

<400>   11
gtctctttac ctgg                                                         14


<210>   12
<211>   14
<212>   DNA
<213>   Artificial Sequence

<220>
<223>   ASO3


<220>
<221>   misc_feature
<222>   (1)..(1)
<223>   a = LNA-a

<220>
<221>   misc_feature
<222>   (2)..(3)
<223>   g = LNA-g

<220>
<221>   misc_feature
<222>   (12)..(12)
<223>   t =  LNA-t

<220>
<221>   misc_feature
<222>   (13)..(13)
<223>   c =  LNA-5-methylcytosine

<220>
<221>   misc_feature
<222>   (14)..(14)
<223>   t =  LNA-t

<400>   12
aggtgctttg gtct                                                         14


<210>   13
<211>   14
<212>   DNA

<213>  Artificial Sequence

<220>
<223>  ASO4



<220>
<221>  misc_feature
<222>  (1)..(1)
<223>  a =  LNA-a

<220>
<221>  misc_feature
<222>  (2)..(3)
<223>  g = LNA-g

<220>
<221>  misc_feature
<222>  (9)..(9)
<223>  t = 5′-dimethyl-thymidine (5′-diMe-T)

<220>
<221>  misc_feature
<222>  (12)..(12)
<223>  t = LNA-t

<220>
<221>  misc_feature
<222>  (13)..(13)
<223>  c = LNA-5-methylcytosine

<220>
<221>  misc_feature
<222>  (14)..(14)
<223>  t = LNA-t

<400>  13
aggtgctttg gtct                                                          14


<210>  14
<211>  20
<212>  DNA
<213>  Artificial Sequence

<220>
<223>  NR3C1 forward

<400>  14
actgtccagc atgccgctat                                                    20


<210>  15
<211>  20
<212>  DNA
<213>  Artificial Sequence

<220>
<223>  NR3C1 reverse

<400>  15
gcagtggctt gctgaattcc                                                    20

<210>  16
<211>  20
<212>  DNA
<213>  Artificial Sequence

<220>
<223>  GAPDH forward

<400>  16
gtgtgaacgg atttggccgt                                                   20


<210>  17
<211>  20
<212>  DNA
<213>  Artificial Sequence

<220>
<223>  GAPDH reverse

<400>  17
gacaagcttc ccattctcgg                                                   20


<210>  18
<211>  12
<212>  DNA
<213>  Artificial Sequence

<220>
<223>  Sequence (13)


<220>
<221>  misc_feature
<222>  (6)..(6)
<223>  t = 5'-diethyl-thymidine (5'-diEt-T)

<400>  18
gcgtttttg ct                                                            12


<210>  19
<211>  12
<212>  DNA
<213>  Artificial Sequence

<220>
<223>  Sequence (14)


<220>
<221>  misc_feature
<222>  (6)..(6)
<223>  t = 5'-diethyl-thymidine (5'-diEt-T)

<220>
<221>  misc_feature
<222>  (8)..(8)
<223>  t = 5'-diethyl-thymidine (5'-diEt-T)

<400>  19
   gcgttttttg ct 12